(19) Europäisches Patentamt / European Patent Office / Office européen des brevets

(11) **EP 3 004 143 B1**

(12) **EUROPEAN PATENT SPECIFICATION**

(45) Date of publication and mention
of the grant of the patent:
**23.10.2019 Bulletin 2019/43**

(21) Application number: **14728234.7**

(22) Date of filing: **06.06.2014**

(51) Int Cl.:
*C07K 14/16* (2006.01)          *A61K 39/21* (2006.01)
*C12N 15/49* (2006.01)

(86) International application number:
**PCT/EP2014/061924**

(87) International publication number:
**WO 2014/195510 (11.12.2014 Gazette 2014/50)**

(54) **MUTATED NON-PRIMATE LENTIVIRAL ENV PROTEINS AND THEIR USE AS DRUGS**

MUTIERTE LENTIVIRALE NICHTPRIMATEN-ENV-PROTEINE UND DEREN VERWENDUNG ALS ARZNEIMITTEL

PROTÉINES ENV LENTIVIRALES NON ORIGINAIRES MUTÉES ET LEUR UTILISATION COMME MÉDICAMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.06.2013 EP 13305767**

(43) Date of publication of application:
**13.04.2016 Bulletin 2016/15**

(73) Proprietors:
• **Viroxis S.A.S.**
**75017 Paris (FR)**
• **Centre National de la Recherche Scientifique**
**75794 Paris Cedex 16 (FR)**
• **Institut Gustave Roussy**
**94805 Villejuif Cédex (FR)**
• **Université Paris Sud XI**
**91405 Orsay (FR)**

(72) Inventor: **HEIDMANN, Thierry**
**F-75017 Paris (FR)**

(74) Representative: **Grosset-Fournier, Chantal
Catherine et al
Grosset-Fournier & Demachy
54, rue Saint-Lazare
75009 Paris (FR)**

(56) References cited:
**WO-A1-2005/095442     WO-A2-2010/022740**

• **DENNER J ET AL: "THE IMMUNOSUPPRESSIVE PEPTIDE OF HIV-1: FUNCTIONAL DOMAINS AND IMMUNE RESPONSE IN AIDS PATIENTS", AIDS, PHILADELPHIA,PA, US, vol. 8, no. 8, 1 August 1994 (1994-08-01), pages 1063-1072, XP000647542,**
• **MANGENEY M ET AL: "Placental syncytins: Genetic disjunction between the fusogenic and immunosuppressive activity of retroviral envelope proteins", PROCEEDINGS OF THE NATIONAL ACADEMY OF SCIENCES, NATIONAL ACADEMY OF SCIENCES, US, vol. 104, no. 51, 18 December 2007 (2007-12-18), pages 20534-20539, XP002633415, ISSN: 0027-8424, DOI: 10.1073/PNAS.0707873105 [retrieved on 2007-12-12]**
• **GÉRALDINE SCHLECHT-LOUF ET AL: "Retroviral infection in vivo requires an immune escape virulence factor encrypted in the envelope protein of oncoretroviruses", PROCEEDINGS OF THE NATIONAL ACADEMY OF SCIENCES, NATIONAL ACADEMY OF SCIENCES, US, vol. 107, no. 8, 23 February 2010 (2010-02-23), pages 3782-3787, XP002675453, ISSN: 0027-8424, DOI: 10.1073/PNAS.0913122107 [retrieved on 2010-02-08]**

Remarks:
The complete document including Reference Tables and the Sequence Listing can be downloaded from the EPO website

**Description**

The present invention relates to mutated non-primate lentiviral ENV proteins and their use as drugs.

Lentivirus is a genus of slow viruses of the Retroviridae family, characterized by a long incubation period. Lentiviruses have the unique ability among retroviruses of being able to integrate into the DNA of the host and replicate in non-dividing cells. They are in most cases pathogenic, infection resulting in a severe immunodeficiency syndrome (AIDS).

Lentiviruses can be found in primates including humans (HIV1, HIV2) and monkeys (SIV), as well as in other animals.

As examples of animal lentiviruses, one may cite feline lentiviruses such as Feline Imunodeficiency Virus (FIV) which can be found in cats, as well as in other felidae (e.g. lion, cougar).

Other examples of animal lentiviruses are bovine immunodeficiency virus (BIV), Jembrana disease virus (JDV, BIV from Indonesia), equine infectious anaemia virus (EIAV), caprine arthritis encephalitis virus (CAEV), Visna/Maedi virus (VMV) and ovine Visna/Maedi virus (OMVV).

Feline imunodeficiency virus (FIV) causes an AIDS-like syndrome in the domestic cat, with marked similarity to AIDS caused by human immunodeficiency virus (HIV) in humans. FIV in cats typically manifests itself as a transient acute-phase syndrome, characterized by febrile episodes, lymphadenopathy, neutropenia, and weight loss. This initial phase is followed by a protracted asymptomatic period with progressive loss of CD4+ T lymphocytes and a terminal AIDS phase characterized by succumbing to opportunistic infections. Variability exists among FIV strains, not only in sequence relatedness but also in pathogenicity *in vivo*. FIV has been reported worldwide with a prevalence rate ranging from 1 to 28%. It affects 2-4% of cats in the US.

Following criteria similar to those for HIV subgroups distinction, FIV has been classified into subgroups according to the envelop protein (Env) diversity. Sodora et al. defined the original Petaluma strain as a prototype subgroup A and the divergent Japanese TM2 strain as prototype subgroup B. A majority of FIV sequences obtained worldwide were categorized in the A and B subgroups. A subtype C was also defined and is the least prevalent FIV subgroup, as well as two other subtypes D and E.

As in the case of HIV, the development of an effective vaccine against FIV is difficult because of the variations of the virus strains, most probably associated with the high intrinsic mutation rate of its replicative machinery. A dual-subtype vaccine for FIV released in 2002 called Fel-O-Vax was shown to have some protective effect against subtypes A and B FIV, but a more effective vaccine is still needed for an efficient protection of cats.

A key feature that could result in higher efficacy of a FIV dedicated vaccine is to increase the immunogenicity of the FIV antigens that are introduced in the vaccine. In this respect, it is essential to ascertain that the antigens do not carry an immunosuppressive activity, which would severely decrease the immunogenicity of the vaccine. In fact, most vaccines against retroviruses contain the Env protein as it is the first exposed viral protein to be « seen » by the immune system of an infected animal, and it is important for a vaccinated animal to be able to mount an efficient line of defence against this protein. Yet, it has been shown in the case of the non-lentiviral retroviruses - including oncoretroviruses such as the feline FeLV or the human HTLV retroviruses - that their Env protein carries an immunosuppressive activity which drastically decreases the immunogenicity of the corresponding proteins, and severely impairs the efficacy of a vaccine expressing the Env protein.

Consequently for a vaccine, there is a need to provide proteins as antigens having lost, or substantially lost, their immunosuppressive functions, in order to generate an efficient response. This will enable the animals once infected by the virus to allow the immune system to destroy the infected cells and prevent/cure the infection.

WO 2010/022740 discloses immunogenic HIV-1 envelope polypeptides, wherein specific amino acid residues are mutated to repress immunosuppression in GP41. In *ex vivo* experiments, said mutated HIV envelope polypeptides inhibit proliferation of PBMC, but such results are not confirmed by *in vivo* data.

Denner *et al.* (1994) discloses immunosuppressive peptide (ISU-peptide) of HIV-1 which corresponds to the amino-acid domain 583-599 of the transmembrane domain glycoprotein GP41 of HIV-1. In this study, the authors suggest that said ISU-peptide might contribute to the development of AIDS.

One aim of the invention is to provide new mutated Env proteins devoid of immunosuppressive properties.

Another aim of the invention is to provide new mutated animal Env proteins.

Another aim of the invention is to provide a new pharmaceutical composition efficient for preventing/treating lentiviral infection.

Another aim of the invention is to provide an efficient vaccine against animal lentiviruses, in particular feline lentiviruses.

The description relates to a pharmaceutical composition comprising as active substance an isolated mutated feline lentiviral ENV protein having a decreased immunosuppressive activity, substantially no immunosuppressive activity or no immunosuppressive activity, or a fragment thereof,

said mutated feline lentiviral ENV protein resulting from mutation of the transmembrane (TM) subunit of a wild type feline lentiviral ENV protein,

said mutated ENV protein having at least 70% identity, preferably at least 80% identity, to the sequence SEQ ID NO: 5, said mutated feline lentiviral ENV protein or fragment thereof comprising a mutated immunosuppressive domain (ISU) containing the following amino acid sequence:

A-[I/M/T/L]-$X_1$-$X_2$-$X_3$-$X_4$-$X_5$-T-A (SEQ ID NO: 1),

wherein,

$X_1$ is any amino acid different from E or deleted, and/or

$X_2$ is any amino acid different from K or deleted, and/or

$X_3$ is any amino acid different from F or deleted, and/or

$X_4$ is any amino acid different from L or deleted, and/or

$X_5$ is any amino acid different from Y or deleted, in association with a pharmaceutically acceptable carrier.

In particular, the description relates to a pharmaceutical composition comprising as active substance an isolated mutated feline lentiviral ENV protein having a decreased immunosuppressive activity, substantially no immunosuppressive activity or no immunosuppressive activity, or a fragment thereof,

in particular said fragment of said isolated mutated feline lentiviral ENV protein comprising at least 40 amino acids, in particular at least 60 amino acids,

said mutated feline lentiviral ENV protein resulting from mutation of the transmembrane (TM) subunit of a wild type feline lentiviral ENV protein,

said mutated ENV protein having at least 70% identity, preferably at least 80% identity, to the sequence SEQ ID NO: 5, said mutated feline lentiviral ENV protein or fragment thereof comprising a mutated immunosuppressive domain (ISU) containing the following amino acid sequence:

A-[I/M/T/L]-$X_1$-$X_2$-$X_3$-$X_4$-$X_5$-T-A (SEQ ID NO: 1),

wherein,

$X_1$ is A, F, G, L or R, and $X_2$, $X_3$, $X_4$ and $X_5$ are any amino acid, or

$X_2$ is A, F, G, L or R, and $X_1$, $X_3$, $X_4$ and $X_5$ are any amino acid, or

$X_3$ is A, G, L or R, and $X_1$, $X_2$, $X_4$ and $X_5$ are any amino acid, or

$X_4$ is A, F, G or R, and $X_1$, $X_2$, $X_3$ and $X_5$ are any amino acid, or

$X_5$ is A, F, G, L or R, and $X_1$, $X_2$, $X_3$ and $X_4$ are any amino acid,

in association with a pharmaceutically acceptable carrier.

In particular, the invention relates to a pharmaceutical composition comprising as active substance an isolated mutated feline lentiviral ENV protein, or a fragment thereof, that has lost at least 50%, at least 75% or 100% of its immunosuppressive activity in comparison to the immunosuppressive activity of the wild type Env protein, said fragment of said isolated mutated feline lentiviral ENV protein comprising at least 40 amino acids, in particular at least 60 amino acids,

said mutated feline lentiviral ENV protein resulting from mutation of the transmembrane (TM) subunit of a wild type feline lentiviral ENV protein,

said mutated ENV protein having at least 70% identity, preferably at least 80% identity, to the sequence SEQ ID NO: 5, said mutated feline lentiviral ENV protein or fragment thereof comprising a mutated immunosuppressive domain (ISU) containing the following amino acid sequence:

A-[I/M/T/L]-$X_1$-$X_2$-$X_3$-$X_4$-$X_5$-T-A (SEQ ID NO: 1),

wherein,

$X_1$ is R, $X_2$ is K, $X_3$ is F or P, $X_4$ is L or V or I and $X_5$ is Y, or

$X_2$ is R, $X_1$ is E, $X_3$ is F or P, $X_4$ is L or V or I and $X_5$ is Y, or

$X_3$ is R, $X_1$ is E, $X_2$ is K, $X_4$ is L or V or I and $X_5$ is Y, or

$X_4$ is R, $X_1$ is E, $X_2$ is K, $X_3$ is F or P and $X_5$ is Y, or

$X_5$ is R, $X_1$ is E, $X_2$ is K, $X_3$ is F or P and $X_4$ is L or V or I,

which respectively correspond to the following amino acid sequence:

A-[I/M/T/L]-R-K-[F/P]-[L/V/I]-Y-T-A (SEQ ID NO: 497), or
A-[I/M/T/L]-E-R-[F/P]-[L/V/I]-Y-T-A (SEQ ID NO: 498), or
A-[I/M/T/L]-E-K-R-[L/V/I]-Y-T-A (SEQ ID NO: 499), or
A-[I/M/T/L]-E-K-[F/P]-R-Y-T-A (SEQ ID NO: 500), or
A-[I/M/T/L]-E-K-[F/P]-[L/V/I]-R-T-A (SEQ ID NO: 501),

in association with a pharmaceutically acceptable carrier,

said loss of at least 50%, at least 75% or 100% of its immunosuppressive activity of the above mentioned mutated feline lentiviral ENV protein or of the above defined fragment being liable to be assessed by the fact that in an *in vivo* assay involving engrafted tumor cells rejection, in animals excluding human beings,

said tumor cells being transduced either so as to express said mutated ENV protein or said fragment (mutated ENV tumor cells),
or said tumor cells being transduced so as to express said wild type ENV protein or a fragment thereof (wild type ENV tumor cells),
or said tumor cells being not transduced (normal tumor cells),
the following ratio:

$$\frac{\text{immunosuppression index of said mutated ENV protein or of said fragment } (i_{\text{mutated env}})}{\text{immunosuppression index of wild type ENV protein } (i_{\text{wild type env}})}$$

is less than 0.5, or even less than 0.25,
$i_{\text{mutated env}}$ being defined by:

$$\frac{\text{maximum area reached by mutated ENV tumor cells} - \text{maximum area reached by normal tumor cells}}{\text{maximum area reached by normal tumor cells}},$$

and
$i_{\text{wild type env}}$ being defined by:

$$\frac{\text{maximum area reached by wild type ENV tumor cells} - \text{maximum area reached by normal tumor cells}}{\text{maximum area reached by normal tumor cells}}.$$

The present application is based on the unexpected observation made by the Inventors that some specific amino acids of the immunosuppressive domain (ISU) of a lentiviral Env protein can be mutated conferring to said lentiviral Env protein decreased immunosuppressive properties, substantially no immunosuppressive properties or no immunosuppressive properties, while retaining its antigenicity, the three-dimentional structure of the immunosuppressive domain, and its expression at the plasma membrane.
The expression *"having decreased immunosuppressive properties"* means that the mutated Env protein has lost at least 50 % of its immunosuppressive activity in comparison to the immunosuppressive activity of the wild type Env protein.
The expression *"having substantially no immunosuppressive properties"* means that the mutated Env protein has lost at least 75 % of its immunosuppressive activity in comparison to the immunosuppressive activity of the wild type Env protein.
The expression *"having no immunosuppressive properties"* means that the mutated Env protein has lost 100 % of its immunosuppressive activity in comparison to the immunosuppressive activity of the wild type Env protein.
Moreover, the mutated lentiviral Env protein according to the invention has retained a part or the totality of its fusogenic activity.
In the description and the invention, the expression *"mutated feline lentiviral ENV proteins"* means that the ENV proteins derive from the expression of an *env* gene of a lentivirus of a feline.
Feline lentiviruses according to the invention encompass the FIV which can be found in cats, as well as in other felidae (e.g. lion, cougar).
Because of the natural variability of feline lentiviruses, the "mutated Env protein", as defined in the invention, encompasses two meanings.
According to the first meaning, the said "mutated ENV protein" is the unnatural result of the intervention of human beings. According to the second meaning, the mutated Env protein also encompasses naturally occurring variants for which up to now the non immunosuppressive properties remain unknown.
This second meaning takes into consideration the natural variability of FIV variants inside a same infected animal, wherein the said "mutated Env protein" might be non immunosuppressive but its property is undetectable because an FIV infected animal carries many FIV variants, the majority of which is immunosuppressive.
The following protein (Accession No. P16090) corresponds to the wild type sequence of the Env protein of FIV (Petaluma strain). In the invention, it is considered as a reference sequence of the wild type ENV protein.

| SEQ ID NO: 4 wild type FIV Env (Petaluma strain) | MAEGFAANRQWIGPEEAEELLDFDIATQMSEEGPLNPGVNPFRVPGI TEKEKQNYCNILQPKLQDLRNEIQEVKLEEGNAGKFRRARFLRYSDE RVLSLVHAFIGYCIYLGNRNKLGSLRHDIDIEAPQEECYNNREKGTT DNIKYGRRCCLGTVTLYLILFTGVIVYSQTAGAQVVWRLPPLVVPVE ESEIIFWDCWAPEEPACQDFLGAMIHLKAKTNISIREGPTLGNWARE IWATLFKKATRQCRRGRIWKRWNETITGPSGCANNTCYNVSVIVPDY QCYLDRVDTWLQGKINISLCLTGGKMLYNKVTKQLSYCTDPLQIPLI NYTFGPNQTCMWNTSQIQDPEIPKCGWWNQMAYYNSCKWEEAKVKFH CQRTQSQPGSWFRAISSWKQRNRWEWRPDFKSKKVKISLPCNSTKNL TFAMRSSGDYGEVTGAWIEFGCHRNKSNLHTEARFRIRCRWNVGSDT SLIDTCGNTPNVSGANPVDCTMYSNKMYNCSLQNGFTMKVDDLIVHF NMTKAVEMYNIAGNWSCTSDLPSSWGYMNCNCTNSSSSYSGTKMACP SNRGILRNWYNPVAGLRQSLEQYQVVKQPDYLLVPEEVMEYKPRRKR AAIHVMLALATVLSIAGAGTGATAIGMVTQYHQVLATHQEAIEKVTG ALKINNLRLVTLEHQVLVIGLKVEAMEKFLYTAFAMQELGCNQNQFF CKIPLELWTRYNMTINQTIWNHGNITLGEWYNQTKDLQQKFYEIIMD IEQNNVQGKTGIQQLQKWEDWVRWIGNIPQYLKGLLGGILGIGLGVL LLILCLPTLVDCIRNCIHKILGYTVIAMPEVEGEEIQPQMELRRNGR QCGMSEKEEE |

Variants of the FIV mutated Env proteins according to the invention have at least 70%, preferably at least 80%, more preferably at least 90% of identity with the wild type amino acid sequence of the FIV Env protein, and comprise the mutations as described above, and harbour a decreased immunosuppressive activity, substantially no immunosuppressive activity or no immunosuppressive activity.

A region of the feline lentiviral Env protein containing the amino acids which may be mutated according to the invention can be delimited by the sequence SEQ ID NO: 2:
G-L-[K/T/R]-[V/I]-[E/R]-A-[I/M/T/L]-E-K-[F/P]-[L/V/I]-Y-T-A-[F/L]-A-M(SEQ ID NO: 2).

SEQ ID NO: 2 corresponds to the consensus sequence of a specific region of the ISU domain. In the invention, the ISU domain comprises SEQ ID NO: 2.

When applying the mutations as defined above, the ISU domain of the Env protein loses partially or totally its immunosuppressive properties.

Examples of fragments of the wild type ISU domains of FIV:

AIEKVTGALKINNLRLVTLEHQVLVIGLKVEAM**EKFLY**TAFAMQELGCNQNQ FFCKIPLELWTR (SEQ ID NO: 6)

AVEKVTEALKINNLRLVTLEHQVLVIGLKVEAM**EKFLY**TAFAMQELGCNQNQ FFCKIPPELWTR (SEQ ID NO: 7)

AIEKVTEALKITNLRLVTLEHQVLVIGLKVEAM**EKFLY**TAFAMQELGCNQNQ FFCKVPPELWRR (SEQ ID NO: 8)

AIEKVTEALKINNLRLVTLEHQVLVIGLKVEAI**EKFLY**TAFAMQELGCNQNQF FCKVPLELWRR (SEQ ID NO: 9)

AIEKVTEALKINNLRLVTLEHQVLVIGLKVEAM**EKFLY**TAFAMQELGCNQNQ FFCKVPSALWER (SEQ ID NO: 10)

AIEKVTEALKINNLRLVTLEHQVLVIGLKVEAM**EKFLY**TAFAMQELGCNQNQ FFCKIPPGLWTR (SEQ ID NO: 11)

(continued)

Examples of fragments of the wild type ISU domains of FIV:

AIEKVTEALKINNLRLVTLEHQVLVIGLKVEAM**EKFLY**TAFAMQELGCNQNQ FFCKVPPQLWKR (SEQ ID NO: 12)

AIEKVTEALKVNNLRLITLEHQVLVIGLKVEAM**EKFLY**TAFAMQELGCNQNQ FFCKVPPELWKR (SEQ ID NO: 13)

TIEKVTEALKINNLRLVTLEHQVLVIGLKVEAM**EKFLY**TAFAMQELGCNQNQ FFCKVPPELWKR (SEQ ID NO: 14)

AIEKVTEALKINNLRLVTLEHQVLVIGLKVEAM**EKFLY**TAFAMQELGCNQNQ FFCKVPPVLWER (SEQ ID NO: 15)

TIEKITEALKVNNLRLVTLEHQVLVIGLKVEAI**EKFLY**TAFAMQELGCNQNQF FCKVPPELWQR (SEQ ID NO: 16)

AIDQITEALKINNLRLVTLEHQVLVIGLKVEAI**EKFIY**TAFAMQELGCNQNQFF CKIPPELWIR (SEQ ID NO: 17)

ALDKITEALKINNLRLITLEHQVLVIGLKVEAI**EKFLY**TAFAMQELGCNQNQFF CKIPPSLWSM (SEQ ID NO: 18)

ALDKITEALKINNLRLVTLEHQVLVIGLKVEAT**EKFLY**TAFAMQELGCNQNQF FCKIPCELWMR (SEQ ID NO: 19)

ALEKITEALKINNLRLVTLEHQVLMIGLKVEAI**EKFLY**TAFAMQELGCNQNQF FCKIPLNLWTM (SEQ ID NO: 20)

ALDKITEALKINNLRLITLVHQVLVIGLKVRAI**EKPLY**TAFAMQELGCNQNQFF CKIPPSLWSM (SEQ ID NO: 21)

ALEKITEALKINNLRLITLEHQVLVIGLRVEAI**EKFLY**TAFAMQELGCNQNQFF CKIPPSLWSM (SEQ ID NO: 22)

ALEKITEALKINNLRLITLEHQVLVIGLKVEAI**EKFLY**TAFAMQELGCHQNQFF CKIPPSLWSM (SEQ ID NO: 23)

ALDKITEALKINNLRLITLEHQVLVIGLKVEAI**EKFLY**TAFAMQELGCNQNQFF CKIPPSLWSM (SEQ ID NO: 24)

ALDKITQALKINNLRLITLEHQVLVIGLKVEAI**EKFLY**TAFAMQELGCNQNQFF CKIPPSLWSM (SEQ ID NO: 25)

ALDKITEALKINNLRLITLEHQVLVIGLKVEAI**EKFLY**TAFAMQELGCNQNQFF CKIPLNLWNM ( SEQ ID NO: 26)

ALDKITEALKINNLRLVTLEHQMLVIGLKVEAI**EKFLY**TAFAMQELGCNQNQF FCEIPKELWLR (SEQ ID NO: 27)

The localization of an ISU domain can be determined in all Env proteins of viruses as described in Benit et al.

2001, Journal of Virology, Vol. 75, No. 23, p.11709-11719. In a broad meaning, the ISU domain is defined by its structure and its localization, irrespective of the fact that it possesses or not an immunosuppressive activity.

In the description and the invention, the ISU domain refers to a specific domain in which a mutation can affect the immunosuppressive property of the ENV protein.

As an example of a mutated feline lentiviral Env protein, SEQ ID NO: 5 corresponds to the sequence of a mutated Env protein of the FIV Petaluma strain. In the invention, it is considered as a reference sequence of the mutated Env protein.

More specifically, SEQ ID NO: 5 corresponds to the SEQ ID NO: 4 in which the amino acid residue F in position 5 ($X_3$) of the sequence A-[I/M/T/L]-$X_1$-$X_2$-$X_3$-$X_4$-$X_5$-T-A (SEQ ID NO: 1) has been substituted by R.

| SEQ ID NO: 5 mutated FIV Env | MAEGFAANRQWIGPEEAEELLDFDIATQMSEEGPLNPGVNPFRV<br>PGITEKEKQNYCNILQPKLQDLRNEIQEVKLEEGNAGKFRRARF<br>LRYSDERVLSLVHAFIGYCIYLGNRNKLGSLRHDIDIEAPQEEC<br>YNNREKGTTDNIKYGRRCCLGTVTLYLILFTGVIVYSQTAGAQV<br>VWRLPPLVVPVEESEIIFWDCWAPEEPACQDFLGAMIHLKAKTN<br>ISIREGPTLGNWAREIWATLFKKATRQCRRGRIWKRWNETITGP<br>SGCANNTCYNVSVIVPDYQCYLDRVDTWLQGKINISLCLTGGKM<br>LYNKVTKQLSYCTDPLQIPLINYTFGPNQTCMWNTSQIQDPEIP<br>KCGWWNQMAYYNSCKWEEAKVKFHCQRTQSQPGSWFRAISSWKQ<br>RNRWEWRPDFKSKKVKISLPCNSTKNLTFAMRSSGDYGEVTGAW<br>IEFGCHRNKSNLHTEARFRIRCRWNVGSDTSLIDTCGNTPNVSG<br>ANPVDCTMYSNKMYNCSLQNGFTMKVDDLIVHFNMTKAVEMYNI<br>AGNWSCTSDLPSSWGYMNCNCTNSSSSYSGTKMACPSNRGILRN<br>WYNPVAGLRQSLEQYQVVKQPDYLLVPEEVMEYKPRRKRAAIHV<br>MLALATVLSIAGAGTGATAIGMVTQYHQVLATHQEAIEKVTGAL<br>KINNLRLVTLEHQVLVIGLKVEAM**EKRLY**TAFAMQELGCNQNQF<br>FCKIPLELWTRYNMTINQTIWNHGNITLGEWYNQTKDLQQKFYE<br>IIMDIEQNNVQGKTGIQQLQKWEDWVRWIGNIPQYLKGLLGGIL<br>GIGLGVLLLILCLPTLVDCIRNCIHKILGYTVIAMPEVEGEEIQ<br>PQMELRRNGRQCGMSEKEEE |

The invention also encompasses the variants of the "mutated feline lentiviral Env protein", harbouring the above mentioned mutations, and conferring a decrease or a lack of immunosuppressive properties to said variant.

Variants of the FIV mutated Env proteins according to the invention have at least 70%, preferably at least 80%, more preferably at least 90% of identity with the reference mutated sequence of FIV env protein (SEQ ID NO: 5), and comprise the mutations as described above, and harbour a decreased immunosuppressive activity, substantially no immunosuppressive activity or no immunosuppressive activity.

Variants of the FIV mutated Env proteins according to the invention have at least 90% of identity with the transmembrane region of SEQ ID NO 5

According to a particular embodiment of the invention, variants of the FIV mutated Env proteins according to the invention have a transmembrane region which has at least 90% of identity with the transmembrane region of the FIV mutated Env protein, said variants comprising a mutated immunosuppressive domain (ISU) containing the sequence SEQ ID NO: 1. According to another particular embodiment of the invention, variants of the FIV mutated Env proteins according to the invention have a 64 amino acid sequence, corresponding to a fragment of the ISU domain, which has at least 95% of identity with a 64 amino acid sequence of the FIV mutated Env protein comprising the sequence SEQ ID NO: 1.

In a particular embodiment, the description relates to a pharmaceutical composition as defined above comprising as active substance an isolated mutated feline lentiviral ENV protein having a decreased immunosuppressive activity, substantially no immunosuppressive activity or no immunosuppressive activity, or a fragment thereof, said mutated feline lentiviral ENV protein resulting from mutation of the transmembrane (TM) subunit of a wild type feline lentiviral ENV protein, said mutated ENV protein having at least 70% identity, preferably at least 80% identity, to the sequence SEQ ID NO: 5, said mutated feline lentiviral ENV protein or fragment thereof comprising a mutated immunosuppressive domain (ISU) containing the following amino acid sequence:

[V/I]-[E/R]-A-[I/M/T/L]-$X_1$-$X_2$-$X_3$-$X_4$-$X_5$-T-A-[F/L]-A-M (SEQ ID NO: 3),

wherein,

$X_1$ is any amino acid different from E or deleted, and/or
$X_2$ is any amino acid different from K or deleted, and/or
$X_3$ is any amino acid different from F or deleted, and/or
$X_4$ is any amino acid different from L or deleted, and/or
$X_5$ is any amino acid different from Y or deleted,
in particular, wherein,
$X_1$ is A, F, G, L or R, and $X_2$, $X_3$, $X_4$ and $X_5$ are any amino acid, or
$X_2$ is A, F, G, L or R, and $X_1$, $X_3$, $X_4$ and $X_5$ are any amino acid, or
$X_3$ is A, G, L or R, and $X_1$, $X_2$, $X_4$ and $X_5$ are any amino acid, or
$X_4$ is A, F, G or R, and $X_1$, $X_2$, $X_3$ and $X_5$ are any amino acid, or
$X_5$ is A, F, G, L or R, and $X_1$, $X_2$, $X_3$ and $X_4$ are any amino acid,
in association with a pharmaceutically acceptable carrier.

In a particular embodiment, the invention relates to a pharmaceutical composition as defined above comprising as active substance an isolated mutated feline lentiviral ENV protein having a decreased immunosuppressive activity, substantially no immunosuppressive activity or no immunosuppressive activity, or a fragment thereof, said mutated feline lentiviral ENV protein resulting from mutation of the transmembrane (TM) subunit of a wild type feline lentiviral ENV protein, said mutated ENV protein having at least 70% identity, preferably at least 80% identity, to the sequence SEQ ID NO: 5, wherein said mutated feline lentiviral ENV protein or fragment thereof comprising a mutated immunosuppressive domain (ISU) containing the following amino acid sequence:

[V/I]-[E/R]-A-[I/M/T/L]-$X_1$-$X_2$-$X_3$-$X_4$-$X_5$-T-A-[F/L]-A-M (SEQ ID NO: 3), wherein,
$X_1$ is R, $X_2$ is K, $X_3$ is F or P, $X_4$ is L or V or I and $X_5$ is Y, or
$X_2$ is R, $X_1$ is E, $X_3$ is F or P, $X_4$ is L or V or I and $X_5$ is Y, or
$X_3$ is R, $X_1$ is E, $X_2$ is K, $X_4$ is L or V or I and $X_5$ is Y, or
$X_4$ is R, $X_1$ is E, $X_2$ is K, $X_3$ is F or P and $X_5$ is Y, or
$X_5$ is R, $X_1$ is E, $X_2$ is K, $X_3$ is F or P and $X_4$ is L or V or I,
in association with a pharmaceutically acceptable carrier.
SEQ ID NO: 3 (14 amino acid long) is a consensus sequence containing SEQ ID NO: 1 (9 amino acid long).

The invention relates to a pharmaceutical composition as defined above comprising as active substance an isolated mutated feline lentiviral ENV protein having a decreased immunosuppressive activity, substantially no immunosuppressive activity or no immunosuppressive activity, or a fragment thereof,
said mutated feline lentiviral ENV protein resulting from mutation of the transmembrane (TM) subunit of a wild type feline lentiviral ENV protein,
said mutated ENV protein having at least 70% identity, preferably at least 80% identity, to the sequence SEQ ID NO: 5,
said mutated feline lentiviral ENV protein or fragment thereof comprising a mutated immunosuppressive domain (ISU) containing the following amino acid sequence:
A-[I/M/T/L]-$X_1$-$X_2$-$X_3$-$X_4$-$X_5$-T-A (SEQ ID NO: 1),
wherein,
$X_1$ is any amino acid different from E or deleted, and/or
$X_2$ is any amino acid different from K or deleted, and/or
$X_3$ is any amino acid different from F or deleted, and/or
$X_4$ is any amino acid different from L or deleted, and/or
$X_5$ is any amino acid different from Y or deleted,
in particular, wherein,
$X_1$ is A, F, G, L or R, and $X_2$, $X_3$, $X_4$ and $X_5$ are any amino acid, or
$X_2$ is A, F, G, L or R, and $X_1$, $X_3$, $X_4$ and $X_5$ are any amino acid, or
$X_3$ is A, G, L or R, and $X_1$, $X_2$, $X_4$ and $X_5$ are any amino acid, or
$X_4$ is A, F, G or R, and $X_1$, $X_2$, $X_3$ and $X_5$ are any amino acid, or
$X_5$ is A, F, G, L or R, and $X_1$, $X_2$, $X_3$ and $X_5$ are any amino acid,
in association with a pharmaceutically acceptable carrier,
said decrease, substantial absence or absence of immunosuppressive activity of the above mentioned mutated feline lentiviral ENV protein or of the above defined fragment being liable to be assessed by the fact that in an *in vivo* assay involving engrafted tumor cells rejection, in animals excluding human beings,
said tumor cells being transduced either so as to express said mutated ENV protein or said fragment (mutated ENV tumor cells),
or said tumor cells being transduced so as to express said wild type ENV protein or a fragment thereof (wild type ENV tumor cells),
or said tumor cells being not transduced (normal tumor cells),
the following ratio:

immunosuppression index of said mutated ENV protein or of said fragment ($i_{mutated\ env}$) / immunosuppression index of wild type ENV protein ($i_{wild\ type\ env}$) is less than 0.5, or even less than 0.25,

$i_{mutated\ env}$ being defined by: (maximum area reached by mutated ENV tumor cells - maximum area reached by normal tumor cells) / (maximum area reached by normal tumor cells), and

$i_{wild\ type\ env}$ being defined by: (maximum area reached by wild type ENV tumor cells - maximum area reached by normal tumor cells) / (maximum area reached by normal tumor cells).

The immunosuppressive property of the ENV protein is preferably measured using in vivo procedures, which are representative of the physiological environment.

The immunosuppressive property of the mutated ENV proteins according to the invention is measured according to an in vivo procedure to assay the immunosuppressive activity of a ENV protein disclosed previously [Mangeney and Heidmann Proc Natl Acad Sci USA 1998;95: 14920-14925; Mangeney et al. Proc Natl Acad Sci USA, 2007, 104(51):20534-9]. As a physiological test, this in vivo procedure is performed using ENV proteins, or fragment thereof, which are not associated to another component or carrier proteins, such as BSA.

Briefly, a wild-type (wild type lentiviral ENV protein) or modified nucleic acid expressing the protein to be tested (mutated lentiviral ENV protein) is transduced in tumour cell lines such as MCA 205 or C18.1 cell lines by known transduction methods. The tumour cells expressing the protein to be tested are then injected especially subcutaneous (s.c.) injection to a host, generally mice. Following said injection, the establishment of tumour or, to the contrary, its rejection, is determined and the tumour area is measured. Tumour establishment is determined by palpation and tumour area (mm2) is determined by measuring perpendicular tumour diameters. Immunosuppression index is defined as $i= (S_{env}-S_{none})/S_{none}$, wherein $S_{env}$ is the maximum area reached by a tumour expressing an envelope protein and $S_{none}$ is the maximum area reached by a tumour not expressing ENV protein (negative control).

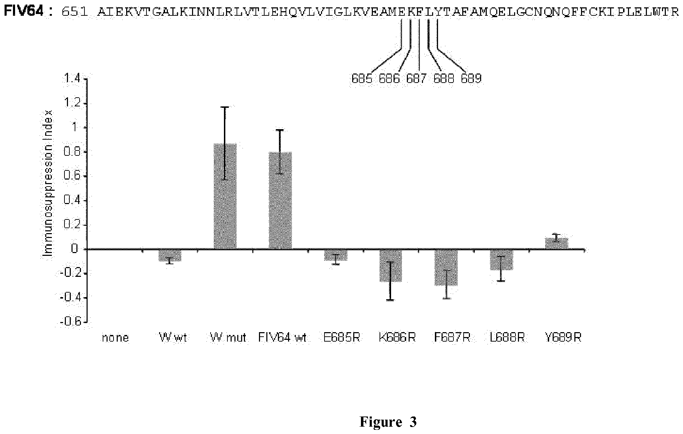

According to an embodiment of the invention, the above defined ratio relative to the immunosuppression index ($i_{mutated\ env}$ / $i_{wild\ type\ env}$) can be less than 0.25, and can even have a negative value (see figure 3).

In vitro assay could be carried out, using high doses of synthetic peptides but they are indirect and less convincing, since the expression "immunosuppressive" is relevant when applied to animals possessing a complete immune system and not to cell lines. An additional difficulty for the functional characterization of an ISU domain relies on the fact that the ISU carried by the retroviral Env proteins is a highly structured proteic domain, with trimer formation within the complete Env proteins (Caffrey M., Biochimica et Biophysica Acta, 1536:116-122, 2001; Caffrey et al., The EMBO Journal, Vol. 17, No. 16, p.4572-4584, 1998). Such structures are not naturally formed with ISU peptides of limited length, and this is most probably why most studies carried out with peptides provide irrelevant results and/or are dependent on specific coupling of the peptides to carrier proteins (such as BSA, e.g. Denner et al., Current Science, AIDS 1994, 8:1063-1072).

As mentioned above, the Env proteins according to the description and the invention are mutated. This mutation is made in vitro. Thus, the mutated Env proteins according to the invention are isolated, and do not correspond to naturally occurring counterpart.

As mentioned above, the lentiviral mutated Env proteins have decreased immunosuppressive properties, substantially no immunosuppressive properties or no immunosuppressive properties. This means that the mutated Env proteins according to the invention have no, or have reduced immunosuppressive properties with respect to the natural non mutated Env proteins from a virus of the same species. For instance, a mutated FIV Env protein according to the invention has reduced immunosuppressive properties with respect to the wild type FIV Env protein.

In the description and the invention, the terms "a decreased immunosuppressive activity, substantially no immunosuppressive activity or no immunosuppressive activity" means that the mutated Env proteins according to the invention have an immunosuppressive index less than about 50 or 25 % of that of the wild type Env protein [Mangeney and Heidmann Proc Natl Acad Sci USA 1998;95: 14920-14925; Mangeney et al. Proc Natl Acad Sci USA, 2007, 104(51):20534-9].

In the description and the invention, structures responsible for the antigenicity of the mutated lentiviral ENV protein are essentially preserved.

As intended herein, the expression "structures responsible for antigenicity" relates to structures of the protein which are liable to interact with components of the immune system such as antibodies or membrane receptors of immune cells, in particular T cells.

The mutation(s) within the immunosuppressive domain of the lentiviral Env proteins is (are) sufficient to decrease the immunosuppressive activity of the mutated lentiviral Env protein with respect to the corresponding wild type Env. However, it might be advantageous that another amino acid be also mutated because it ensures that the structure of the mutated Env protein is essentially conserved with respect to the corresponding wild type Env protein.

The mutated lentiviral Env protein has substantially retained the structure, especially the antigenic structure, e.g., immunogenic determinants, of the original determined lentiviral Env protein, i.e. the wild type non mutated lentiviral Env protein.

These properties can be evaluated by testing the ability of the Env proteins to be normally expressed at the cell membrane under conditions which preserve the surface and transmembrane subunits (SU:TM) interactions and recognition by specific anti-Env antibodies, and by measuring the functional fusogenic activity of said mutated lentiviral Env with respect to the same properties in the wild type non mutated lentiviral Env protein (see examples).

Generally speaking, the mutated ENV protein involved in the present invention has an average length of about 750 to about 1000 amino acids.

The invention encompasses fragments of the mutated ENV protein as defined above, provided that said fragment:

- comprises at least the sequence SEQ ID NO: 1, as defined above,
- comprises at least 40 amino acids, preferably comprises at least 50 amino acids,
- has decreased immunosuppressive properties, substantially no immunosuppressive properties or no immunosuppressive properties, as defined above,
- preferably, comprises the extracellular parts of the ENV protein,
- retains the structure of the ENV protein from which it derives,
- harbours the same epitopes as the corresponding fragment in the wild type ENV protein.

According to a particular embodiment, the fragment of the mutated ENV protein of the invention can comprise about 40, 50, 100, 150, 200, 250, 300, 350, 400, 450, 500, 550, 600, 650, 700, 750, 800, 850, 900, 950 amino acids. These values are given only in an illustrative way, as the man skilled in the art will understand that the fragment can comprise any number of amino acids comprised from about 40 to about 950 amino acids.

Advantageously, the fragments according to the invention are such that, while retaining the antigenic structure of the full length mutated ENV protein, and thus of the wild type ENV protein, they have lost major antigenic regions that are responsible for antigenicity in another region than the region corresponding to the immunosuppressive domain, because said regions could be detrimental for targeting an immune response against the immunosuppressive domain.

The invention also relates to a pharmaceutical composition wherein the active substance is an isolated non naturally occurring feline lentiviral ENV protein having a decreased immunosuppressive activity, substantially no immunosuppressive activity or no immunosuppressive activity, as above defined, or fragments thereof.

In a particular embodiment, the invention relates to a pharmaceutical composition as defined above wherein:

$X_1$ is any amino acid different from E or deleted, and $X_2$, $X_3$, $X_4$ and $X_5$ are any amino acid, or

$X_2$ is any amino acid different from K or deleted, and $X_1$, $X_3$, $X_4$ and $X_5$ are any amino acid, or

$X_3$ is any amino acid different from F or deleted, and $X_1$, $X_2$, $X_4$ and $X_5$ are any amino acid, or

$X_4$ is any amino acid different from L or deleted, and $X_1$, $X_2$, $X_3$ and $X_5$ are any amino acid, or

$X_5$ is any amino acid different from Y or deleted, and $X_1$, $X_2$, $X_3$ and $X_4$ are any amino acid, or

$X_1$ is any amino acid different from E or deleted, $X_2$ is any amino acid different from K or deleted, and $X_3$, $X_4$ and $X_5$ are any amino acid, or

$X_1$ is any amino acid different from E or deleted, $X_3$ is any amino acid different from F or deleted, and $X_2$, $X_4$ and $X_5$ are any amino acid, or

$X_1$ is any amino acid different from E or deleted, $X_4$ is any amino acid different from L or deleted, and $X_2$, $X_3$ and $X_5$ are any amino acid, or

$X_1$ is any amino acid different from E or deleted, $X_5$ is any amino acid different from Y or deleted, and $X_2$, $X_3$ and $X_4$ are any amino acid, or

$X_2$ is any amino acid different from K or deleted, $X_3$ is any amino acid different from F or deleted, and $X_1$, $X_4$ and $X_5$ are any amino acid, or

$X_2$ is any amino acid different from K or deleted, $X_4$ is any amino acid different from L or deleted, and $X_2$, $X_4$ and $X_5$ are any amino acid, or

$X_2$ is any amino acid different from K or deleted, $X_5$ is any amino acid different from Y or deleted, and $X_2$, $X_3$ and $X_4$ are any amino acid, or

$X_3$ is any amino acid different from F or deleted, $X_4$ is any amino acid different from L or deleted, and $X_2$, $X_4$ and $X_5$ are any amino acid, or

$X_3$ is any amino acid different from F or deleted, $X_5$ is any amino acid different from Y or deleted, and $X_2$, $X_3$ and $X_4$ are any amino acid, or

$X_4$ is any amino acid different from L or deleted, $X_5$ is any amino acid different from Y or deleted, and $X_2$, $X_4$ and $X_5$ are any amino acid, or

$X_1$ is any amino acid different from E or deleted, $X_2$ is any amino acid different from K or deleted, $X_3$ is any amino acid different from F or deleted, and $X_4$ and $X_5$ are any amino acid, or

$X_1$ is any amino acid different from E or deleted, $X_2$ is any amino acid different from K or deleted, $X_4$ is any amino acid different from L or deleted, and $X_3$ and $X_5$ are any amino acid, or

$X_1$ is any amino acid different from E or deleted, $X_2$ is any amino acid different from K or deleted, $X_5$ is any amino acid different from Y or deleted, and $X_3$ and $X_4$ are any amino acid, or

$X_1$ is any amino acid different from E or deleted, $X_3$ is any amino acid different from F or deleted, $X_4$ is any amino acid different from L or deleted, and $X_2$ and $X_5$ are any amino acid, or

$X_1$ is any amino acid different from E or deleted, $X_3$ is any amino acid different from F or deleted, $X_5$ is any amino acid different from Y or deleted, and $X_2$ and $X_4$ are any amino acid, or

$X_1$ is any amino acid different from E or deleted, $X_4$ is any amino acid different from L or deleted, $X_5$ is any amino acid different from Y or deleted, and $X_2$ and $X_3$ are any amino acid, or

$X_2$ is any amino acid different from K or deleted, $X_3$ is any amino acid different from F or deleted, $X_4$ is any amino acid different from L or deleted, and $X_1$ and $X_5$ are any amino acid, or

$X_2$ is any amino acid different from K or deleted, $X_3$ is any amino acid different from F or deleted, $X_5$ is any amino acid different from Y or deleted, and $X_1$ and $X_4$ are any amino acid, or

$X_2$ is any amino acid different from K or deleted, $X_4$ is any amino acid different from L or deleted, $X_5$ is any amino acid different from Y or deleted, and $X_1$ and $X_3$ are any amino acid, or

$X_3$ is any amino acid different from F or deleted, $X_4$ is any amino acid different from L or deleted, $X_5$ is any amino acid different from Y or deleted, and $X_1$ and $X_2$ are any amino acid, or

$X_1$ is any amino acid different from E or deleted, $X_2$ is any amino acid different from K or deleted, $X_3$ is any amino acid different from F or deleted, $X_4$ is any amino acid different from L or deleted, and $X_5$ is any amino acid, or

$X_1$ is any amino acid different from E or deleted, $X_2$ is any amino acid different from K or deleted, $X_3$ is any amino acid different from F or deleted, $X_5$ is any amino acid different from Y or deleted, and $X_4$ is any amino acid, or

$X_1$ is any amino acid different from E or deleted, $X_2$ is any amino acid different from K or deleted, $X_4$ is any amino acid different from L or deleted, $X_5$ is any amino acid different from Y or deleted, and $X_3$ is any amino acid, or

$X_1$ is any amino acid different from E or deleted, $X_3$ is any amino acid different from F or deleted, $X_4$ is any amino acid different from L or deleted, $X_5$ is any amino acid different from Y or deleted, and $X_2$ is any amino acid, or

$X_2$ is any amino acid different from K or deleted, $X_3$ is any amino acid different from F or deleted, $X_4$ is any amino acid different from L or deleted, $X_5$ is any amino acid different from Y or deleted and $X_1$ is any amino acid, or

$X_1$ is any amino acid different from E or deleted, $X_2$ is any amino acid different from K or deleted, $X_3$ is any amino acid different from F or deleted, $X_4$ is any amino acid different from L or deleted and $X_5$ is any amino acid different from Y or deleted.

In a particular embodiment, the invention relates to a pharmaceutical composition as defined above, wherein said mutated immunosuppressive domain contains the amino acid sequence SEQ ID NO: 1 or SEQ ID NO: 3, wherein:

$X_1$ is A, F, G, L or R, $X_2$ is A, F, G, L or R, and $X_3$, $X_4$ and $X_5$ are any amino acid, or
$X_1$ is A, F, G, L or R, $X_3$ is A, G, L or R, and $X_2$, $X_4$ and $X_5$ are any amino acid, or
$X_1$ is A, F, G, L or R, $X_4$ is A, F, G or R, and $X_2$, $X_3$ and $X_5$ are any amino acid, or
$X_1$ is A, F, G, L or R, $X_5$ is A, F, G, L or R, and $X_2$, $X_3$ and $X_4$ are any amino acid, or
$X_2$ is A, F, G, L or R, $X_3$ is A, G, L or R, and $X_1$, $X_4$ and $X_5$ are any amino acid, or
$X_2$ is A, F, G, L or R, $X_4$ is A, F, G or R, and $X_1$, $X_3$ and $X_5$ are any amino acid, or
$X_2$ is A, F, G, L or R, $X_5$ is A, F, G, L or R, and $X_1$, $X_3$ and $X_4$ are any amino acid, or
$X_3$ is A, G, L or R, $X_4$ is A, F, G or R, and $X_1$, $X_2$ and $X_5$ are any amino acid, or
$X_3$ is A, G, L or R, $X_5$ is A, F, G, L or R, and $X_1$, $X_2$ and $X_4$ are any amino acid, or
$X_4$ is A, F, G or R, $X_5$ is A, F, G, L or R, and $X_1$, $X_2$ and $X_3$ are any amino acid.

In another embodiment, the invention relates to a pharmaceutical composition as defined above, wherein:

$X_1$ is any amino acid different from E or deleted, and/or
$X_2$ is any amino acid different from K, or deleted, and/or
$X_3$ is any amino acid different from F, P or deleted, and/or
$X_4$ is any amino acid different from L, V, I or deleted, and/or
$X_5$ is any amino acid different from Y or deleted.

In another embodiment, the invention relates to a pharmaceutical composition as defined above, wherein:

$X_1$ is any amino acid different from E or deleted, and/or
$X_2$ is any amino acid different from K, A, N, Y, R or deleted, and/or
$X_3$ is any amino acid different from F, P or deleted, and/or
$X_4$ is any amino acid different from L, V, I or deleted, and/or

$X_5$ is any amino acid different from Y, K, E, Q, R, A or deleted.

In another embodiment, the invention relates to a pharmaceutical composition as defined above, wherein:

$X_1$ is R, G, L, A, F or deleted, and/or
$X_2$ is R, G, L, A, F or deleted, and/or
$X_3$ is R, G, L, A, or deleted, and/or
$X_4$ is R, G, A, F or deleted, and/or
$X_5$ is R, G, L, A, F or deleted.

In another embodiment, the invention relates to a pharmaceutical composition as defined above, wherein:

$X_1$ is R, G, L, A, F or deleted, and $X_2$, $X_3$, $X_4$ and $X_5$ are any amino acid, or
$X_2$ is R, G, L, A, F or deleted, and $X_1$, $X_3$, $X_4$ and $X_5$ are any amino acid, or
$X_3$ is R, G, L, A or deleted, and $X_1$, $X_2$, $X_4$ and $X_5$ are any amino acid, or
$X_4$ is R, G, A, F or deleted, and $X_1$, $X_2$, $X_3$ and $X_5$ are any amino acid, or
$X_5$ is R, G, L, A, F or deleted, and $X_1$, $X_2$, $X_3$ and $X_4$ are any amino acid, or
$X_1$ is R, G, L, A, F or deleted, $X_2$ is R, G, L, A, F or deleted, and $X_3$, $X_4$ and $X_5$ are any amino acid, or
$X_1$ is R, G, L, A, F or deleted, $X_3$ is R, G, L, A or deleted, and $X_2$, $X_4$ and $X_5$ are any amino acid, or
$X_1$ is R, G, L, A, F or deleted, $X_4$ is R, G, A, F or deleted, and $X_2$, $X_3$ and $X_5$ are any amino acid, or
$X_1$ is R, G, L, A, F or deleted, $X_5$ is R, G, L, A, F or deleted, and $X_2$, $X_3$ and $X_4$ are any amino acid, or
$X_2$ is R, G, L, A, F or deleted, $X_3$ is R, G, L, A or deleted, and $X_1$, $X_4$ and $X_5$ are any amino acid, or
$X_2$ is R, G, L, A, F or deleted, $X_4$ is R, G, A, F or deleted, and $X_2$, $X_4$ and $X_5$ are any amino acid, or
$X_2$ is R, G, L, A, F or deleted, $X_5$ is R, G, L, A, F or deleted, and $X_2$, $X_3$ and $X_4$ are any amino acid, or
$X_3$ is R, G, L, A or deleted, $X_4$ is R, G, A, F or deleted, and $X_2$, $X_4$ and $X_5$ are any amino acid, or
$X_3$ is R, G, L, A or deleted, $X_5$ is R, G, L, A, F or deleted, and $X_2$, $X_3$ and $X_4$ are any amino acid, or
$X_4$ is R, G, A, F or deleted, $X_5$ is R, G, L, A, F or deleted and $X_2$, $X_4$ and $X_5$ are any amino acid, or
$X_1$ is R, G, L, A, F or deleted, $X_2$ is R, G, L, A, F or deleted, $X_3$ is R, G, L, A or deleted, and $X_4$ and $X_5$ are any amino acid, or
$X_1$ is any amino acid different from E or deleted, $X_2$ is R, G, L, A, F or deleted, $X_4$ is R, G, A, F or deleted, and $X_3$ and $X_5$ are any amino acid, or
$X_1$ is R, G, L, A, F or deleted, $X_2$ is R, G, L, A, F or deleted, $X_5$ is R, G, L, A, F or deleted and $X_3$ and $X_4$ are any amino acid, or
$X_1$ is R, G, L, A, F or deleted, $X_3$ is R, G, L, A or deleted, $X_4$ is R, G, A, F or deleted, and $X_2$ and $X_5$ are any amino acid, or
$X_1$ is R, G, L, A, F or deleted, $X_3$ is R, G, L, A or deleted, $X_5$ is R, G, L, A, F or deleted, and $X_2$ and $X_4$ are any amino acid, or
$X_1$ is R, G, L, A, F or deleted, $X_4$ is R, G, A, F or deleted, $X_5$ is R, G, L, A, F or deleted, and $X_2$ and $X_3$ are any amino acid, or
$X_2$ is R, G, L, A, F or deleted, $X_3$ is R, G, L, A or deleted, $X_4$ is R, G, A, F or deleted, and $X_1$ and $X_5$ are any amino acid, or
$X_2$ is R, G, L, A, F or deleted, $X_3$ is R, G, L, A or deleted, $X_5$ is R, G, L, A, F or deleted, and $X_1$ and $X_4$ are any amino acid, or
$X_2$ is R, G, L, A, F or deleted, $X_4$ is R, G, A, F or deleted, $X_5$ is R, G, L, A, F or deleted, and $X_1$ and $X_3$ are any amino acid, or
$X_3$ is R, G, L, A or deleted, $X_4$ is R, G, A, F or deleted, $X_5$ is R, G, L, A, F or deleted, and $X_1$ and $X_2$ are any amino acid, or
$X_1$ is R, G, L, A, F or deleted, $X_2$ is R, G, L, A, F or deleted, $X_3$ is R, G, L, A or deleted, $X_4$ is R, G, A, F or deleted, and $X_5$ is any amino acid, or
$X_1$ is R, G, L, A, F or deleted, $X_2$ is R, G, L, A, F or deleted, $X_3$ is R, G, L, A or deleted, $X_5$ is R, G, L, A, F or deleted, and $X_4$ is any amino acid, or
$X_1$ is R, G, L, A, F or deleted, $X_2$ is R, G, L, A, F or deleted, $X_4$ is R, G, A, F or deleted, $X_5$ is R, G, L, A, F or deleted, and $X_3$ is any amino acid, or
$X_1$ is R, G, L, A, F or deleted, $X_3$ is R, G, L, A or deleted, $X_4$ is R, G, A, F or deleted, $X_5$ is R, G, L, A, F or deleted, and $X_2$ is any amino acid, or
$X_2$ is R, G, L, A, F or deleted, $X_3$ is R, G, L, A or deleted, $X_4$ is R, G, A, F or deleted, $X_5$ is R, G, L, A, F or deleted, and $X_1$ is any amino acid, or
$X_1$ is R, G, L, A, F or deleted, $X_2$ is R, G, L, A, F or deleted, $X_3$ is R, G, L, A or deleted, $X_4$ is R, G, A, F or deleted and $X_5$ is R, G, L, A, F or deleted.

In another embodiment, the invention relates to a pharmaceutical composition as defined above, wherein said mutated immunosuppressive domain contains the amino acid sequence SEQ ID NO: 1 or SEQ ID NO: 3, wherein:

$X_1$ is A, G or R, and $X_2$, $X_3$, $X_4$ and $X_5$ are any amino acid, or
$X_2$ is A, G or R, and $X_1$, $X_3$, $X_4$ and $X_5$ are any amino acid, or
$X_3$ is A, G or R, and $X_1$, $X_2$, $X_4$ and $X_5$ are any amino acid, or
$X_4$ is A, G or R, and $X_1$, $X_2$, $X_3$ and $X_5$ are any amino acid, or
$X_5$ is A, G or R, and $X_1$, $X_2$, $X_3$ and $X_4$ are any amino acid,

in particular:

$X_1$ is R, and $X_2$, $X_3$, $X_4$ and $X_5$ are any amino acid, or
$X_2$ is R, and $X_1$, $X_3$, $X_4$ and $X_5$ are any amino acid, or
$X_3$ is R, and $X_1$, $X_2$, $X_4$ and $X_5$ are any amino acid, or
$X_4$ is R, and $X_1$, $X_2$, $X_3$ and $X_5$ are any amino acid, or
$X_5$ is R, and $X_1$, $X_2$, $X_3$ and $X_4$ are any amino acid.

In another embodiment, the invention relates to a pharmaceutical composition as defined above, wherein:

$X_1$ is R, G, L, A, F or deleted, and/or
$X_2$ is G, L, F or deleted, and/or
$X_3$ is R, G, L, A, or deleted, and/or
$X_4$ is R, G, A, F or deleted, and/or
$X_5$ is G, L, F or deleted.

In another embodiment, the invention relates to a pharmaceutical composition as defined above, wherein:

$X_3$ is A, G, L, R, C, D, E, H, K, M, N, Q, S, T, V, W, Y or deleted, $X_4$ is C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_4$ is A, F, G, R, C, D, E, H, K, M, N, P, Q, S, T, W, Y or deleted, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_3$ is A, G, L, R, C, D, E, H, K, M, N, Q, S, T, V, W, Y or deleted, $X_4$ is A, F, G, R, C, D, E, H, K, M, N, P, Q, S, T, W, Y or deleted, and $X_1$, $X_2$, $X_5$ are A, F, G, L, R, C, D, E, H, I, K, M, N, P, Q, S, T, V, W or Y.

In another embodiment, the invention relates to a pharmaceutical composition as defined above, wherein:

$X_3$ is R, G, L, A or deleted, $X_4$ is L, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_4$ is R, G, L, A, F or deleted, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_3$ is R, G, L, A or deleted, $X_4$ is R, G, A, F or deleted, and $X_1$, $X_2$, $X_5$ are A, F, G, L, R, C, D, E, H, I, K, M, N, P, Q, S, T, V, W or Y.

In another embodiment, the invention relates to a pharmaceutical composition as defined above, wherein:

$X_3$ is R, A or deleted, $X_4$ is L, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_4$ is R, A or deleted, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_3$ is R, A or deleted, $X_4$ is R, A or deleted, and $X_1$, $X_2$, $X_5$ are A, F, G, L, R, C, D, E, H, I, K, M, N, P, Q, S, T, V, W or Y.

In another embodiment, the invention relates to a pharmaceutical composition as defined above, wherein said mutated immunosuppressive domain contains the amino acid sequence SEQ ID NO: 1 or SEQ ID NO: 3, wherein:

$X_1$ is A, G or R, $X_2$ is A, G or R, and $X_3$, $X_4$ and $X_5$ are any amino acid, or
$X_1$ is A, G or R, $X_3$ is A, G or R, and $X_2$, $X_4$ and $X_5$ are any amino acid, or
$X_1$ is A, G or R, $X_4$ is A, G or R, and $X_2$, $X_3$ and $X_5$ are any amino acid, or
$X_1$ is A, G or R, $X_5$ is A, G or R, and $X_2$, $X_3$ and $X_4$ are any amino acid, or
$X_2$ is A, G or R, $X_3$ is A, G or R, and $X_1$, $X_4$ and $X_5$ are any amino acid, or

$X_2$ is A, G or R, $X_4$ is A, G or R, and $X_1$, $X_3$ and $X_5$ are any amino acid, or
$X_2$ is A, G or R, $X_5$ is A, G or R, and $X_1$, $X_3$ and $X_4$ are any amino acid, or
$X_3$ is A, G or R, $X_4$ is A, G or R, and $X_1$, $X_2$ and $X_5$ are any amino acid, or
$X_3$ is A, G or R, $X_5$ is A, G or R, and $X_1$, $X_2$ and $X_4$ are any amino acid, or
$X_4$ is A, G or R, $X_5$ is A, G or R, and $X_1$, $X_2$ and $X_3$ are any amino acid,

in particular:

$X_1$ is R, $X_2$ is R, and $X_3$, $X_4$ and $X_5$ are any amino acid, or
$X_1$ is R, $X_3$ is R, and $X_2$, $X_4$ and $X_5$ are any amino acid, or
$X_1$ is R, $X_4$ is R, and $X_2$, $X_3$ and $X_5$ are any amino acid, or
$X_1$ is R, $X_5$ is R, and $X_2$, $X_3$ and $X_4$ are any amino acid, or
$X_2$ is R, $X_3$ is R, and $X_1$, $X_4$ and $X_5$ are any amino acid, or
$X_2$ is R, $X_4$ is R, and $X_1$, $X_3$ and $X_5$ are any amino acid, or
$X_2$ is R, $X_5$ is R, and $X_1$, $X_3$ and $X_4$ are any amino acid, or
$X_3$ is R, $X_4$ is R, and $X_1$, $X_2$ and $X_5$ are any amino acid, or
$X_3$ is R, $X_5$ is R, and $X_1$, $X_2$ and $X_4$ are any amino acid, or
$X_4$ is R, $X_5$ is R, and $X_1$, $X_2$ and $X_3$ are any amino acid.

In another embodiment, the invention relates to a pharmaceutical composition as defined above, wherein said mutated immunosuppressive domain contains the amino acid sequence SEQ ID NO: 1 or SEQ ID NO: 3, wherein: $X_1$ is A, G or R, and $X_2$, $X_3$, $X_4$ and $X_5$ are any amino acid different from A, G or R, or
$X_2$ is A, G or R, and $X_1$, $X_3$, $X_4$ and $X_5$ are any amino acid different from A, G or R, or
$X_3$ is A, G or R, and $X_1$, $X_2$, $X_4$ and $X_5$ are any amino acid different from A, G or R, or
$X_4$ is A, G or R, and $X_1$, $X_2$, $X_3$ and $X_5$ are any amino acid different from A, G or R, or
$X_5$ is A, G or R, and $X_1$, $X_2$, $X_3$ and $X_4$ are any amino acid different from A, G or R,
in particular:

$X_1$ is R, and $X_2$, $X_3$, $X_4$ and $X_5$ are any amino acid different from A, G or R, or
$X_2$ is R, and $X_1$, $X_3$, $X_4$ and $X_5$ are any amino acid different from A, G or R, or
$X_3$ is R, and $X_1$, $X_2$, $X_4$ and $X_5$ are any amino acid different from A, G or R, or
$X_4$ is R, and $X_1$, $X_2$, $X_3$ and $X_5$ are any amino acid different from A, G or R, or
$X_5$ is R, and $X_1$, $X_2$, $X_3$ and $X_4$ are any amino acid different from A, G or R.

In another embodiment, the invention relates to a pharmaceutical composition as defined above, wherein said mutated immunosuppressive domain contains the amino acid sequence SEQ ID NO: 1 or SEQ ID NO: 3, wherein:

$X_1$ is A, G or R, $X_2$ is A, G or R, and $X_3$, $X_4$ and $X_5$ are any amino acid different from A, G or R, or
$X_1$ is A, G or R, $X_3$ is A, G or R, and $X_2$, $X_4$ and $X_5$ are any amino acid different from A, G or R, or
$X_1$ is A, G or R, $X_4$ is A, G or R, and $X_2$, $X_3$ and $X_5$ are any amino acid different from A, G or R, or
$X_1$ is A, G or R, $X_5$ is A, G or R, and $X_2$, $X_3$ and $X_4$ are any amino acid different from A, G or R, or
$X_2$ is A, G or R, $X_3$ is A, G or R, and $X_1$, $X_4$ and $X_5$ are any amino acid different from A, G or R, or
$X_2$ is A, G or R, $X_4$ is A, G or R, and $X_1$, $X_3$ and $X_5$ are any amino acid different from A, G or R, or
$X_2$ is A, G or R, $X_5$ is A, G or R, and $X_1$, $X_3$ and $X_4$ are any amino acid different from A, G or R, or
$X_3$ is A, G or R, $X_4$ is A, G or R, and $X_1$, $X_2$ and $X_5$ are any amino acid different from A, G or R, or
$X_3$ is A, G or R, $X_5$ is A, G or R, and $X_1$, $X_2$ and $X_4$ are any amino acid different from A, G or R, or
$X_4$ is A, G or R, $X_5$ is A, G or R, and $X_1$, $X_2$ and $X_3$ are any amino acid different from A, G or R,

in particular:

$X_1$ is R, $X_2$ is R, and $X_3$, $X_4$ and $X_5$ are any amino acid different from A, G or R, or
$X_1$ is R, $X_3$ is R, and $X_2$, $X_4$ and $X_5$ are any amino acid different from A, G or R, or
$X_1$ is R, $X_4$ is R, and $X_2$, $X_3$ and $X_5$ are any amino acid different from A, G or R, or
$X_1$ is R, $X_5$ is R, and $X_2$, $X_3$ and $X_4$ are any amino acid different from A, G or R, or
$X_2$ is R, $X_3$ is R, and $X_1$, $X_4$ and $X_5$ are any amino acid different from A, G or R, or
$X_2$ is R, $X_4$ is R, and $X_1$, $X_3$ and $X_5$ are any amino acid different from A, G or R, or
$X_2$ is R, $X_5$ is R, and $X_1$, $X_3$ and $X_4$ are any amino acid different from A, G or R, or
$X_3$ is R, $X_4$ is R, and $X_1$, $X_2$ and $X_5$ are any amino acid different from A, G or R, or

$X_3$ is R, $X_5$ is R, and $X_1$, $X_2$ and $X_4$ are any amino acid, different from A, G or R or
$X_4$ is R, $X_5$ is R, and $X_1$, $X_2$ and $X_3$ are any amino acid different from A, G or R.

In another embodiment, the invention relates to a pharmaceutical composition as defined above, wherein:

$X_3$ is R, $X_4$ is L, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_4$ is R, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or
$X_3$ is R, $X_4$ is R, and $X_1$, $X_2$, $X_5$ are A, F, G, L, R, C, D, E, H, I, K, M, N, P, Q, S, T, V, W or Y.

In another embodiment, the invention relates to a pharmaceutical composition as defined above, wherein:

$X_3$ is R, and $X_1$ is E, $X_2$ is K, $X_4$ is L, $X_5$ is Y, or
$X_4$ is R, and $X_1$ is E, $X_2$ is K, $X_3$ is F, $X_5$ is Y, or
$X_3$ is R, $X_4$ is R, and $X_1$ is E, $X_2$ is K, $X_5$ is Y.

In another embodiment, the invention relates to a pharmaceutical composition as defined above, wherein: $X_3$ is R, and $X_1$, $X_2$, $X_4$, $X_5$ are A, F, G, L, R, C, D, E, H, I, K, M, N, P, Q, S, T, V, W or Y.

In another embodiment, the invention relates to a pharmaceutical composition as defined above, wherein: $X_3$ is R, and $X_1$ is E, $X_2$ is K, $X_4$ is L, $X_5$ is Y.

In another embodiment, the invention relates to a pharmaceutical composition as defined above, wherein said mutated feline lentiviral ENV protein or fragment thereof comprising a mutated immunosuppressive domain (ISU) contains the following amino acid sequence:

A-[I/M/T/L]-[A/G/R]-K-[F/P]-[L/V/I]-Y-T-A (SEQ ID NO: 489), or
A-[I/M/T/L]-E-[A/G/R]-[F/P]-[L/V/I]-Y-T-A (SEQ ID NO: 490), or
A-[I/M/T/L]-E-K-[A/G/R]-[L/V/I]-Y-T-A (SEQ ID NO: 491), or
A-[I/M/T/L]-E-K-[F/P]-[A/G/R]-Y-T-A (SEQ ID NO: 492), or
A-[I/M/T/L]-E-K-[F/P]-[L/V/I]-[A/G/R]-T-A (SEQ ID NO: 493), or
A-[I/M/T/L]-E-K-[A/G/R]-[A/G/R]-Y-T-A (SEQ ID NO: 494),

in particular:

A-[I/M/T/L]-[A/G/R]-K-[F/P]-[L/V/I]-Y-T-A (SEQ ID NO: 489), or
A-[I/M/T/L]-E-G-[F/P]-[L/V/I]-Y-T-A (SEQ ID NO: 495), or
A-[I/M/T/L]-E-K-[A/G/R]-[L/V/I]-Y-T-A (SEQ ID NO: 491), or
A-[I/M/T/L]-E-K-[F/P]-[A/G/R]-Y-T-A (SEQ ID NO: 492), or
A-[I/M/T/L]-E-K-[F/P]-[L/V/I]-G-T-A (SEQ ID NO: 496), or
A-[I/M/T/L]-E-K-[A/G/R]-[A/G/R]-Y-T-A (SEQ ID NO: 494).

In another embodiment, the invention relates to a pharmaceutical composition as defined above, wherein said mutated feline lentiviral ENV protein or fragment thereof comprising a mutated immunosuppressive domain (ISU) contains the following amino acid sequence:

A-[I/M/T/L]-R-K-[F/P]-[L/V/I]-Y-T-A (SEQ ID NO: 497), or
A-[I/M/T/L]-E-R-[F/P]-[L/V/I]-Y-T-A (SEQ ID NO: 498), or
A-[I/M/T/L]-E-K-R-[L/V/I]-Y-T-A (SEQ ID NO: 499), or
A-[I/M/T/L]-E-K-[F/P]-R-Y-T-A (SEQ ID NO: 500), or
A-[I/M/T/L]-E-K-[F/P]-[L/V/I]-R-T-A (SEQ ID NO: 501), or
A-[I/M/T/L]-E-K-R-R-Y-T-A (SEQ ID NO: 502).

In another embodiment, the invention relates to a pharmaceutical composition as defined above, wherein said isolated mutated feline lentiviral ENV protein or said fragment thereof, comprises one of the amino acid sequences SEQ ID NO: 28 to 171 .

In the invention "SEQ ID NO: 28 to 171" encompasses SEQ ID NO: 28, 29, 30, 31 , 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170 and 171.

The correspondence is the following one:

| | |
|---|---|
| AIEKRLYTA | SEQ ID NO: 28 |
| AMEKRLYTA | SEQ ID NO: 29 |
| ATEKRLYTA | SEQ ID NO: 30 |
| ALEKRLYTA | SEQ ID NO: 31 |
| AIEKRVYTA | SEQ ID NO: 32 |
| AMEKRVYTA | SEQ ID NO: 33 |
| ATEKRVYTA | SEQ ID NO: 34 |
| ALEKRVYTA | SEQ ID NO: 35 |
| AIEKRIYTA | SEQ ID NO: 36 |
| AMEKRIYTA | SEQ ID NO: 37 |
| A TEKRIYTA | SEQ ID NO: 38 |
| ALEKRIYTA | SEQ ID NO: 39 |
| AIEKGLYTA | SEQ ID NO: 40 |
| AMEKGLYTA | SEQ ID NO: 41 |
| ATEKGLYTA | SEQ ID NO: 42 |
| ALEKGLYTA | SEQ ID NO: 43 |
| AIEKGVYTA | SEQ ID NO: 44 |
| AMEKGVYTA | SEQ ID NO: 45 |
| ATEKGVYTA | SEQ ID NO: 46 |
| ALEKGVYTA | SEQ ID NO: 47 |
| AIEKGIYTA | SEQ ID NO: 48 |
| AMEKGIYTA | SEQ ID NO: 49 |
| ATEKGIYTA | SEQ ID NO: 50 |
| ALEKGIYTA | SEQ ID NO: 51 |
| AIEKLLYTA | SEQ ID NO: 52 |
| AMEKLLYTA | SEQ ID NO: 53 |
| ATEKLLYTA | SEQ ID NO: 54 |
| ALEKLLYTA | SEQ ID NO: 55 |
| AIEKLVYTA | SEQ ID NO: 56 |
| AMEKLVYTA | SEQ ID NO: 57 |
| ATEKLVYTA | SEQ ID NO: 58 |
| ALEKLVYTA | SEQ ID NO: 59 |
| AIEKLIYTA | SEQ ID NO: 60 |
| AMEKLIYTA | SEQ ID NO: 61 |
| ATEKLIYTA | SEQ ID NO: 62 |
| ALEKLIYTA | SEQ ID NO: 63 |

(continued)

| | |
|---|---|
| AIEKALYTA | SEQ ID NO: 64 |
| AMEKALYTA | SEQ ID NO: 65 |
| ATEKALYTA | SEQ ID NO: 66 |
| ALEKALYTA | SEQ ID NO: 67 |
| AIEKAVYTA | SEQ ID NO: 68 |
| AMEKAVYTA | SEQ ID NO: 69 |
| ATEKAVYTA | SEQ ID NO: 70 |
| ALEKAVYTA | SEQ ID NO: 71 |
| AIEKAIYTA | SEQ ID NO: 72 |
| AMEKAIYTA | SEQ ID NO: 73 |
| ATEKAIYTA | SEQ ID NO: 74 |
| ALEKAIYTA | SEQ ID NO: 75 |
| AIEKFRYTA | SEQ ID NO: 76 |
| AMEKFRYTA | SEQ ID NO: 77 |
| ATEKFRYTA | SEQ ID NO: 78 |
| ALEKFRYTA | SEQ ID NO: 79 |
| AIEKPRYTA | SEQ ID NO: 80 |
| AMEKPRYTA | SEQ ID NO: 81 |
| ATEKPRYTA | SEQ ID NO: 82 |
| ALEKPRYTA | SEQ ID NO: 83 |
| AIEKFGYTA | SEQ ID NO: 84 |
| AMEKFGYTA | SEQ ID NO: 85 |
| ATEKFGYTA | SEQ ID NO: 86 |
| ALEKFGYTA | SEQ ID NO: 87 |
| AIEKPGYTA | SEQ ID NO: 88 |
| AMEKPGYTA | SEQ ID NO: 89 |
| ATEKPGYTA | SEQ ID NO: 90 |
| ALEKPGYTA | SEQ ID NO: 91 |
| AIEKFFYTA | SEQ ID NO: 92 |
| AMEKFFYTA | SEQ ID NO: 93 |
| ATEKFFYTA | SEQ ID NO: 94 |
| ALEKFFYTA | SEQ ID NO: 95 |
| AIEKPFYTA | SEQ ID NO: 96 |
| AMEKPFYTA | SEQ ID NO: 97 |
| ATEKPFYTA | SEQ ID NO: 98 |
| ALEKPFYTA | SEQ ID NO: 99 |
| AIEKFA YTA | SEQ ID NO: 100 |
| AMEKFA YTA | SEQ ID NO: 101 |
| ATEKFAYTA | SEQ ID NO: 102 |

(continued)

| | |
|---|---|
| ALEKFAYTA | SEQ ID NO: 103 |
| AIEKPAYTA | SEQ ID NO: 104 |
| AMEKPAYTA | SEQ ID NO: 105 |
| ATEKPAYTA | SEQ ID NO: 106 |
| ALEKPAYTA | SEQ ID NO: 107 |
| AIEKRRYTA | SEQ ID NO: 108 |
| AMEKRRYTA | SEQ ID NO: 109 |
| ATEKRRYTA | SEQ ID NO: 110 |
| ALEKRRYTA | SEQ ID NO: 111 |
| AIEKRAYTA | SEQ ID NO: 112 |
| AMEKRAYTA | SEQ ID NO: 113 |
| ATEKRAYTA | SEQ ID NO: 114 |
| ALEKRAYTA | SEQ ID NO: 115 |
| AIEKRFYTA | SEQ ID NO: 116 |
| AMEKRFYTA | SEQ ID NO: 117 |
| ATEKRFYTA | SEQ ID NO: 118 |
| ALEKRFYTA | SEQ ID NO: 119 |
| AIEKRGYTA | SEQ ID NO: 120 |
| AMEKRGYTA | SEQ ID NO: 121 |
| ATEKRGYTA | SEQ ID NO: 122 |
| ALEKRGYTA | SEQ ID NO: 123 |
| AIEKARYTA | SEQ ID NO: 124 |
| AMEKARYTA | SEQ ID NO: 125 |
| ATEKARYTA | SEQ ID NO: 126 |
| ALEKARYTA | SEQ ID NO: 127 |
| AIEKAA YTA | SEQ ID NO: 128 |
| AMEKAAYTA | SEQ ID NO: 129 |
| ATEKAAYTA | SEQ ID NO: 130 |
| ALEKAAYTA | SEQ ID NO: 131 |
| AIEKAFYTA | SEQ ID NO: 132 |
| AMEKAFYTA | SEQ ID NO: 133 |
| ATEKAFYTA | SEQ ID NO: 134 |
| ALEKAFYTA | SEQ ID NO: 135 |
| AIEKAGYTA | SEQ ID NO: 136 |
| AMEKAGYTA | SEQ ID NO: 137 |
| ATEKAGYTA | SEQ ID NO: 138 |
| ALEKAGYTA | SEQ ID NO: 139 |
| AIEKGRYTA | SEQ ID NO: 140 |
| AMEKGRYTA | SEQ ID NO: 141 |

(continued)

| | |
|---|---|
| ATEKGRYTA | SEQ ID NO: 142 |
| ALEKGRYTA | SEQ ID NO: 143 |
| AIEKGA YTA | SEQ ID NO: 144 |
| AMEKGAYTA | SEQ ID NO: 145 |
| ATEKGAYTA | SEQ ID NO: 146 |
| ALEKGAYTA | SEQ ID NO: 147 |
| AIEKGFYTA | SEQ ID NO: 148 |
| AMEKGFYTA | SEQ ID NO: 149 |
| ATEKGFYTA | SEQ ID NO: 150 |
| ALEKGFYTA | SEQ ID NO: 151 |
| AIEKGGYTA | SEQ ID NO: 152 |
| AMEKGGYTA | SEQ ID NO: 153 |
| ATEKGGYTA | SEQ ID NO: 154 |
| ALEKGGYTA | SEQ ID NO: 155 |
| AIEKFRYTA | SEQ ID NO: 156 |
| AMEKFRYTA | SEQ ID NO: 157 |
| ATEKFRYTA | SEQ ID NO: 158 |
| ALEKFRYTA | SEQ ID NO: 159 |
| AIEKFA YTA | SEQ ID NO: 160 |
| AMEKFA YTA | SEQ ID NO: 161 |
| ATEKFAYTA | SEQ ID NO: 162 |
| ALEKFAYTA | SEQ ID NO: 163 |
| AIEKFFYTA | SEQ ID NO: 164 |
| AMEKFFYTA | SEQ ID NO: 165 |
| A TEKFFYTA | SEQ ID NO: 166 |
| ALEKFFYTA | SEQ ID NO: 167 |
| AIEKFGYTA | SEQ ID NO: 168 |
| AMEKFGYTA | SEQ ID NO: 169 |
| ATEKFGYTA | SEQ ID NO: 170 |
| ALEKFGYTA | SEQ ID NO: 171 |

In another embodiment, the invention relates to a pharmaceutical composition as defined above, wherein said fragment of said isolated mutated feline lentiviral ENV protein comprises at least 40 amino acids, in particular at least 60 amino acids.

In another embodiment, the invention relates to a pharmaceutical composition as defined above, wherein said isolated mutated feline lentiviral ENV protein or said fragment thereof, comprises one of the amino acid sequences SEQ ID NO: 172 to 315.

In the invention "SEQ ID NO: 172 to 315" encompasses SEQ ID NO: 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314 and 315.

The correspondence is the following one:

| | |
|---|---|
| AIEKVTGALKINNLRLVTLEHQVLVIGLKVEAIEKRLYT AFAMQELGCNQNQFFCKIPLELWTR | SEQ ID NO: 172 |
| AIEKVTGALKINNLRLVTLEHQVLVIGLKVEAMEKRLY TAFAMQELGCNQNQFFCKIPLELWTR | SEQ ID NO: 173 |
| AIEKVTGALKINNLRLVTLEHQVLVIGLKVEATEKRLYT AFAMQELGCNQNQFFCKIPLELWTR | SEQ ID NO: 174 |
| AIEKVTGALKINNLRLVTLEHQVLVIGLKVEALEKRLYT AFAMQELGCNQNQFFCKIPLELWTR | SEQ ID NO: 175 |
| AIEKVTGALKINNLRLVTLEHQVLVIGLKVEAIEKRVYT AFAMQELGCNQNQFFCKIPLELWTR | SEQ ID NO: 176 |
| AIEKVTGALKINNLRLVTLEHQVLVIGLKVEAMEKRVY TAFAMQELGCNQNQFFCKIPLELWTR | SEQ ID NO: 177 |
| AIEKVTGALKINNLRLVTLEHQVLVIGLKVEATEKRVY TAFAMQELGCNQNQFFCKIPLELWTR | SEQ ID NO: 178 |
| AIEKVTGALKINNLRLVTLEHQVLVIGLKVEALEKRVY TAFAMQELGCNQNQFFCKIPLELWTR | SEQ ID NO: 179 |
| AIEKVTGALKINNLRLVTLEHQVLVIGLKVEAIEKRIYT AFAMQELGCNQNQFFCKIPLELWTR | SEQ ID NO: 180 |
| AIEKVTGALKINNLRLVTLEHQVLVIGLKVEAMEKRIYT AFAMQELGCNQNQFFCKIPLELWTR | SEQ ID NO: 181 |
| AIEKVTGALKINNLRLVTLEHQVLVIGLKVEATEKRIYT AFAMQELGCNQNQFFCKIPLELWTR | SEQ ID NO: 182 |
| AIEKVTGALKINNLRLVTLEHQVLVIGLKVEALEKRIYT AFAMQELGCNQNQFFCKIPLELWTR | SEQ ID NO: 183 |
| AIEKVTGALKINNLRLVTLEHQVLVIGLKVEAIEKGLYT AFAMQELGCNQNQFFCKIPLELWTR | SEQ ID NO: 184 |
| AIEKVTGALKINNLRLVTLEHQVLVIGLKVEAMEKGLY TAFAMQELGCNQNQFFCKIPLELWTR | SEQ ID NO: 185 |
| AIEKVTGALKINNLRLVTLEHQVLVIGLKVEATEKGLYT AFAMQELGCNQNQFFCKIPLELWTR | SEQ ID NO: 186 |
| AIEKVTGALKINNLRLVTLEHQVLVIGLKVEALEKGLYT AFAMQELGCNQNQFFCKIPLELWTR | SEQ ID NO: 187 |
| AIEKVTGALKINNLRLVTLEHQVLVIGLKVEAIEKGVYT AFAMQELGCNQNQFFCKIPLELWTR | SEQ ID NO: 188 |
| AAIEKVTGALKINNLRLVTLEHQVLVIGLKVEMEKGVY TAFAMQELGCNQNQFFCKIPLELWTR | SEQ ID NO: 189 |

(continued)

| | |
|---|---|
| AIEKVTGALKINNLRLVTLEHQVLVIGLKVEATEKGVY TAFAMQELGCNQNQFFCKIPLELWTR | SEQ ID NO: 190 |
| AIEKVTGALKINNLRLVTLEHQVLVIGLKVEALEKGVY TAFAMQELGCNQNQFFCKIPLELWTR | SEQ ID NO: 191 |
| AIEKVTGALKINNLRLVTLEHQVLVIGLKVEAIEKGIYT AFAMQELGCNQNQFFCKIPLELWTR | SEQ ID NO: 192 |
| AIEKVTGALKINNLRLVTLEHQVLVIGLKVEAMEKGIYT AFAMQELGCNQNQFFCKIPLELWTR | SEQ ID NO: 193 |
| AIEKVTGALKINNLRLVTLEHQVLVIGLKVEATEKGIYT AFAMQELGCNQNQFFCKIPLELWTR | SEQ ID NO: 194 |
| AIEKVTGALKINNLRLVTLEHQVLVIGLKVEALEKGIYT AFAMQELGCNQNQFFCKIPLELWTR | SEQ ID NO: 195 |
| AIEKVTGALKINNLRLVTLEHQVLVIGLKVEAIEKLLYT AFAMQELGCNQNQFFCKIPLELWTR | SEQ ID NO: 196 |
| AIEKVTGALKINNLRLVTLEHQVLVIGLKVEAMEKLLY TAFAMQELGCNQNQFFCKIPLELWTR | SEQ ID NO: 197 |
| AIEKVTGALKINNLRLVTLEHQVLVIGLKVEATEKLLYT AFAMQELGCNQNQFFCKIPLELWTR | SEQ ID NO: 198 |
| AIEKVTGALKINNLRLVTLEHQVLVIGLKVEALEKLLYT AFAMQELGCNQNQFFCKIPLELWTR | SEQ ID NO: 199 |
| AIEKVTGALKINNLRLVTLEHQVLVIGLKVEAIEKLVYT AFAMQELGCNQNQFFCKIPLELWTR | SEQ ID NO: 200 |
| AIEKVTGALKINNLRLVTLEHQVLVIGLKVEAMEKLVY TAFAMQELGCNQNQFFCKIPLELWTR | SEQ ID NO: 201 |
| AIEKVTGALKINNLRLVTLEHQVLVIGLKVEATEKLVYT AFAMQELGCNQNQFFCKIPLELWTR | SEQ ID NO: 202 |
| AIEKVTGALKINNLRLVTLEHQVLVIGLKVEALEKLVYT AFAMQELGCNQNQFFCKIPLELWTR | SEQ ID NO: 203 |
| AIEKVTGALKINNLRLVTLEHQVLVIGLKVEAIEKLIYT AFAMQELGCNQNQFFCKIPLELWTR | SEQ ID NO: 204 |
| AIEKVTGALKINNLRLVTLEHQVLVIGLKVEAMEKLIYT AFAMQELGCNQNQFFCKIPLELWTR | SEQ ID NO: 205 |
| AIEKVTGALKINNLRLVTLEHQVLVIGLKVEATEKLIYT AFAMQELGCNQNQFFCKIPLELWTR | SEQ ID NO: 206 |
| AIEKVTGALKINNLRLVTLEHQVLVIGLKVEALEKLIYT AFAMQELGCNQNQFFCKIPLELWTR | SEQ ID NO: 207 |

(continued)

| | |
|---|---|
| AIEKVTGALKINNLRLVTLEHQVLVIGLKVEAIEKALYT AFAMQELGCNQNQFFCKIPLELWTR | SEQ ID NO: 208 |
| AIEKVTGALKINNLRLVTLEHQVLVIGLKVEAMEKALY TAFAMQELGCNQNQFFCKIPLELWTR | SEQ ID NO: 209 |
| AIEKVTGALKINNLRLVTLEHQVLVIGLKVEATEKALYT AFAMQELGCNQNQFFCKIPLELWTR | SEQ ID NO: 210 |
| AIEKVTGALKINNLRLVTLEHQVLVIGLKVEALEKALYT AFAMQELGCNQNQFFCKIPLELWTR | SEQ ID NO: 211 |
| AIEKVTGALKINNLRLVTLEHQVLVIGLKVEAIEKAVYT AFAMQELGCNQNQFFCKIPLELWTR | SEQ ID NO: 212 |
| AIEKVTGALKINNLRLVTLEHQVLVIGLKVEAMEKAVY TAFAMQELGCNQNQFFCKIPLELWTR | SEQ ID NO: 213 |
| AIEKVTGALKINNLRLVTLEHQVLVIGLKVEATEKAVY TAFAMQELGCNQNQFFCKIPLELWTR | SEQ ID NO: 214 |
| AIEKVTGALKINNLRLVTLEHQVLVIGLKVEALEKAVY TAFAMQELGCNQNQFFCKIPLELWTR | SEQ ID NO: 215 |
| AIEKVTGALKINNLRLVTLEHQVLVIGLKVEAIEKAIYT AFAMQELGCNQNQFFCKIPLELWTR | SEQ ID NO: 216 |
| AIEKVTGALKINNLRLVTLEHQVLVIGLKVEAMEKAIYT AFAMQELGCNQNQFFCKIPLELWTR | SEQ ID NO: 217 |
| AIEKVTGALKINNLRLVTLEHQVLVIGLKVEATEKAIYT AFAMQELGCNQNQFFCKIPLELWTR | SEQ ID NO: 218 |
| AIEKVTGALKINNLRLVTLEHQVLVIGLKVEALEKAIYT AFAMQELGCNQNQFFCKIPLELWTR | SEQ ID NO: 219 |
| AIEKVTGALKINNLRLVTLEHQVLVIGLKVEAIEKFRYT AFAMQELGCNQNQFFCKIPLELWTR | SEQ ID NO: 220 |
| AIEKVTGALKINNLRLVTLEHQVLVIGLKVEAMEKFRY TAFAMQELGCNQNQFFCKIPLELWTR | SEQ ID NO: 221 |
| AIEKVTGALKINNLRLVTLEHQVLVIGLKVEATEKFRYT AFAMQELGCNQNQFFCKIPLELWTR | SEQ ID NO: 222 |
| AIEKVTGALKINNLRLVTLEHQVLVIGLKVEALEKFRYT AFAMQELGCNQNQFFCKIPLELWTR | SEQ ID NO: 223 |
| AIEKVTGALKINNLRLVTLEHQVLVIGLKVEAIEKPRYT AFAMQELGCNQNQFFCKIPLELWTR | SEQ ID NO: 224 |
| AIEKVTGALKINNLRLVTLEHQVLVIGLKVEAMEKPRY TAFAMQELGCNQNQFFCKIPLELWTR | SEQ ID NO: 225 |

(continued)

| | |
|---|---|
| AIEKVTGALKINNLRLVTLEHQVLVIGLKVEATEKPRYT AFAMQELGCNQNQFFCKIPLELWTR | SEQ ID NO: 226 |
| AIEKVTGALKINNLRLVTLEHQVLVIGLKVEALEKPRYT AFAMQELGCNQNQFFCKIPLELWTR | SEQ ID NO: 227 |
| AIEKVTGALKINNLRLVTLEHQVLVIGLKVEAIEKFGYT AFAMQELGCNQNQFFCKIPLELWTR | SEQ ID NO: 228 |
| AIEKVTGALKINNLRLVTLEHQVLVIGLKVEAMEKFGY TAFAMQELGCNQNQFFCKIPLELWTR | SEQ ID NO: 229 |
| AIEKVTGALKINNLRLVTLEHQVLVIGLKVEATEKFGYT AFAMQELGCNQNQFFCKIPLELWTR | SEQ ID NO: 230 |
| AIEKVTGALKINNLRLVTLEHQVLVIGLKVEALEKFGYT AFAMQELGCNQNQFFCKIPLELWTR | SEQ ID NO: 231 |
| AIEKVTGALKINNLRLVTLEHQVLVIGLKVEAIEKPGYT AFAMQELGCNQNQFFCKIPLELWTR | SEQ ID NO: 232 |
| AIEKVTGALKINNLRLVTLEHQVLVIGLKVEAMEKPGY TAFAMQELGCNQNQFFCKIPLELWTR | SEQ ID NO: 233 |
| AIEKVTGALKINNLRLVTLEHQVLVIGLKVEATEKPGYT AFAMQELGCNQNQFFCKIPLELWTR | SEQ ID NO: 234 |
| AIEKVTGALKINNLRLVTLEHQVLVIGLKVEALEKPGYT AFAMQELGCNQNQFFCKIPLELWTR | SEQ ID NO: 235 |
| AIEKVTGALKINNLRLVTLEHQVLVIGLKVEAIEKFFYT AFAMQELGCNQNQFFCKIPLELWTR | SEQ ID NO: 236 |
| AIEKVTGALKINNLRLVTLEHQVLVIGLKVEAMEKFFY TAFAMQELGCNQNQFFCKIPLELWTR | SEQ ID NO: 237 |
| AIEKVTGALKINNLRLVTLEHQVLVIGLKVEATEKFFYT AFAMQELGCNQNQFFCKIPLELWTR | SEQ ID NO: 238 |
| AIEKVTGALKINNLRLVTLEHQVLVIGLKVEALEKFFYT AFAMQELGCNQNQFFCKIPLELWTR | SEQ ID NO: 239 |
| AIEKVTGALKINNLRLVTLEHQVLVIGLKVEAIEKPFYT AFAMQELGCNQNQFFCKIPLELWTR | SEQ ID NO: 240 |
| AIEKVTGALKINNLRLVTLEHQVLVIGLKVEAMEKPFY TAFAMQELGCNQNQFFCKIPLELWTR | SEQ ID NO: 241 |
| AIEKVTGALKINNLRLVTLEHQVLVIGLKVEATEKPFYT AFAMQELGCNQNQFFCKIPLELWTR | SEQ ID NO: 242 |
| AIEKVTGALKINNLRLVTLEHQVLVIGLKVEALEKPFYT AFAMQELGCNQNQFFCKIPLELWTR | SEQ ID NO: 243 |

(continued)

| | |
|---|---|
| AIEKVTGALKINNLRLVTLEHQVLVIGLKVEAIEKFAYT AFAMQELGCNQNQFFCKIPLELWTR | SEQ ID NO: 244 |
| AIEKVTGALKINNLRLVTLEHQVLVIGLKVEAMEKFAY TAFAMQELGCNQNQFFCKIPLELWTR | SEQ ID NO: 245 |
| AIEKVTGALKINNLRLVTLEHQVLVIGLKVEATEKFAYT AFAMQELGCNQNQFFCKIPLELWTR | SEQ ID NO: 246 |
| AIEKVTGALKINNLRLVTLEHQVLVIGLKVEALEKFAYT AFAMQELGCNQNQFFCKIPLELWTR | SEQ ID NO: 247 |
| AIEKVTGALKINNLRLVTLEHQVLVIGLKVEAIEKPAYT AFAMQELGCNQNQFFCKIPLELWTR | SEQ ID NO: 248 |
| AIEKVTGALKINNLRLVTLEHQVLVIGLKVEAMEKPAY TAFAMQELGCNQNQFFCKIPLELWTR | SEQ ID NO: 249 |
| AIEKVTGALKINNLRLVTLEHQVLVIGLKVEATEKPAYT AFAMQELGCNQNQFFCKIPLELWTR | SEQ ID NO: 250 |
| AIEKVTGALKINNLRLVTLEHQVLVIGLKVEALEKPAYT AFAMQELGCNQNQFFCKIPLELWTR | SEQ ID NO: 251 |
| AIEKVTGALKINNLRLVTLEHQVLVIGLKVEAIEKRRYT AFAMQELGCNQNQFFCKIPLELWTR | SEQ ID NO: 252 |
| AIEKVTGALKINNLRLVTLEHQVLVIGLKVEAMEKRRY TAFAMQELGCNQNQFFCKIPLELWTR | SEQ ID NO: 253 |
| AIEKVTGALKINNLRLVTLEHQVLVIGLKVEATEKRRYT AFAMQELGCNQNQFFCKIPLELWTR | SEQ ID NO: 254 |
| AIEKVTGALKINNLRLVTLEHQVLVIGLKVEALEKRRYT AFAMQELGCNQNQFFCKIPLELWTR | SEQ ID NO: 255 |
| AIEKVTGALKINNLRLVTLEHQVLVIGLKVEAIEKRAYT AFAMQELGCNQNQFFCKIPLELWTR | SEQ ID NO: 256 |
| AIEKVTGALKINNLRLVTLEHQVLVIGLKVEAMEKRAY TAFAMQELGCNQNQFFCKIPLELWTR | SEQ ID NO: 257 |
| AIEKVTGALKINNLRLVTLEHQVLVIGLKVEATEKRAY TAFAMQELGCNQNQFFCKIPLELWTR | SEQ ID NO: 258 |
| AIEKVTGALKINNLRLVTLEHQVLVIGLKVEALEKRAY TAFAMQELGCNQNQFFCKIPLELWTR | SEQ ID NO: 259 |
| AIEKVTGALKINNLRLVTLEHQVLVIGLKVEAIEKRFYT AFAMQELGCNQNQFFCKIPLELWTR | SEQ ID NO: 260 |
| AIEKVTGALKINNLRLVTLEHQVLVIGLKVEAMEKRFY TAFAMQELGCNQNQFFCKIPLELWTR | SEQ ID NO: 261 |

(continued)

| | |
|---|---|
| AIEKVTGALKINNLRLVTLEHQVLVIGLKVEATEKRFYT AFAMQELGCNQNQFFCKIPLELWTR | SEQ ID NO: 262 |
| AIEKVTGALKINNLRLVTLEHQVLVIGLKVEALEKRFYT AFAMQELGCNQNQFFCKIPLELWTR | SEQ ID NO: 263 |
| AIEKVTGALKINNLRLVTLEHQVLVIGLKVEAIEKRGYT AFAMQELGCNQNQFFCKIPLELWTR | SEQ ID NO: 264 |
| AIEKVTGALKINNLRLVTLEHQVLVIGLKVEAMEKRGY TAFAMQELGCNQNQFFCKIPLELWTR | SEQ ID NO: 265 |
| AIEKVTGALKINNLRLVTLEHQVLVIGLKVEATEKRGY TAFAMQELGCNQNQFFCKIPLELWTR | SEQ ID NO: 266 |
| AIEKVTGALKINNLRLVTLEHQVLVIGLKVEALEKRGY TAFAMQELGCNQNQFFCKIPLELWTR | SEQ ID NO: 267 |
| AIEKVTGALKINNLRLVTLEHQVLVIGLKVEAIEKARYT AFAMQELGCNQNQFFCKIPLELWTR | SEQ ID NO: 268 |
| AIEKVTGALKINNLRLVTLEHQVLVIGLKVEAMEKARY TAFAMQELGCNQNQFFCKIPLELWTR | SEQ ID NO: 269 |
| AIEKVTGALKINNLRLVTLEHQVLVIGLKVEATEKARY TAFAMQELGCNQNQFFCKIPLELWTR | SEQ ID NO: 270 |
| AIEKVTGALKINNLRLVTLEHQVLVIGLKVEALEKARY TAFAMQELGCNQNQFFCKIPLELWTR | SEQ ID NO: 271 |
| AIEKVTGALKINNLRLVTLEHQVLVIGLKVEAIEKAAYT AFAMQELGCNQNQFFCKIPLELWTR | SEQ ID NO: 272 |
| AIEKVTGALKINNLRLVTLEHQVLVIGLKVEAMEKAAY TAFAMQELGCNQNQFFCKIPLELWTR | SEQ ID NO: 273 |
| AIEKVTGALKINNLRLVTLEHQVLVIGLKVEATEKAAY TAFAMQELGCNQNQFFCKIPLELWTR | SEQ ID NO: 274 |
| AIEKVTGALKINNLRLVTLEHQVLVIGLKVEALEKAAY TAFAMQELGCNQNQFFCKIPLELWTR | SEQ ID NO: 275 |
| AIEKVTGALKINNLRLVTLEHQVLVIGLKVEAIEKAFYT AFAMQELGCNQNQFFCKIPLELWTR | SEQ ID NO: 276 |
| AIEKVTGALKINNLRLVTLEHQVLVIGLKVEAMEKAFY TAFAMQELGCNQNQFFCKIPLELWTR | SEQ ID NO: 277 |
| AIEKVTGALKINNLRLVTLEHQVLVIGLKVEATEKAFYT AFAMQELGCNQNQFFCKIPLELWTR | SEQ ID NO: 278 |
| AIEKVTGALKINNLRLVTLEHQVLVIGLKVEALEKAFYT AFAMQELGCNQNQFFCKIPLELWTR | SEQ ID NO: 279 |

(continued)

| | |
|---|---|
| AIEKVTGALKINNLRLVTLEHQVLVIGLKVEAIEKAGYT AFAMQELGCNQNQFFCKIPLELWTR | SEQ ID NO: 280 |
| AIEKVTGALKINNLRLVTLEHQVLVIGLKVEAMEKAGY TAFAMQELGCNQNQFFCKIPLELWTR | SEQ ID NO: 281 |
| AIEKVTGALKINNLRLVTLEHQVLVIGLKVEATEKAGY TAFAMQELGCNQNQFFCKIPLELWTR | SEQ ID NO: 282 |
| AIEKVTGALKINNLRLVTLEHQVLVIGLKVEALEKAGY TAFAMQELGCNQNQFFCKIPLELWTR | SEQ ID NO: 283 |
| AIEKVTGALKINNLRLVTLEHQVLVIGLKVEAIEKGRYT AFAMQELGCNQNQFFCKIPLELWTR | SEQ ID NO: 284 |
| AIEKVTGALKINNLRLVTLEHQVLVIGLKVEAMEKGRY TAFAMQELGCNQNQFFCKIPLELWTR | SEQ ID NO: 285 |
| AIEKVTGALKINNLRLVTLEHQVLVIGLKVEATEKGRY TAFAMQELGCNQNQFFCKIPLELWTR | SEQ ID NO: 286 |
| AIEKVTGALKINNLRLVTLEHQVLVIGLKVEALEKGRY TAFAMQELGCNQNQFFCKIPLELWTR | SEQ ID NO: 287 |
| AIEKVTGALKINNLRLVTLEHQVLVIGLKVEAIEKGAYT AFAMQELGCNQNQFFCKIPLELWTR | SEQ ID NO: 288 |
| AIEKVTGALKINNLRLVTLEHQVLVIGLKVEAMEKGAY TAFAMQELGCNQNQFFCKIPLELWTR | SEQ ID NO: 289 |
| AIEKVTGALKINNLRLVTLEHQVLVIGLKVEATEKGAY TAFAMQELGCNQNQFFCKIPLELWTR | SEQ ID NO: 290 |
| AIEKVTGALKINNLRLVTLEHQVLVIGLKVEALEKGAY TAFAMQELGCNQNQFFCKIPLELWTR | SEQ ID NO: 291 |
| AIEKVTGALKINNLRLVTLEHQVLVIGLKVEAIEKGFYT AFAMQELGCNQNQFFCKIPLELWTR | SEQ ID NO: 292 |
| AIEKVTGALKINNLRLVTLEHQVLVIGLKVEAMEKGFY TAFAMQELGCNQNQFFCKIPLELWTR | SEQ ID NO: 293 |
| AIEKVTGALKINNLRLVTLEHQVLVIGLKVEATEKGFYT AFAMQELGCNQNQFFCKIPLELWTR | SEQ ID NO: 294 |
| AIEKVTGALKINNLRLVTLEHQVLVIGLKVEALEKGFYT AFAMQELGCNQNQFFCKIPLELWTR | SEQ ID NO: 295 |
| AIEKVTGALKINNLRLVTLEHQVLVIGLKVEAIEKGGYT AFAMQELGCNQNQFFCKIPLELWTR | SEQ ID NO: 296 |
| AIEKVTGALKINNLRLVTLEHQVLVIGLKVEAMEKGGY TAFAMQELGCNQNQFFCKIPLELWTR | SEQ ID NO: 297 |

(continued)

| | |
|---|---|
| AIEKVTGALKINNLRLVTLEHQVLVIGLKVEATEKGGYTAFAMQELGCNQNQFFCKIPLELWTR | SEQ ID NO: 298 |
| AIEKVTGALKINNLRLVTLEHQVLVIGLKVEALEKGGYTAFAMQELGCNQNQFFCKIPLELWTR | SEQ ID NO: 299 |
| AIEKVTGALKINNLRLVTLEHQVLVIGLKVEAIEKFRYTAFAMQELGCNQNQFFCKIPLELWTR | SEQ ID NO: 300 |
| AIEKVTGALKINNLRLVTLEHQVLVIGLKVEAMEKFRYTAFAMQELGCNQNQFFCKIPLELWTR | SEQ ID NO: 301 |
| AIEKVTGALKINNLRLVTLEHQVLVIGLKVEATEKFRYTAFAMQELGCNQNQFFCKIPLELWTR | SEQ ID NO: 302 |
| AIEKVTGALKINNLRLVTLEHQVLVIGLKVEALEKFRYTAFAMQELGCNQNQFFCKIPLELWTR | SEQ ID NO: 303 |
| AIEKVTGALKINNLRLVTLEHQVLVIGLKVEAIEKFAYTAFAMQELGCNQNQFFCKIPLELWTR | SEQ ID NO: 304 |
| AIEKVTGALKINNLRLVTLEHQVLVIGLKVEAMEKFAYTAFAMQELGCNQNQFFCKIPLELWTR | SEQ ID NO: 305 |
| AIEKVTGALKINNLRLVTLEHQVLVIGLKVEATEKFAYTAFAMQELGCNQNQFFCKIPLELWTR | SEQ ID NO: 306 |
| AIEKVTGALKINNLRLVTLEHQVLVIGLKVEALEKFAYTAFAMQELGCNQNQFFCKIPLELWTR | SEQ ID NO: 307 |
| AIEKVTGALKINNLRLVTLEHQVLVIGLKVEAIEKFFYTAFAMQELGCNQNQFFCKIPLELWTR | SEQ ID NO: 308 |
| AIEKVTGALKINNLRLVTLEHQVLVIGLKVEAMEKFFYTAFAMQELGCNQNQFFCKIPLELWTR | SEQ ID NO: 309 |
| AIEKVTGALKINNLRLVTLEHQVLVIGLKVEATEKFFYTAFAMQELGCNQNQFFCKIPLELWTR | SEQ ID NO: 310 |
| AIEKVTGALKINNLRLVTLEHQVLVIGLKVEALEKFFYTAFAMQELGCNQNQFFCKIPLELWTR | SEQ ID NO: 311 |
| AIEKVTGALKINNLRLVTLEHQVLVIGLKVEAIEKFGYTAFAMQELGCNQNQFFCKIPLELWTR | SEQ ID NO: 312 |
| AIEKVTGALKINNLRLVTLEHQVLVIGLKVEAMEKFGYTAFAMQELGCNQNQFFCKIPLELWTR | SEQ ID NO: 313 |
| AIEKVTGALKINNLRLVTLEHQVLVIGLKVEATEKFGYTAFAMQELGCNQNQFFCKIPLELWTR | SEQ ID NO: 314 |
| AIEKVTGALKINNLRLVTLEHQVLVIGLKVEALEKFGYTAFAMQELGCNQNQFFCKIPLELWTR | SEQ ID NO: 315 |

As mentioned above, the previous ENV proteins having their ISU comprising the above sequence have decreased immunosuppressive properties, substantially no immunosuppressive properties or no immunosuppressive properties.

Thus, in other words, any feline lentiviral Env protein having in their ISU an amino acid sequence comprising the sequences SEQ ID NO: 28 to 315, have decreased immunosuppressive properties, substantially no immunosuppressive properties or no immunosuppressive properties.

In other words, a feline lentiviral ENV protein comprising, within its ISU domain, an amino acid sequence selected from SEQ ID NO: 28 to 315 have decreased immunosuppressive properties, substantially no immunosuppressive properties or no immunosuppressive properties.

In the invention, the fragments of the mutated ENV proteins according to the invention have decreased immunosuppressive properties, substantially no immunosuppressive properties or no immunosuppressive properties. However, these fragments retain the structure and the antigenicity of the corresponding immunosuppressive domain that is not mutated, *i.e.* the wild type immunosuppressive domain.

In another embodiment, the invention relates to a pharmaceutical composition as defined above, wherein mutated feline lentiviral ENV protein or fragment thereof further comprises an additional mutation of one at least of the amino acids $X_1^*$, $X_2^*$, $X_3^*$, $X_4^*$ and $X_5^*$ in the following sequence:

$X_1^*$-$X_2^*$-$X_3^*$-$X_4^*$-$X_5^*$-[V/I]-[E/R]-A-[I/M/T/L]-$X_1$-$X_2$-$X_3$-$X_4$-$X_5$-T-A (SEQ ID NO: 316)
wherein

- $X_1$, $X_2$, $X_3$, $X_4$, $X_5$ are defined as above, and
- $X_1^*$ is either deleted, or is A, C, D, E, F, G, H, I, K, L, N, P, Q, R, S, Y or W, in particular A, F, G, L or R, and/or
- $X_2^*$ is either deleted, or is A, C, D, E, F, G, H, K, M, N, P, Q, R, S, T, V, Y or W, in particular A, F, G, or R, and/or
- $X_3^*$ is either deleted, or is A, C, D, E, F, L, H, I, K, M, N, P, Q, R, S, T, V, Y or W, in particular A, F, L or R, and/or
- $X_4^*$ is either deleted, or is A, C, D, E, F, G, H, I, K, M, N, P, Q, R, S, T, V, Y or W, in particular A, F, G, or R, and/or
- $X_5^*$ is either deleted, or is A, C, D, E, F, G, L, H, I, M, N, P, Q, S, V, Y or W, in particular A, F, G or L,

in association with a pharmaceutically acceptable carrier.

In such a pharmaceutical composition, the mutated feline lentiviral ENV protein, or fragment thereof, comprises a mutation of one at least of the amino acids $X_1$, $X_2$, $X_3$, $X_4$ and $X_5$ in association with an additional mutation of one at least of the amino acids $X_1^*$, $X_2^*$, $X_3^*$, $X_4^*$ and $X_5^*$.

In the invention, "*a mutation of one at least of the amino acids $X_1^*$, $X_2^*$, $X_3^*$, $X_4^*$ and $X_5^*$*" means that said mutated acids are:

- $X_1^*$ or $X_2^*$ or $X_3^*$ or $X_4^*$ or $X_5^*$ or
- $X_1^*$/$X_2^*$ or $X_1^*$/$X_3$ or $X_1^*$/$X_4$ or $X_1^*$/$X_5^*$ or $X_2^*$/$X_3^*$ or $X_2^*$/$X_4^*$ or $X_2^*$/$X_5^*$ or $X_3^*$/$X_4^*$ or $X_3^*$/$X_5^*$ or $X_4^*$/$X_5^*$ or
- $X_1^*$/$X_2^*$/$X_3^*$ or $X_1^*$/$X_2^*$/$X_4^*$ or $X_1^*$/$X_2^*$/$X_5^*$ or $X_1^*$/$X_3^*$/$X_4^*$ or $X_1^*$/$X_3^*$/$X_5^*$ or $X_1^*$/$X_4^*$/$X_5^*$ or $X_2^*$/$X_3^*$/$X_4^*$ or $X_2^*$/$X_3^*$/$X_5^*$ or $X_2^*$/$X_4^*$/$X_5^*$ or $X_3^*$/$X_4^*$/$X_5^*$ or
- $X_1^*$/$X_2^*$/$X_3^*$/$X_4^*$ or $X_1^*$/$X_2^*$/ $X_3^*$/$X_5^*$ or $X_1^*$/$X_2^*$/$X_4^*$/$X_5^*$ or $X_1^*$/$X_3^*$/$X_4^*$/$X_5^*$ or $X_2^*$/$X_3^*$/$X_4^*$/$X_5^*$ or
- $X_1^*$/$X_2^*$/$X_3^*$/$X_4^*$/$X_5^*$.

In another embodiment, the invention relates to a pharmaceutical composition as defined above, wherein mutated feline lentiviral ENV protein or fragment thereof further comprises an additional mutation of the amino acids $X_3^*$ in the following sequence:

[T/V/M]-[L/I]-$X_3^*$-L-[K/T/R]-[V/I]-[E/R]-A-[I/M/T/L]-$X_1$-$X_2$-$X_3$-$X_4$-$X_5$-T-A (SEQ ID NO: 434)
wherein

- $X_1$, $X_2$, $X_3$, $X_4$, $X_5$ are defined as above, and
- $X_3^*$ is either deleted, or is A, C, D, E, F, L, H, I, K, M, N, P, Q, R, S, T, V, Y or W, in particular A, F, L or R,

in association with a pharmaceutically acceptable carrier.

In such a pharmaceutical composition, the mutated feline lentiviral ENV protein, or fragment thereof, comprises a mutation of one at least of the amino acids $X_1$, $X_2$, $X_3$, $X_4$ and $X_5$ in association with an additional mutation of $X_3^*$.

The sequences SEQ ID NO: 316 and SEQ ID NO: 434 contain the following amino acid sequence A[I/M/T/L]$X_1X_2X_3X_4X_5$TA (SEQ ID NO: 1) elongated on its N-terminal end, in which an additional mutation is present.

In another aspect, the invention relates to a pharmaceutical composition comprising as active substance an isolated mutated feline lentiviral ENV protein having substantially no immunosuppressive activity, or a fragment thereof, said mutated feline lentiviral ENV protein resulting from mutation of the transmembrane (TM) subunit of a wild type feline lentiviral ENV protein,

said mutated feline lentiviral ENV protein or fragment thereof comprising a mutated immunosuppressive domain (ISU) containing the following amino acid sequence:
$X_1$*-$X_2$*-$X_3$*-$X_4$*-$X_5$*-[V/I]-[E/R]-A-[I/M/T/L]-E-K-[F/P]-[L/V/I]-Y-T-A (SEQ ID NO: 467)
wherein

- $X_1$* is either deleted, or is A, C, D, E, F, G, H, I, K, L, N, P, Q, R, S, T, V, Y or W, in particular A, F, G, L or R, and/or
- $X_2$* is either deleted, or is A, C, D, E, F, G, H, K, M, N, P, Q, R, S, T, V, Y or W, in particular A, F, G or R, and/or
- $X_3$* is either deleted, or is A, C, D, E, F, L, H, I, K, M, N, P, Q, R, S, T, V, Y or W, in particular A, F, L or R, and/or
- $X_4$* is either deleted, or is A, C, D, E, F, G, H, I, K, M, N, P, Q, R, S, T, V, Y or W, in particular A, F, G, or R, and/or
- $X_5$* is either deleted, or is A, C, D, E, F, G, L, H, I, M, N, P, Q, S, V, Y or W, in particular A, F, G, or L.

in association with a pharmaceutically acceptable carrier.

In another aspect, the invention relates to a pharmaceutical composition comprising as active substance an isolated mutated feline lentiviral ENV protein having substantially no immunosuppressive activity, or a fragment thereof, said mutated feline lentiviral ENV protein resulting from mutation of the transmembrane (TM) subunit of a wild type feline lentiviral ENV protein,

said mutated feline lentiviral ENV protein or fragment thereof comprising a mutated immunosuppressive domain (ISU) containing the following amino acid sequence:

[T/V/M] - [L/I] -$X_3$*-L-[K/T/R]-[V/I]-[E/R]-A-[I/M/T/L]-E-K-[F/P]-[L/V/I]-Y-T-A (SEQ ID NO: 468)
wherein

- $X_3$* is either deleted, or is A, C, D, E, F, L, H, I, K, M, N, P, Q, R, S, T, V, Y or W, in particular A, F, L or R,

in association with a pharmaceutically acceptable carrier.

In another advantageous embodiment, the invention relates to a pharmaceutical composition as defined above, wherein said mutated feline lentiviral protein, said variant or said fragment harbour a three-dimensional structure similar to the structure of the natural non mutated feline lentiviral ENV protein, non mutated variant or non fragment thereof. The skilled person knows how to measure the antigenicity, by using standard proceedings.

In another advantageous embodiment, the invention relates to a pharmaceutical composition as defined above, wherein said mutated feline lentiviral protein or said fragment thereof are expressed at the plasma membrane at a level substantially identical to the expression at the plasma membrane of the natural non mutated lentiviral ENV protein or non mutated fragment thereof.

The membrane expression of the lentiviral ENV protein according to the invention can be measured by any techniques allowing determination of a plasma membrane protein. For instance, cells can be transfected with an expression vector allowing the expression of the mutated lentiviral ENV protein according to the invention. Cells are then incubated with an antibody recognizing specifically the extracellular part of said lentiviral mutated ENV protein. The complex (antibody/ENV protein) is detected by another antibody, and the complex can be quantified by flow cytometry (see examples).

If no complex is detected, the mutated ENV protein is not expressed at the plasma membrane. On the contrary, if the complex is detected, this means that the mutated ENV protein is expressed at the plasma membrane and in an appropriate conformation so as to be detected by the antibody recognizing the extracellular part of the protein.

In another advantageous embodiment, the invention relates to a pharmaceutical composition as defined above, wherein said mutated feline lentiviral ENV protein is such that it has conserved, totally or partially, its fusogenic activity, which is responsible for virus-cell or cell-cell membrane fusion and can be measured by appropriate assays.

In another embodiment, the invention relates to a pharmaceutical composition as defined above, wherein said isolated mutated feline lentiviral ENV protein consists of one of the amino acid sequences: SEQ ID NO: 5 and SEQ ID NO: 317 to 342 and 374 to 419.

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**G**LYTAFAMQELGCNQNQFFC KIPLELWTRYNMTINQTIWNHGNITLGEWYNQTKDLQQK FYEIIMDIEQNNVQGKTGIQQLQKWEDWVRWIGNIPQYL KGLLGGILGIGLGVLLLILCLPTLVDCIRNCIHKILGYTVIA MPEVEGEEIQPQMELRRNGRQCGMSEKEEE | SEQ ID NO: 317 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**L**LYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 318 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**A**LYTAFAMQELGCNQNQFFCK IPLELWTRYNMTINQTIWNHGNITLGEWYNQTKDLQQKF YEIIMDIEQNNVQKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 319 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKF**R**YTAFAMQELGCNQNQFFCK IPLELWTRYNMTINQTIWNHGNITLGEWYNQTKDLQQKF YEIIMDIEQNNVQKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 320 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKF**G**YTAFAMQELGCNQNFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 321 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKF**A**YTAFAMQELGCNQNFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 322 |

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKF**F**YTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 323 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAM**R**KFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 324 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAM**G**KFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 325 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAM**L**KFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 326 |

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMAKFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 327 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMFKFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 328 |
| MAEGFAANRQWIGPEEAEELLDFDIATQMSEEGPLNPGV NPFRVPGITEKEKQNYCNILQPKLQDLRNEIQEVKLEEGN | SEQ ID NO: 329 |

(continued)

| | |
|---|---|
| AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAME**R**FLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEGFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 330 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMELFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 331 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEAFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 332 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEFFLYTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 333 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKFL**R**TAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 334 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKFLGTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 335 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKFLLTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 336 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKFL**A**TAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 337 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEKFL**F**TAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEEE | SEQ ID NO: 338 |

(continued)

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**RR**YTAFAMQELGCNQNQFFC KIPLELWTRYNMTINQTIWNHGNITLGEWYNQTKDLQQK FYEIIMDIEQNNVQGKTGIQQLQKWEDWVRWIGNIPQYL KGLLGGILGIGLGVLLLILCLPTLVDCIRNCIHKILGYTVIA MPEVEGEEIQPQMELRRNGRQCGMSEKEEE | SEQ ID NO: 339 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**RG**YTAFAMQELGCNQNQFFC KIPLELWTRYNMTINQTIWNHGNITLGEWYNQTKDLQQK FYEIIMDIEQNNVQGKTGIQQLQKWEDWVRWIGNIPQYL KGLLGGILGIGLGVLLLILCLPTLVDCIRNCIHKILGYTVIA MPEVEGEEIQPQMELRRNGRQCGMSEKEEE | SEQ ID NO: 340 |

| | |
|---|---|
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**RA**YTAFAMQELGCNQNQFFC KIPLELWTRYNMTINQTIWNHGNITLGEWYNQTKDLQQK FYEIIMDIEQNNVQGKTGIQQLQKWEDWVRWIGNIPQYL KGLLGGILGIGLGVLLLILCLPTLVDCIRNCIHKILGYTVIA MPEVEGEEIQPQMELRRNGRQCGMSEKEEE | SEQ ID NO: 341 |
| MAEGFAANRQWIGPEEAEELLDFDIATQMSEEGPLNPGV NPFRVPGITEKEKQNYCNILQPKLQDLRNEIQEVKLEEGN AGKFRRARFLRYSDERVLSLVHAFIGYCIYLGNRNKLGSL RHDIDIEAPQEECYNNREKGTTDNIKYGRRCCLGTVTLYLI LFTGVIVYSQTAGAQVVWRLPPLVVPVEESEIIFWDCWAP EEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNSCKWEEAKVKFHCQRTQSQPGSWFRAISSWKQ<br><br>RNRWEWRPDFKSKKVKISLPCNSTKNLTFAMRSSGDYGE VTGAWIEFGCHRNKSNLHTEARFRIRCRWNVGSDTSLIDT CGNTPNVSGANPVDCTMYSNKMYNCSLQNGFTMKVDDL IVHFNMTKAVEMYNIAGNWSCTSDLPSSWGYMNCNCTN SSSSYSGTKMACPSNRGILRNWYNPVAGLRQSLEQYQVV KQPDYLLVPEEVMEYKPRRKRAAIHVMLALATVLSIAGA GTGATAIGMVTQYHQVLATHQEAIEKVTGALKINNLRLV TLEHQVLVIGLKVEAMEK**RF**YTAFAMQELGCNQNQFFCK IPLELWTRYNMTINQTIWNHGNITLGEWYNQTKDLQQKF YEIIMDIEQNNVQGKTGIQQLQKWEDWVRWIGNIPQYLK GLLGGILGIGLGVLLLILCLPTLVDCIRNCIHKILGYTVIAM PEVEGEEIQPQMELRRNGRQCGMSEKEE | SEQ ID NO: 342 |

| | |
|---|---|
| MAEGFAANRQWIGLEEAEELLDFDIATQMSEEGPLNPGV NPFRVPGITEKEKQNYCNILQPKLQDLRNEIQEVKLEEGN AGKFRRARFLRYSDESVLSLVHAFIGYCIYLGNRNKLGSL RHDIDIEAPQEECYNNREKGTTDNIKYGRRCCLGTVTLYLI LFIGIIIYSQTTNAQVVWRLPPLVVPVEESEIIFWDCWAPEE PACQDFLGAMIHLKAKTNISIREGPTLGNWAREIWATLFK KATRQCRRGRIWKRWNETITGPSGCANNTCYNVSVIVPD YQCYLDRVDTWLQGKINISLCLTGGKMLYNKVTKQLSYC TDPLQIPLINYTFGPNQTCMWNTSQIQDPEIPKCGWWNQM AYYNSCKWEEAKVKFHCQRTQSQPGSWFRAISSWKQRN RWEWRPDFESKKVKISLQCNSTKNLTFAMRSSGDYGEVT GAWIEFGCHRNKSKLHAEARFRIRCRWNVGSNTSLIDTCG NTQKVSGANPVDCTMYSNKMYNCSLQNGFTMKVDDLIM HFNMKKAVEMYNIAGNWSCTSDLPSSWGYMNCNCTNSS SSYSGTKMACPSNRGILRNWYNPVAGLRQSLEQYQVVKQ PDYLVVPEEVMEYKPRRKRAAIHVMLALAAVLSIAGAGT GATAIGMVTQYHQVLATHQEAVEKVTEALKINNLRLVTL EHQVLVIGLKVEAMEK**R**LYTAFAMQELGCNQNQFFCKIP PELWTRYNMTINQTIWNHGNITLGEWYNQTKDLQQKFYE IIMDIEQNNVQGKKGIQQLQKWEDWVGWIGNIPQYLKGL LGGILGIGLGVLLLILCLPTLVDCIRNCIHKILGYTVIAMPE VEGEEIQPQMELRRNGRQCGMSEKEEE | SEQ ID NO: 374 |
| MAEGFAANRQWIGLEEAEELLDFDIATQMSEEGPLNPGV NPFRVPGITEKEKQNYCNILQPKLQDLRNEIQEVKLEEGN AGKFRRARFLRYSDESVLSLVHAFIGYCIYLGNRNKLGSL RHDIDIEAPQEECYNNREKGTTDNIKYGRRCCLGTVTLYLI LFIGIIIYSQTTNAQVVWRLPPLVVPVEESEIIFWDCWAPEE PACQDFLGAMIHLKAKTNISIREGPTLGNWAREIWATLFK KATRQCRRGRIWKRWNETITGPSGCANNTCYNVSVIVPD YQCYLDRVDTWLQGKINISLCLTGGKMLYNKVTKQLSYC TDPLQIPLINYTFGPNQTCMWNTSQIQDPEIPKCGWWNQM AYYNSCKWEEAKVKFHCQRTQSQPGSWFRAISSWKQRN RWEWRPDFESKKVKISLQCNSTKNLTFAMRSSGDYGEVT GAWIEFGCHRNKSKLHAEARFRIRCRWNVGSNTSLIDTCG NTQKVSGANPVDCTMYSNKMYNCSLQNGFTMKVDDLIM HFNMKKAVEMYNIAGNWSCTSDLPSSWGYMNCNCTNSS SSYSGTKMACPSNRGILRNWYNPVAGLRQSLEQYQVVKQ<br><br>PDYLVVPEEVMEYKPRRKRAAIHVMLALAAVLSIAGAGT GATAIGMVTQYHQVLATHQEAVEKVTEALKINNLRLVTL EHQVLVIGLKVEAMEKF**R**YTAFAMQELGCNQNQFFCKIP PELWTRYNMTINQTIWNHGNITLGEWYNQTKDLQQKFYE IIMDIEQNNVQGKKGIQQLQKWEDWVGWIGNIPQYLKGL LGGILGIGLGVLLLILCLPTLVDCIRNCIHKILGYTVIAMPE VEGEEIQPQMELRRNGRQCGMSEKEEE | SEQ ID NO: 375 |

(continued)

| | |
|---|---|
| MAEGFVANGQWIGPEEAEELVDFEIATQMNEEGPLNPGIN PFRVPGITKQEKQEYCSTMPKLQALRNEIQEVKLEEGNA GKFRRARFLRYSDETILSLIYLFIGYFRYLVDRKRFGSLRH DIDIEAPQEECYNNKEKGMTENIKYGKRCLVGTAALYLIL AIGIIIIRTTDAQVVWRLPPLVVPVEESEIIFWDCWAPEEPA CQDFLGAMIHLKASTNISNTEGPTLGNWAREIWATLFKKA TRQCRRGRIWKRWNETITGPIGCANNTCYNISVIVPDYQC YIDRVDTWLQGKVNISLCLTGGKMLYNKETKQLSYCTDP LQIPLINYTFGPNQTCMWNISQIQDPEIPKCGWWNQQAYY NNCKWERTDVKFQCQRTQSQPGSWIRAISSWKQGNRWE WRPDFESERVKVSLQCNSTRNLTFAMRSSGDYGEITGAWI EFGCHRNKSIRHNAARFRIRCRWNEGDNNSLIDTCGETQN VSGANPVDCTMYANKMYNCSLQDGFTMKVDDLIMHFN MTKAVEMYNIAGNWSCMSDLPTEWGYMNCNCTNDTSN NNTRKMKCPKENGILRNWYNPVAGLRQSLEKYQVVKQP DYLLVPEEVMEYKPRRKRAAIHVMLALATVLSMAGAGT GATAIGMVTQYHQVLATQQEAIEKVTEALKITNLRLVTLE HQVLVIGLKVEAMEK**R**LYTAFAMQELGCNQNQFFCKVPP ELWRRYNMTINQTIWNHGNITLGEWYNQTKDLQKKFYGI IMDIEQNNVQGKKGLQQLQKWEDWVGWIGNIPQYLKGL LGSIVGIGLGILLLILCLPTLVDCIRNCIHKILGYTVIAMPEV DGEEIQPQMELRRNGRQCGMSEKEEE | SEQ ID NO: 376 |
| MAEGFVANGQWIGPEEAEELVDFEIATQMNEEGPLNPGIN PFRVPGITKQEKQEYCSTMPKLQALRNEIQEVKLEEGNA GKFRRARFLRYSDETILSLIYLFIGYFRYLVDRKRFGSLRH DIDIEAPQEECYNNKEKGMTENIKYGKRCLVGTAALYLIL AIGIIIIRTTDAQVVWRLPPLVVPVEESEIIFWDCWAPEEPA CQDFLGAMIHLKASTNISNTEGPTLGNWAREIWATLFKKA TRQCRRGRIWKRWNETITGPIGCANNTCYNISVIVPDYQC YIDRVDTWLQGKVNISLCLTGGKMLYNKETKQLSYCTDP LQIPLINYTFGPNQTCMWNISQIQDPEIPKCGWWNQQAYY NNCKWERTDVKFQCQRTQSQPGSWIRAISSWKQGNRWE WRPDFESERVKVSLQCNSTRNLTFAMRSSGDYGEITGAWI EFGCHRNKSIRHNAARFRIRCRWNEGDNNSLIDTCGETQN VSGANPVDCTMYANKMYNCSLQDGFTMKVDDLIMHFN MTKAVEMYNIAGNWSCMSDLPTEWGYMNCNCTNDTSN NNTRKMKCPKENGILRNWYNPVAGLRQSLEKYQVVKQP DYLLVPEEVMEYKPRRKRAAIHVMLALATVLSMAGAGT GATAIGMVTQYHQVLATQQEAIEKVTEALKITNLRLVTLE HQVLVIGLKVEAMEKF**R**YTAFAMQELGCNQNQFFCKVPP ELWRRYNMTINQTIWNHGNITLGEWYNQTKDLQKKFYGI IMDIEQNNVQGKKGLQQLQKWEDWVGWIGNIPQYLKGL<br><br>LGSIVGIGLGILLLILCLPTLVDCIRNCIHKILGYTVIAMPEV DGEEIQPQMELRRNGRQCGMSEKEEE | SEQ ID NO: 377 |

(continued)

| | |
|---|---|
| MAEGFVANGQWIGPEEAEELLDFDIATQLNEEGPLNPGIN PFRVPGITQKEKQEYCNTLQPKLQALRNEIQEVKLEERNA GKFRRARFLRYSDETILSLIYSFVGYFRYLVDRKKFGSLRH DIDIEAPQEEYYNNKEKGMTDNVKYGKRCLVGTAAFYLL LAIGIIIIRTVDAQVVWRLPPLVVPVEESEIIFWDCWAPEEP ACQDFLGAMIHLKASTNIRIQEGPTLGNWAREIWATLFKK ATRQCRRGRIWKRWNETITGPIGCANNTCYNISVIIPDYQC YIDRVDTWLQGKVNISICLTGGKMLYNKETKQLSYCTDP LQIPLINYTFGPQQTCMWNTSQIQDPEIPKCGWWNQKAY YNQCSWEQTDVKFQCQRTQSQPGSWIRAISSWRQRNRWE WRPDFESERVKVSLQCNSTQNLTFAMRSSGDYGEITGAWI EFGCHRNKSKHHNEARFRIRCRWNEGNNNSLIDTCGKTQ NVLGANPVDCTMYANRMYNCSLQDGFTMKVDDLIMHF NMTKAVEMYNIAGNWSCMSDLPTNWGYMKCNCTNDTS NNHTIKMECPEEKGILRNWYNPVAGLRQSLEKYQVVKQP DYLVVPGEVMEYKPRRKRAAIHVMLALATVLSMAGAGT GATAIGMVTQYHQVLATHQEAIEKVTEALKINNLRLVTLE HQVLVIGLKVEAIEK**R**LYTAFAMQELGCNQNQFFCKVPL ELWRRYNMTINQTIWNHGNITLGEWYNQTKALQHKFYEI IMDIEQNNVQGKKGLQQLQKWEDWVGWIGNIPQYLKGL LGGILGIGLGILLLILCLPTLVDCIRNCIHKILGYTVIAMPDV DEEEIQPQMELRRNGRQCGMSEKEEE | SEQ ID NO: 378 |
| MAEGFVANGQWIGPEEAEELLDFDIATQLNEEGPLNPGIN PFRVPGITQKEKQEYCNTLQPKLQALRNEIQEVKLEERNA GKFRRARFLRYSDETILSLIYSFVGYFRYLVDRKKFGSLRH DIDIEAPQEEYYNNKEKGMTDNVKYGKRCLVGTAAFYLL LAIGIIIIRTVDAQVVWRLPPLVVPVEESEIIFWDCWAPEEP ACQDFLGAMIHLKASTNIRIQEGPTLGNWAREIWATLFKK ATRQCRRGRIWKRWNETITGPIGCANNTCYNISVIIPDYQC YIDRVDTWLQGKVNISICLTGGKMLYNKETKQLSYCTDP LQIPLINYTFGPQQTCMWNTSQIQDPEIPKCGWWNQKAY YNQCSWEQTDVKFQCQRTQSQPGSWIRAISSWRQRNRWE WRPDFESERVKVSLQCNSTQNLTFAMRSSGDYGEITGAWI EFGCHRNKSKHHNEARFRIRCRWNEGNNNSLIDTCGKTQ NVLGANPVDCTMYANRMYNCSLQDGFTMKVDDLIMHF NMTKAVEMYNIAGNWSCMSDLPTNWGYMKCNCTNDTS NNHTIKMECPEEKGILRNWYNPVAGLRQSLEKYQVVKQP DYLVVPGEVMEYKPRRKRAAIHVMLALATVLSMAGAGT GATAIGMVTQYHQVLATHQEAIEKVTEALKINNLRLVTLE HQVLVIGLKVEAIEKF**R**YTAFAMQELGCNQNQFFCKVPL ELWRRYNMTINQTIWNHGNITLGEWYNQTKALQHKFYEI IMDIEQNNVQGKKGLQQLQKWEDWVGWIGNIPQYLKGL LGGILGIGLGILLLILCLPTLVDCIRNCIHKILGYTVIAMPDV DEEEIQPQMELRRNGRQCGMSEKEEE | SEQ ID NO: 379 |

(continued)

| | |
|---|---|
| MAEGFAANRQWIGPEEAEELLDFDIAIQMNEEGPLNPGVN PFRVPGITEAEKQEYCNILQPKLQDLKGKIQEVKLEEGNA GKFRRARFLRYSDETVLSLIHLFIGYCPHLCRRHELGSLRH DIDIEALQEERYNDREKGITDNIKYGKRCLIGTAVLYLLLS LGIIIHTCKAQVVWRLPPLVVPVEESEIIFWDCWAPEEPAC QDFLGAMIHLKASTNISIQEGPTLGNWAREIWGTLFKKAT RQCRRGRIWRRWNETITGPLGCANNTCYNISVIVPDYQCY LDRVDTWLQGKVNISLCLTGGKMLYNKETKQLSYCTDPL QIPLINYTFGPNQTCMWNTSQIQDPEIPKCGWWNQNAYY NSCRWEHTDVQFQCQRTQSQPGSWIRAISSWKQRNRWE WRPDFESEKVKVSLQCNSTKNLTFAMRSSGDYGEVTGA WIEFGCHRTKSKYHTEARFRIRCRWNVGDNTSLIDTCGET QNVSRANPVDCTMYANRMYNCSLQNGFTMKVDDLIMHF NKTKAVEMYNIAGNWSCKSDLPPTWGYMNCNCTNSTNS GTGIRMACPRNQGILRNWYNPVAGLRQSLEKYQVVKQPD YLVVPGEVMEYKPRRKRAAIHVMLALATVLSMAGAGTG ATAIGMVTQYQQVLATHQEAIEKVTEALKINNLRLVTLEH QVLVIGLKVEAMEK**R**LYTAFAMQELGCNQNQFFCKVPSA LWERYNMTINQTIWNHGNITLGEWYNQTKDLQQRFYEII MDIEQNNVQGKKGLQQLQEWEDWVGWIGNIPQYLKGLL GGILGIGLGMLLLILCLPTLVDCIRNCIHKILGYTVIAMPEV EEEEIQPQMELRRNGRQCGMSEKEEE | SEQ ID NO: 380 |
| MAEGFAANRQWIGPEEAEELLDFDIAIQMNEEGPLNPGVN PFRVPGITEAEKQEYCNILQPKLQDLKGKIQEVKLEEGNA GKFRRARFLRYSDETVLSLIHLFIGYCPHLCRRHELGSLRH DIDIEALQEERYNDREKGITDNIKYGKRCLIGTAVLYLLLS LGIIIHTCKAQVVWRLPPLVVPVEESEIIFWDCWAPEEPAC QDFLGAMIHLKASTNISIQEGPTLGNWAREIWGTLFKKAT RQCRRGRIWRRWNETITGPLGCANNTCYNISVIVPDYQCY LDRVDTWLQGKVNISLCLTGGKMLYNKETKQLSYCTDPL QIPLINYTFGPNQTCMWNTSQIQDPEIPKCGWWNQNAYY NSCRWEHTDVQFQCQRTQSQPGSWIRAISSWKQRNRWE WRPDFESEKVKVSLQCNSTKNLTFAMRSSGDYGEVTGA WIEFGCHRTKSKYHTEARFRIRCRWNVGDNTSLIDTCGET QNVSRANPVDCTMYANRMYNCSLQNGFTMKVDDLIMHF NKTKAVEMYNIAGNWSCKSDLPPTWGYMNCNCTNSTNS GTGIRMACPRNQGILRNWYNPVAGLRQSLEKYQVVKQPD YLVVPGEVMEYKPRRKRAAIHVMLALATVLSMAGAGTG ATAIGMVTQYQQVLATHQEAIEKVTEALKINNLRLVTLEH QVLVIGLKVEAMEKF**R**YTAFAMQELGCNQNQFFCKVPSA LWERYNMTINQTIWNHGNITLGEWYNQTKDLQQRFYEII MDIEQNNVQGKKGLQQLQEWEDWVGWIGNIPQYLKGLL GGILGIGLGMLLLILCLPTLVDCIRNCIHKILGYTVIAMPEV EEEEIQPQMELRRNGRQCGMSEKEEE | SEQ ID NO: 381 |

(continued)

| | |
|---|---|
| MAEGFAANRQWIGPEEAEELLDFDIATQMSEEGPLNPGV NPFRVPGITEKEKQNYCNILQPKLQDLRDEIQEVKLEEGN AGKFRRTRFLRYSDEHVLSLVHAFIGYCIYLGNRNKLGSL RHDIDIEAPQEEYYNNREKGTTDNIKYGRRCCLGTVTLYL ILFIGVIVYSQTAGAQVVWRLPPLVVPVEESEIIFWDCWAP DEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNRCKWEEAKVKFHCQRTQSQPGSWRRAISSWKQ RNRWEWRPDLESEKVKISLQCNSKKNLTFAMRSSGDYGE VTGAWIEFGCHRNKSKHHSEARFRIRCRWNVGSNTSLIDT CGNTQDVSGANPVDCTMYSNKMYNCSLQNGFTMKVDD LIVHFSMTKAVKMYNIAGNWSCTSDLPSSWGYMNCNCT NSSSSYSGTKMACPSNRGILRNWYNPVAGLRQSLEQYQV VKQPDYLVVPEEVMEYKPRRKRAAIHVMLALATVLSIAG AGTGATAIGMVTQYHQVLATHQEAIEKVTEALKINNLRL VTLEHQVLVIGLKVEAMEK**R**LYTAFAMQELGCNQNQFFC KIPPGLWTRYNMTINQTIWNHGNITLGEWYNKTKDLQQK FYEIIMDIEQNNVQGKTGIQQLQKWEDWVGWIGNIPQYL KGLLGGILGIGLGVLLLILCLPTLVDCIRNCIHKILGYTVIA MPEVEGEEIQPQMELRRNGRQCGMSEKEEE | SEQ ID NO: 382 |
| MAEGFAANRQWIGPEEAEELLDFDIATQMSEEGPLNPGV NPFRVPGITEKEKQNYCNILQPKLQDLRDEIQEVKLEEGN AGKFRRTRFLRYSDEHVLSLVHAFIGYCIYLGNRNKLGSL RHDIDIEAPQEEYYNNREKGTTDNIKYGRRCCLGTVTLYL ILFIGVIVYSQTAGAQVVWRLPPLVVPVEESEIIFWDCWAP DEPACQDFLGAMIHLKAKTNISIREGPTLGNWAREIWATL FKKATRQCRRGRIWKRWNETITGPSGCANNTCYNVSVIV PDYQCYLDRVDTWLQGKINISLCLTGGKMLYNKVTKQLS YCTDPLQIPLINYTFGPNQTCMWNTSQIQDPEIPKCGWWN QMAYYNRCKWEEAKVKFHCQRTQSQPGSWRRAISSWKQ RNRWEWRPDLESEKVKISLQCNSKKNLTFAMRSSGDYGE VTGAWIEFGCHRNKSKHHSEARFRIRCRWNVGSNTSLIDT CGNTQDVSGANPVDCTMYSNKMYNCSLQNGFTMKVDD LIVHFSMTKAVKMYNIAGNWSCTSDLPSSWGYMNCNCT NSSSSYSGTKMACPSNRGILRNWYNPVAGLRQSLEQYQV VKQPDYLVVPEEVMEYKPRRKRAAIHVMLALATVLSIAG AGTGATAIGMVTQYHQVLATHQEAIEKVTEALKINNLRL VTLEHQVLVIGLKVEAMEKF**R**YTAFAMQELGCNQNQFFC KIPPGLWTRYNMTINQTIWNHGNITLGEWYNKTKDLQQK FYEIIMDIEQNNVQGKTGIQQLQKWEDWVGWIGNIPQYL KGLLGGILGIGLGVLLLILCLPTLVDCIRNCIHKILGYTVIA MPEVEGEEIQPQMELRRNGRQCGMSEKEEE | SEQ ID NO: 383 |

| | |
|---|---|
| MAKGFAANRQWIGPEEAEELLDFDIATQMNEEGPLNPGV NPFRVPGITEQEKQDYCNILQPKLQELRNEIQEVKLEEGNA GKFRRARFLRYSDETILSLIHLFIGYCTYLCKRNELGSLRH DIDIEAPQEECYNNKEKGTTNNIKYGGRCFIGTMIMYLLIFI GIIIYIQTTEAQVVWRLPPLIVPVKESEIIFWDCWAPEEPAC QDFLGAMIHLKASTNISIQEGPTLGNWAREIWGTLFKKAT RQCRRGRIWKRWNETITGPLGCANNTCYNISVIVPDYQCY LDRVDTWLQGKVNISLCLTGGKMLYNKDTKQLSYCTDPL QIPLINYTFGPNQTCMWNTSQIQDPEIPKCGWWNQIAYYN SCKWEKTDVKFHCQRTQSQPGSWIRAISSWRQRNRWEW RPDFESEKVKVSLQCNSTKNLTFAMRSSGDYGEVTGAWI EFGCHRKKSKLHTEARFRIRCRWNVGNNASLIDTCGNTPD VSGANPVNCTMYANKMYNCWLQNGFTIKVDDLIMHFN MTKAVEMYNIAGNWSCTSDLPPTWGYMKCNCTNNSDDT RGKMACPRTQGILRNWYNPVAGLRQSLEKYQVVKQPDY LVVPGEVMEYKPRRKRAAIHVMLALATVLSMAGAGTGA TAIGMVTQYHQVLATHQEAIEKVTEALKINNLRLVTLEHQ VLVIGLKVEAMEK**R**LYTAFAMQELGCNQNQFFCKVPPQL WKRYNMTINQTIWNHGNITLGEWYNQTKDLQQKFYEIIM DIEQNNVQGKTGIQQLQRWEDWVGWIGNIPQYLKGLLGG ILGIGLGVLLLILCLPTLVDCIRNCIHKILGYTVIAMPEVEEE EIQQQMELRRNGRQCGMSEKEEE | SEQ ID NO: 384 |
| MAKGFAANRQWIGPEEAEELLDFDIATQMNEEGPLNPGV NPFRVPGITEQEKQDYCNILQPKLQELRNEIQEVKLEEGNA GKFRRARFLRYSDETILSLIHLFIGYCTYLCKRNELGSLRH DIDIEAPQEECYNNKEKGTTNNIKYGGRCFIGTMIMYLLIFI GIIIYIQTTEAQVVWRLPPLIVPVKESEIIFWDCWAPEEPAC QDFLGAMIHLKASTNISIQEGPTLGNWAREIWGTLFKKAT RQCRRGRIWKRWNETITGPLGCANNTCYNISVIVPDYQCY LDRVDTWLQGKVNISLCLTGGKMLYNKDTKQLSYCTDPL QIPLINYTFGPNQTCMWNTSQIQDPEIPKCGWWNQIAYYN SCKWEKTDVKFHCQRTQSQPGSWIRAISSWRQRNRWEW RPDFESEKVKVSLQCNSTKNLTFAMRSSGDYGEVTGAWI EFGCHRKKSKLHTEARFRIRCRWNVGNNASLIDTCGNTPD VSGANPVNCTMYANKMYNCWLQNGFTIKVDDLIMHFN MTKAVEMYNIAGNWSCTSDLPPTWGYMKCNCTNNSDDT RGKMACPRTQGILRNWYNPVAGLRQSLEKYQVVKQPDY LVVPGEVMEYKPRRKRAAIHVMLALATVLSMAGAGTGA TAIGMVTQYHQVLATHQEAIEKVTEALKINNLRLVTLEHQ VLVIGLKVEAMEKF**R**YTAFAMQELGCNQNQFFCKVPPQL WKRYNMTINQTIWNHGNITLGEWYNQTKDLQQKFYEIIM DIEQNNVQGKTGIQQLQRWEDWVGWIGNIPQYLKGLLGG ILGIGLGVLLLILCLPTLVDCIRNCIHKILGYTVIAMPEVEEE EIQQQMELRRNGRQCGMSEKEEE | SEQ ID NO: 385 |

(continued)

| | |
|---|---|
| MAEGFAANRQWIGPEEAEELLDFDIATQMNEEGPLNPGIN PFRVPGITEIEKQAYCKILPRLQALRNEIQEVKLEEGNAG KFRRARFLRYSDVAILSLIHVFIGYCTYLCNQQKLGSLRHD IDIEAPQEEYYSNNEKGTTDNIKYGRRCVIGTVALYLLICT GIIIYTRTATAQVVWRLPPLVVPVEESEVIFWDCWAPEEPA CQDFLGAMIHLKASTNISIQEGPTLGNWAREIWGTLFKKA TRQCRRGRIWRRWNETITGPLGCANNTCYNISVIVPDYQC YLDRVDTWLQGKINISLCLTGGKMLYNRYTKQLSYCTDP LQIPLINYTFGPNQTCMWNTSQIQDPEIPKCGWWNQRAY YNSCRWESTDVKFHCQRTQSQPGSWLRAISSWRQRNRW EWRPDFESEKVKVSLQCNSTKNLTFAMRSSGDYGDVTGA WIEFGCHRNKSRLHTEARFRIRCRWNVGDNTSLIDTCGKT QNIAGANPVDCTMYVNRMYNCSLQNGFTMKVDDLIMHF NMTKAVEMYNIAGNWSCTSNLPPTWGYINCNCTNSSDSN KMACPSSQGILRNWYNPVAGLRQSLEKYQVVKQPDYLV VPGEVMEYKPRRKRAAIHVMLALATILSMAGAGTGATAI GMVTQYHQVLATHQEAIEKVTEALKVNNLRLITLEHQVL VIGLKVEAMEK**R**LYTAFAMQELGCNQNQFFCKVPPELW<br><br>KRYNMTINQTIWNHGNITLGEWYNQTKGLQQKFYEIIMDI EQNSVQGKKGIQQLQEWEDWIGWIGNIPQYLKGLLGGIL GIGLGVLLLILCLPTLVDCIRNCIHKILGYTVIAMPEVEGEE IQPQMELRRNGRQCGMSEKEEE | SEQ ID NO: 386 |
| MAEGFAANRQWIGPEEAEELLDFDIATQMNEEGPLNPGIN PFRVPGITEIEKQAYCKILPRLQALRNEIQEVKLEEGNAG KFRRARFLRYSDVAILSLIHVFIGYCTYLCNQQKLGSLRHD IDIEAPQEEYYSNNEKGTTDNIKYGRRCVIGTVALYLLICT GIIIYTRTATAQVVWRLPPLVVPVEESEVIFWDCWAPEEPA CQDFLGAMIHLKASTNISIQEGPTLGNWAREIWGTLFKKA TRQCRRGRIWRRWNETITGPLGCANNTCYNISVIVPDYQC YLDRVDTWLQGKINISLCLTGGKMLYNRYTKQLSYCTDP LQIPLINYTFGPNQTCMWNTSQIQDPEIPKCGWWNQRAY YNSCRWESTDVKFHCQRTQSQPGSWLRAISSWRQRNRW EWRPDFESEKVKVSLQCNSTKNLTFAMRSSGDYGDVTGA WIEFGCHRNKSRLHTEARFRIRCRWNVGDNTSLIDTCGKT QNIAGANPVDCTMYVNRMYNCSLQNGFTMKVDDLIMHF NMTKAVEMYNIAGNWSCTSNLPPTWGYINCNCTNSSDSN KMACPSSQGILRNWYNPVAGLRQSLEKYQVVKQPDYLV VPGEVMEYKPRRKRAAIHVMLALATILSMAGAGTGATAI GMVTQYHQVLATHQEAIEKVTEALKVNNLRLITLEHQVL VIGLKVEAMEKF**R**YTAFAMQELGCNQNQFFCKVPPELW KRYNMTINQTIWNHGNITLGEWYNQTKGLQQKFYEIIMDI EQNSVQGKKGIQQLQEWEDWIGWIGNIPQYLKGLLGGIL GIGLGVLLLILCLPTLVDCIRNCIHKILGYTVIAMPEVEGEE IQPQMELRRNGRQCGMSEKEEE | SEQ ID NO: 387 |

(continued)

| | |
|---|---|
| MNEEGPLNPGINPFRVPGITETEKQDYCNMLQPKLQALRN EIQEVKLEEGNAGKFRRARFLRYSDETILSLIHLFIGYCTYL LNRKELGSLRHDIDIEAPQEECYSSREQSITDNIKYGKRCFI GTAGLYLLLFIGVGIYLGTAKAQVVWRLPPLVVPVEESEII FWDCWAPEEPACQDFLGAMIHLKASTNISIQEGPTLGNW AKEIWGTLFKKATRQCRRGRIWKRWNETISGPLGCANNT CYNISVIVPDYQCYLDRVDTWLQGKVNVSLCLTGGKILY NKYTKQLSYCTDPLQIPLISYTFGPNQTCMWDTSQIQDPEI PKCGWWNQIAYYNSCRWESTDVKFHCQRTQSQPGLWLR AISSWKQRNRWEWRPDFESEKAKVSLQCNSTKNLTFAMR SSGDYGEVTGAWIEFGCHRNKSKLHTEARFRIRCRWNVG DNTSLIDTCGETQNVSGANPVDCTMYANRMYNCSLQNGF TMKVDDLIMHFNMTKAVEMYDIAGNWSCTSDLPPTWGY MNCNCTNSSSTNSVKMACPKNQGILRNWYNPVAGLRQS LEKYQVVKQPDYLVVPGEVMEYKPRRKRAAIHVMLALA TVLSMAGAGTGATAIGMVTQYHQVLATHQETIEKVTEAL KINNLRLVTLEHQVLVIGLKVEAMEK**R**LYTAFAMQELGC NQNQFFCKVPPELWKRYNMTINQTIWNHGNITLGEWYNQ TKELQQKFYEIIMNIEQNNVQVKKGLQQLQEWEDWVGWI GNIPQYLKGLLGGILGIGIGVLLLILCLPTLVDCIRNCISKVL GYTVIAMPEIGDEEETVQMELRKNGRQCGMSEKEEE | SEQ ID NO: 388 |
| MNEEGPLNPGINPFRVPGITETEKQDYCNMLQPKLQALRN EIQEVKLEEGNAGKFRRARFLRYSDETILSLIHLFIGYCTYL LNRKELGSLRHDIDIEAPQEECYSSREQSITDNIKYGKRCFI GTAGLYLLLFIGVGIYLGTAKAQVVWRLPPLVVPVEESEII FWDCWAPEEPACQDFLGAMIHLKASTNISIQEGPTLGNW AKEIWGTLFKKATRQCRRGRIWKRWNETISGPLGCANNT CYNISVIVPDYQCYLDRVDTWLQGKVNVSLCLTGGKILY NKYTKQLSYCTDPLQIPLISYTFGPNQTCMWDTSQIQDPEI PKCGWWNQIAYYNSCRWESTDVKFHCQRTQSQPGLWLR AISSWKQRNRWEWRPDFESEKAKVSLQCNSTKNLTFAMR SSGDYGEVTGAWIEFGCHRNKSKLHTEARFRIRCRWNVG DNTSLIDTCGETQNVSGANPVDCTMYANRMYNCSLQNGF TMKVDDLIMHFNMTKAVEMYDIAGNWSCTSDLPPTWGY MNCNCTNSSSTNSVKMACPKNQGILRNWYNPVAGLRQS LEKYQVVKQPDYLVVPGEVMEYKPRRKRAAIHVMLALA TVLSMAGAGTGATAIGMVTQYHQVLATHQETIEKVTEAL KINNLRLVTLEHQVLVIGLKVEAMEKF**R**YTAFAMQELGC NQNQFFCKVPPELWKRYNMTINQTIWNHGNITLGEWYNQ TKELQQKFYEIIMNIEQNNVQVKKGLQQLQEWEDWVGWI GNIPQYLKGLLGGILGIGIGVLLLILCLPTLVDCIRNCISKVL GYTVIAMPEIGDEEETVQMELRKNGRQCGMSEKEEE | SEQ ID NO: 389 |

(continued)

| | |
|---|---|
| <SEQ ID NO: 390;PRT; Artificial sequence > <br><br>MAEGFAANRQWIGPEEAEELLDFDIATQMNEEGPLNPGIN PFRVPGITEIEKRDYCKILQPKLQDLKNEIQEVKLEEGNAG KFRRARFLRYSDENILSLIHLFIGYCTYLCRKNELGSLRHDI DIDEHQEEYYTNIEKGTTANIKYGRRCLIGTAALYLLFIGIII YTQTTKAQVVWRLPPFVVPVEESEIIFWDCWAPEEPACQD FLGAMIHLKASTNISIQEGPTLGNWAREIWGTLFKKATRQ CRRGRVWRRWNETITGPSGCANNTCYNISVIVPDYQCYL DRVDTWLQGKVNISLCLTGGKMLYNKYTKQLSYCTDPL QIPLINYTFGPNQTCMWNTSQIQDPEIPKCGWWNQAAYY NSCRWESTDVKFHCQRTQSLPGTWLRTISSWRPKNRWEW RPDFESEKVKVSLQCNSTNNLTFAMRSSGDYGEVTGAWI EFGCHRKKSKLHSEARFRIRCRWDKGDNTSLIDTCGKTQN VLGANPVDCTMYANRMYNCSLQNGFTMKIDDLVMHFN MTKAVEMYNIAGNWSCTSDLPPTWGYMNCNCTNSSSTSS SSGNKMACPGDKGILRNWYNPVAGLRQSLEKYQVVKQP DYLVVPGEVMEYKPRRKRAAIHVMLALATVLSMAGAGT GATAIGMVTQYHQVLATHQEAIEKVTEALKINNLRLVTLE HQVLVIGLKVEAMEK**R**LYTAFAMQELGCNQNQFFCKVPP VLWERYNMTINQTIWNHGNITLGEWYNQTKDLQQKFYEI IMDIEQNNVQGKKGLQQLQKWEDWVGWIGNIPKYLKGL LGGILGIGLGVILLILCLPTLVDCVRNCIHKILGYTVIAMPE VEEEEIQPQMELRRNGRQCGISEKEEE | SEQ ID NO: 390 |
| MAEGFAANRQWIGPEEAEELLDFDIATQMNEEGPLNPGIN PFRVPGITEIEKRDYCKILQPKLQDLKNEIQEVKLEEGNAG KFRRARFLRYSDENILSLIHLFIGYCTYLCRKNELGSLRHDI DIDEHQEEYYTNIEKGTTANIKYGRRCLIGTAALYLLFIGIII YTQTTKAQVVWRLPPFVVPVEESEIIFWDCWAPEEPACQD FLGAMIHLKASTNISIQEGPTLGNWAREIWGTLFKKATRQ CRRGRVWRRWNETITGPSGCANNTCYNISVIVPDYQCYL <br><br>DRVDTWLQGKVNISLCLTGGKMLYNKYTKQLSYCTDPL QIPLINYTFGPNQTCMWNTSQIQDPEIPKCGWWNQAAYY NSCRWESTDVKFHCQRTQSLPGTWLRTISSWRPKNRWEW RPDFESEKVKVSLQCNSTNNLTFAMRSSGDYGEVTGAWI EFGCHRKKSKLHSEARFRIRCRWDKGDNTSLIDTCGKTQN VLGANPVDCTMYANRMYNCSLQNGFTMKIDDLVMHFN MTKAVEMYNIAGNWSCTSDLPPTWGYMNCNCTNSSSTSS SSGNKMACPGDKGILRNWYNPVAGLRQSLEKYQVVKQP DYLVVPGEVMEYKPRRKRAAIHVMLALATVLSMAGAGT GATAIGMVTQYHQVLATHQEAIEKVTEALKINNLRLVTLE HQVLVIGLKVEAMEKF**R**YTAFAMQELGCNQNQFFCKVPP VLWERYNMTINQTIWNHGNITLGEWYNQTKDLQQKFYEI IMDIEQNNVQGKKGLQQLQKWEDWVGWIGNIPKYLKGL LGGILGIGLGVILLILCLPTLVDCVRNCIHKILGYTVIAMPE VEEEEIQPQMELRRNGRQCGISEKEEE | SEQ ID NO: 391 |

(continued)

| | |
|---|---|
| MAEGFAVNRQWIGPEEAEELLDFDIATQMNEEGPLNPGIN LFRVPGITETEKQEYCNILQPKLQDLRNEIQEVKLEEGNAG KFRRARFLRYSDETILSLIHLFIGYCTYLCKRNKLGTLVHN IDIEAPQEECYSNRERGTTVNIKYSRRCCIGTTALYLLLLT GIIIYTQTTQAQVVWRLPPLVVPVEESEIIFWDCWAPEEPA CQDFLGAMIYLKASTNISIQEGPTLGNWAREIWGTLFKKA TRQCRRGRIWRRWNETITGPSGCANNTCYNISVIVPDYQC YLDRVDTWLQGKVNISLCLTGGKMLYNKDTKQLSYCTD PLQIPLINYTFGPNQTCMWNTSQIQDSDIPKCGWWNQIAY YNSCRWEQTDVKFHCQRTQSQPGTWLRTISSWKQKNRW EWRPDFESEKVRVSLQCNTTKNLTFAMRSSGDYGEVTGA WIEFGCHRNKSKLHSDARFRIRCRWNVGDNTSLIDTCGN DPNVSGANPVDCTMYANRMYNCSLQNGFTMKVDDLIMH FNMTKAVEMYNIAGNWSCTSDLPSTWGYMNCNCTNSSS TDSNKMACPKRQGILRNWYNPVAGLRQSLEKYQVVKQP DYLVVPREVMEYKPRRKRAAIHVMLALATVLSMAGAGT GATAIGMVTQYHQVLATHQETIEKITEALKVNNLRLVTLE HQVLVIGLKVEAIEK**R**LYTAFAMQELGCNQNQFFCKVPP ELWQRYNMTINQTIWNHGNITLGEWYNQTKDLQQKFYEI IMDMEQNNVQGRKGLQQLQEWEDWVGWLGNIPRYLKG LLGGILGIGLGVLLLILCLPTLVDCIRNCISKVLGYTVIAMP EVEEEEIQPPMELRRNGRQCDMSEKEEE | SEQ ID NO: 392 |
| MAEGFAVNRQWIGPEEAEELLDFDIATQMNEEGPLNPGIN LFRVPGITETEKQEYCNILQPKLQDLRNEIQEVKLEEGNAG KFRRARFLRYSDETILSLIHLFIGYCTYLCKRNKLGTLVHN IDIEAPQEECYSNRERGTTVNIKYSRRCCIGTTALYLLLLT GIIIYTQTTQAQVVWRLPPLVVPVEESEIIFWDCWAPEEPA CQDFLGAMIYLKASTNISIQEGPTLGNWAREIWGTLFKKA TRQCRRGRIWRRWNETITGPSGCANNTCYNISVIVPDYQC YLDRVDTWLQGKVNISLCLTGGKMLYNKDTKQLSYCTD PLQIPLINYTFGPNQTCMWNTSQIQDSDIPKCGWWNQIAY YNSCRWEQTDVKFHCQRTQSQPGTWLRTISSWKQKNRW EWRPDFESEKVRVSLQCNTTKNLTFAMRSSGDYGEVTGA WIEFGCHRNKSKLHSDARFRIRCRWNVGDNTSLIDTCGN DPNVSGANPVDCTMYANRMYNCSLQNGFTMKVDDLIMH FNMTKAVEMYNIAGNWSCTSDLPSTWGYMNCNCTNSSS TDSNKMACPKRQGILRNWYNPVAGLRQSLEKYQVVKQP DYLVVPREVMEYKPRRKRAAIHVMLALATVLSMAGAGT GATAIGMVTQYHQVLATHQETIEKITEALKVNNLRLVTLE HQVLVIGLKVEAIEKF**R**YTAFAMQELGCNQNQFFCKVPPE LWQRYNMTINQTIWNHGNITLGEWYNQTKDLQQKFYEII MDMEQNNVQGRKGLQQLQEWEDWVGWLGNIPRYLKGL LGGILGIGLGVLLLILCLPTLVDCIRNCISKVLGYTVIAMPE VEEEEIQPPMELRRNGRQCDMSEKEEE | SEQ ID NO: 393 |

(continued)

| | |
|---|---|
| MAAGFSQNRQWIGPEEAEELLDFDIATQINEEGPLNPGVN PFRVPGITDTEKQDYCKILQPKLQELREEINEVKLDEDNAG KFRRVRYLRYADETVLSLIYALVGYLRYLGNRNKLGSLR HDIDIEVSAKEQFDKKEKGTTINQKYCTRCCVGISVLYLIL FIIIVAVTGSQAQVVWRHPPLVVPVEETEIIFWDCWAPEEP ACQDFLGTMVQLKASINIGIQEGPTLGHWAREIWSTLF KKATRQCRRGRVWRRWNETITGPLGCANNTCYNISVVVP DYQCYVDRVDTWLQGRINISLCLTGGKMLYNKDTQQLS YCTEPLQIPLINYTFGPNQTCMWNTSLIEDSEIPKCGWWN QAAYYNSCKWEQTDVKFQCLRTQSQPGNWLRTISSWKQ SNRWIWRPDFESDKVKISLQCNSTKNLTFAMRSSGDYGEI TGAWIEFGCYRNKSKSHGEARFRIRCRWNEGTNTSLIDTC GSTPNVKGANPVDCTMKANTMYNCSLQNGFTMKIEDLIV HFNMTKAVEMYNIAGNWSCNSDIPPGWGYMNCNCTNST SSSFPTCPKMACPRERGILRNWYNPIAGLRHALQKYQVVK QPAYLLVPEEVMEYKPSQKRAAIHIILALATVLSIAGAGTG ATAIGMVTQYHQVLATHQKAIDQITEALKINNLRLVTLEH QVLVIGLKVEAIEK**R**IYTAFAMQELGCNQNQFFCKIPPEL WIRYNMSINQTIWNHGNISLRDWYNNTQQLQKKFYEIIYD IEQNNVQGKQGLQQLQKWETWVSWIGKIPQYLKGLFGSI LGIGLGILLMILCLPTLVDCMRNCLNRVLGYTVIAMPTIDD EGTDFPVELRRNGGQCGMSEKEEE | SEQ ID NO: 394 |
| MAAGFSQNRQWIGPEEAEELLDFDIATQINEEGPLNPGVN PFRVPGITDTEKQDYCKILQPKLQELREEINEVKLDEDNAG KFRRVRYLRYADETVLSLIYALVGYLRYLGNRNKLGSLR HDIDIEVSAKEQFDKKEKGTTINQKYCTRCCVGISVLYLIL FIIIVAVTGSQAQVVWRHPPLVVPVEETEIIFWDCWAPEEP ACQDFLGTMVQLKASINIGIQEGPTLGHWAREIWSTLFKK ATRQCRRGRVWRRWNETITGPLGCANNTCYNISVVVPDY QCYVDRVDTWLQGRINISLCLTGGKMLYNKDTQQLSYCT EPLQIPLINYTFGPNQTCMWNTSLIEDSEIPKCGWWNQAA YYNSCKWEQTDVKFQCLRTQSQPGNWLRTISSWKQSNR WIWRPDFESDKVKISLQCNSTKNLTFAMRSSGDYGEITGA WIEFGCYRNKSKSHGEARFRIRCRWNEGTNTSLIDTCGST PNVKGANPVDCTMKANTMYNCSLQNGFTMKIEDLIVHFN MTKAVEMYNIAGNWSCNSDIPPGWGYMNCNCTNSTSSSF PTCPKMACPRERGILRNWYNPIAGLRHALQKYQVVKQPA YLLVPEEVMEYKPSQKRAAIHIILALATVLSIAGAGTGATA IGMVTQYHQVLATHQKAIDQITEALKINNLRLVTLEHQVL VIGLKVEAIEKF**R**YTAFAMQELGCNQNQFFCKIPPELWIR YNMSINQTIWNHGNISLRDWYNNTQQLQKKFYEIIYDIEQ NNVQGKQGLQQLQKWETWVSWIGKIPQYLKGLFGSILGI GLGILLMILCLPTLVDCMRNCLNRVLGYTVIAMPTIDDEG TDFPVELRRNGGQCGMSEKEEE | SEQ ID NO: 395 |

| | SEQ ID NO: 396 |
|---|---|
| MAAGFTQNRQWIGPEEAEELLDFDIVTQINEEGPLNPGVN PFRVPAITDTEKQDYCKILQPKLQELREEIKETKLDESNAG KFRRVRYLRYADETALSLIYALVGYLRYLLERRKLGSLR HDVDIEVSAKEQFNKKEKGTTVNQNYCTKCCVGISVLYFI LFLILVAVTRSQAQVVWRLPPLVVPVEETEIIFWDCWAPE EPACQDFLGTMVQLKASINISIQEGPTLGHWAREILETLF KKATRQCRRGRVWKRWNETITGPLGCANNTCYNISVVVP DYQCYVDRVDTWLQGRINISLCLTGGKMLYNKDTQQLS YCTEPLQIPLINYTFGPNQTCMWNTSLIEDSEIPKCGWWN QAAYYNSCKWEQTDVKFQCQRTQSQPGTWLRAIASWKQ ANRWIWRPDFESDKVKISLQCNSTKNLTFAMRSSSDYGEI TGAWIEFGCYRNKSKFHDEARFRIRCRWNEGTNTSLIDTC GDNPNVTGANPVDCTMRANIMYNCSLQNGFTMKIEDLIV HFNMTKAVEMYNIAGNWSCTSDLPKGWGYMNCNCTNG TDNTKMTCPENQGILRNWYNPVAGLRQALMKYQVVKQP EYLIVPEEVMQYKSKQKRAAIHIMLALATVLSMAGAGTG ATAIGMVTQYHQVLATHQQALDKITEALKINNLRLITLEH QVLVIGLKVEAIEK**R**LYTAFAMQELGCNQNQFFCKIPPSL WSMYNMTLNQTIWNHGNISLGNWYNQTRDLQNKFYEII MDIEQNNVQGKTGIQQLQKWENWVGWIGKIPQYLKGLL GSVLGIGLGILLLLICLPTLVDCIRNCTNKILGYTVIAMPEI DDEEVHLSVELRRNGRQCGISEKEEE | |
| MAAGFTQNRQWIGPEEAEELLDFDIVTQINEEGPLNPGVN PFRVPAITDTEKQDYCKILQPKLQELREEIKETKLDESNAG KFRRVRYLRYADETALSLIYALVGYLRYLLERRKLGSLR HDVDIEVSAKEQFNKKEKGTTVNQNYCTKCCVGISVLYFI LFLILVAVTRSQAQVVWRLPPLVVPVEETEIIFWDCWAPE EPACQDFLGTMVQLKASINISIQEGPTLGHWAREILETLF KKATRQCRRGRVWKRWNETITGPLGCANNTCYNISVVVP DYQCYVDRVDTWLQGRINISLCLTGGKMLYNKDTQQLS YCTEPLQIPLINYTFGPNQTCMWNTSLIEDSEIPKCGWWN QAAYYNSCKWEQTDVKFQCQRTQSQPGTWLRAIASWKQ ANRWIWRPDFESDKVKISLQCNSTKNLTFAMRSSSDYGEI TGAWIEFGCYRNKSKFHDEARFRIRCRWNEGTNTSLIDTC GDNPNVTGANPVDCTMRANIMYNCSLQNGFTMKIEDLIV HFNMTKAVEMYNIAGNWSCTSDLPKGWGYMNCNCTNG TDNTKMTCPENQGILRNWYNPVAGLRQALMKYQVVKQP EYLIVPEEVMQYKSKQKRAAIHIMLALATVLSMAGAGTG ATAIGMVTQYHQVLATHQQALDKITEALKINNLRLITLEH QVLVIGLKVEAIEKF**R**YTAFAMQELGCNQNQFFCKIPPSL WSMYNMTLNQTIWNHGNISLGNWYNQTRDLQNKFYEII MDIEQNNVQGKTGIQQLQKWENWVGWIGKIPQYLKGLL GSVLGIGLGILLLLICLPTLVDCIRNCTNKILGYTVIAMPEI DDEEVHLSVELRRNGRQCGISEKEEE | SEQ ID NO: 397 |

(continued)

| | |
|---|---|
| MAEGFCQNRQWIGPEEAEELLDFDIATQVSEEGPLNPGIN PFRQPGLTDGEKEEYCKILQPRLQALREEYKEGSLNSESA GKYRRVRYLRYSDLRVLSLLYLFIGYLAFFVRKRGLGKQ RQDIDIESKGTEEKFSKNEKGQTVNIRNCRILTIAICSFYIFL FIGIGIYAGKGEAQVIWRLPPLVVPVEDSEIIFWDCWAPEE PACQDFLGAMMHLKASTNISIQEGPTLGKWAKEIWATLF KKATRQCRRGKVWRKWNETITGPKGCANNTCYNVTVSIP DYQCYLDRVDTWLQGKVNISLCLTGGKMLYNKETKQLS YCTDPLQIPLINYTFGPNQTCMWNTSLIKNPDIPKCGWWN QAVYYNSCRWEKADVQFQCQRTQSQPGTWLRKISSWKQ KNRWEWRPDFESERVKISLQCNSTKNLTFAMRSSSDYSD VVGAWIEFGCHRNKSRRHTDARFRIRCKWNVGSNTSLID TCGKENITGANPVDCTMTAKTLYNCSLQEGFTMKIEDLIM HFNMTKAVEMYEIAGNWSCKSDLPTDWGYMKCNCTSRN ESEKMKCPAKDGILRNWYNPVAGLRQALDKYQVVKQPD YIVVPEEVLNYQSRQKRAAIHIMLALATVLSIAGAGTGAT AIGMVTQYHQVLATHQEALDKITEALKINNLRLVTLEHQ VLVIGLKVEATEK**R**LYTAFAMQELGCNQNQFFCKIPCEL WMRYNLTLNQTIWNHGNVTLQDWYNQTKQLQQKFYEII MDIEQNNVQGKKGIQQLQSWEYWTGWMGKIPQYLKGLL GGVLGIGLGILLLILCLPTLLDCMRNCINKVMGYTVIVMPE IDDEELSQNMELRRNGRQCGMSEKEEE | SEQ ID NO: 398 |
| MAEGFCQNRQWIGPEEAEELLDFDIATQVSEEGPLNPGIN PFRQPGLTDGEKEEYCKILQPRLQALREEYKEGSLNSESA GKYRRVRYLRYSDLRVLSLLYLFIGYLAFFVRKRGLGKQ RQDIDIESKGTEEKFSKNEKGQTVNIRNCRILTIAICSFYIFL FIGIGIYAGKGEAQVIWRLPPLVVPVEDSEIIFWDCWAPEE PACQDFLGAMMHLKASTNISIQEGPTLGKWAKEIWATLF KKATRQCRRGKVWRKWNETITGPKGCANNTCYNVTVSIP DYQCYLDRVDTWLQGKVNISLCLTGGKMLYNKETKQLS YCTDPLQIPLINYTFGPNQTCMWNTSLIKNPDIPKCGWWN QAVYYNSCRWEKADVQFQCQRTQSQPGTWLRKISSWKQ KNRWEWRPDFESERVKISLQCNSTKNLTFAMRSSSDYSD VVGAWIEFGCHRNKSRRHTDARFRIRCKWNVGSNTSLID TCGKENITGANPVDCTMTAKTLYNCSLQEGFTMKIEDLIM HFNMTKAVEMYEIAGNWSCKSDLPTDWGYMKCNCTSRN ESEKMKCPAKDGILRNWYNPVAGLRQALDKYQVVKQPD YIVVPEEVLNYQSRQKRAAIHIMLALATVLSIAGAGTGAT AIGMVTQYHQVLATHQEALDKITEALKINNLRLVTLEHQ VLVIGLKVEATEKF**R**YTAFAMQELGCNQNQFFCKIPCEL WMRYNLTLNQTIWNHGNVTLQDWYNQTKQLQQKFYEII MDIEQNNVQGKKGIQQLQSWEYWTGWMGKIPQYLKGLL GGVLGIGLGILLLILCLPTLLDCMRNCINKVMGYTVIVMPE IDDEELSQNMELRRNGRQCGMSEKEEE | SEQ ID NO: 399 |

(continued)

| | |
|---|---|
| MAEGGFAQNQQWIGPEEAEELLDFDIAVQMNEEGPLNPG VNPFRVPGITPQEKDNYCTILQPRLQELKREIKEVKIEEN AGKFRRARFLRYSDENVLSIVYLLTGYLRYLIDRKSLGSL RHDIDIEVPQKEQYSNNEKGNTVNRKYGRICCISTLFLYLL LFAGIGVWYGTTAQVVWRLPPLVVPIDDTEIIFWDCWAPE EPACQDFLGAMIHLKANINISIQEGPTLGNWAREIWSTLFK KATRQCRRGRIWRRWNETITGPLGCANNTCYNISVVIPDY QCYVDRVDTWLQGKVNISLCLTGGKMLFNKETKQLSYC TDPLQIPLINYTFGPNQTCEWNTSLIKDPEIPKCGWWNQN AYYNSCKWEQTDVKFQCQRIQSQPGSWIRAISSWRQRNR WEWRPDFESEKVKISLQCNSTRNLTFAMRSSSDYYDVQG AWIEFGCYRNKSIRHTGTRFRIRCRWNEGKNMSLIDTCGT DPNVTRANPVNCTLKTNTMYNCTLQDSFTMKIEDLIVHF NMSKAVEMYNIAGNWSCTSDLPTGWGYMKCNCTSTNNT GKMKCPEPEGILRNWYNPVAGLRQALMKYQVVKQPEYL IVPEEVMKYKSKQKRAAIHIMLALATVLSIAGAGTGATAI GMVTQYHQVLATHQQALEKITEALKINNLRLVTLEHQVL MIGLKVEAIEK**R**LYTAFAMQELGCNQNQFFCKIPLNLWT MYNMTINQTLWNHGNITLGDWYNQTKGLQEKFYEIIMDL EQNNVQGKLGIQQLQKWENWVGWIGKIPQYLKGLLGSV LGIGVGILLLIICLPTLVDCIRNCINKVLGYSVIAMPELDDE EVSMELRRNGRQCGMSEKEEE | SEQ ID NO: 400 |
| MAEGGFAQNQQWIGPEEAEELLDFDIAVQMNEEGPLNPG VNPFRVPGITPQEKDNYCTILQPRLQELKREIKEVKIEEN AGKFRRARFLRYSDENVLSIVYLLTGYLRYLIDRKSLGSL RHDIDIEVPQKEQYSNNEKGNTVNRKYGRICCISTLFLYLL LFAGIGVWYGTTAQVVWRLPPLVVPIDDTEIIFWDCWAPE EPACQDFLGAMIHLKANINISIQEGPTLGNWAREIWSTLFK KATRQCRRGRIWRRWNETITGPLGCANNTCYNISVVIPDY QCYVDRVDTWLQGKVNISLCLTGGKMLFNKETKQLSYC TDPLQIPLINYTFGPNQTCEWNTSLIKDPEIPKCGWWNQN AYYNSCKWEQTDVKFQCQRIQSQPGSWIRAISSWRQRNR WEWRPDFESEKVKISLQCNSTRNLTFAMRSSSDYYDVQG AWIEFGCYRNKSIRHTGTRFRIRCRWNEGKNMSLIDTCGT DPNVTRANPVNCTLKTNTMYNCTLQDSFTMKIEDLIVHF NMSKAVEMYNIAGNWSCTSDLPTGWGYMKCNCTSTNNT GKMKCPEPEGILRNWYNPVAGLRQALMKYQVVKQPEYL IVPEEVMKYKSKQKRAAIHIMLALATVLSIAGAGTGATAI GMVTQYHQVLATHQQALEKITEALKINNLRLVTLEHQVL MIGLKVEAIEKF**R**YTAFAMQELGCNQNQFFCKIPLNLWT MYNMTINQTLWNHGNITLGDWYNQTKGLQEKFYEIIMDL EQNNVQGKLGIQQLQKWENWVGWIGKIPQYLKGLLGSV LGIGVGILLLIICLPTLVDCIRNCINKVLGYSVIAMPELDDE EVSMELRRNGRQCGMSEKEEE | SEQ ID NO: 401 |

(continued)

| | |
|---|---|
| MAEGGFTQNHQWIGPEEAEELLDFDIAIQMNEEGPLNPGV NPFRVPGITSQEKDDYCKILQTKLQELKNEVKEVKIEEGN AGKFRRARFLRYSDENVLSIVYLLIGYLRYLIDHRSLGSLR HDIDIEAPQEEHYNNSEKGTTLNIKYGRRCCISTFIMYLILF AGVGIWFGARAQVVWRLPPLVVPVDDTEIIFWDCWAPEE PACQDFLGTMIHLKAKTNISIQEGPTLGNWAREIWATLFK KATRQCRRGRIWRRWNETITGPLGCANNTCYNISVVPD YQCYLDRVDTWLQGKLNISLCLTGGKMLYNKVTKQLSY CTDPLQIPLINYTFGPNQTCMWNTSQIQDPEIPKCGWWNQ MAYYNSCKWEEAKVKFHCQRTQSQPGSWHRAISSWKQR NRWEWRPDFKSKKVKISLQCNSTKNLTFAMRSSSDYYDV QGAWIEFGCHRNKSKGFSEARFRTRCKWNEGNNISLIDTC GTNPNVTGANPVDCTMKANIMYNCSLQDSFTMKIEDLIV HFNMTKAVEMYNIAGNWSCTSDLPKGWGYMNCNCTNG TDNSKTKMACPRNQGILRNWYNPVAGLRQALIKYQVVK QPEYLIVPEEVMQYKVKQKRAAIHIMLALATVLSMAGAG TGATAIGMVTQYHQVLATHQHALDKITEALKINNLRLITL VHQVLVIGLKVRAIEK**R**LYTAFAMQELGCNQNQFFCKIPP SLWSMYNMTLNQTIWNHGNISLGNWYNQTRDLQNKFYE IIMDIEQNNVQGKTGIQQLQKWENWVGWIGKIPQYLKGL LGSVLGIGLGILLLLICLPTLVDCIRNCTHKILGYTVIAMPEI DDEEVHLSVELRRNGRQCGISEKEEE | SEQ ID NO: 402 |
| MAEGGFTQNHQWIGPEEAEELLDFDIAIQMNEEGPLNPGV NPFRVPGITSQEKDDYCKILQTKLQELKNEVKEVKIEEGN AGKFRRARFLRYSDENVLSIVYLLIGYLRYLIDHRSLGSLR HDIDIEAPQEEHYNNSEKGTTLNIKYGRRCCISTFIMYLILF AGVGIWFGARAQVVWRLPPLVVPVDDTEIIFWDCWAPEE PACQDFLGTMIHLKAKTNISIQEGPTLGNWAREIWATLFK KATRQCRRGRIWRRWNETITGPLGCANNTCYNISVVPD YQCYLDRVDTWLQGKLNISLCLTGGKMLYNKVTKQLSY CTDPLQIPLINYTFGPNQTCMWNTSQIQDPEIPKCGWWNQ MAYYNSCKWEEAKVKFHCQRTQSQPGSWHRAISSWKQR NRWEWRPDFKSKKVKISLQCNSTKNLTFAMRSSSDYYDV QGAWIEFGCHRNKSKGFSEARFRTRCKWNEGNNISLIDTC GTNPNVTGANPVDCTMKANIMYNCSLQDSFTMKIEDLIV HFNMTKAVEMYNIAGNWSCTSDLPKGWGYMNCNCTNG TDNSKTKMACPRNQGILRNWYNPVAGLRQALIKYQVVK QPEYLIVPEEVMQYKVKQKRAAIHIMLALATVLSMAGAG TGATAIGMVTQYHQVLATHQHALDKITEALKINNLRLITL VHQVLVIGLKVRAIEKP**R**YTAFAMQELGCNQNQFFCKIPP SLWSMYNMTLNQTIWNHGNISLGNWYNQTRDLQNKFYE IIMDIEQNNVQGKTGIQQLQKWENWVGWIGKIPQYLKGL LGSVLGIGLGILLLLICLPTLVDCIRNCTHKILGYTVIAMPEI DDEEVHLSVELRRNGRQCGISEKEEE | SEQ ID NO: 403 |

(continued)

| | |
|---|---|
| MAEGFAANRQWIGPEEAEELLDFDKATQMNEEGPLNPGV NPFRVPAVTEADKQEYCKILQPRLQEIRNEIQEVKLEEGN AGKFRRARFLRYSDESILSLIHLFIGYCTYLVNRRRLGSLR HDINIEAPQEEQYSSREQGTTENIKYGRRCLIGTASLYLLLF IGVAIYLGTTNAQIVWRLPPLVVPVEESEIIFWDCWAPEEP ACQDFLGAMIHLKASTNISIQEGPTLGNWAREIWGTLFKK ATRHCRRNKIWKRWNETITGPVGCANNTCYNISVIIPDYQ CYLDRVDTWLQGKVNISLCLTGGKMLYNRDTKQLSYCT DPLQIPLINYTFGPNQTCMWNTSQIQDPEIPKCGWWNQIA YYNSCRWESTNVKFYCQRTQSQPGTWIRTISSWRQKNRW EWRPDFESEKVKISLQCNSTHNLTFAMRSSGDYGEVMGA WIEFGCHRNKSRFHTEARFRIRCRWNVGDNTSLIDTCGKN LNVSGANPVDCTMYANKMYNCSLQNGFTMKVDDLIMHF NMTKAVEMYNIAGNWSCKSDLPQNWGYMNCNCTNGTS NDNKMACPEDKGILRNWYNPVAGLRQALEKYQVVKQPE<br><br>YIVVPTEVMTYKYQKRAAIHIMLALATVLSIAGAGTGAT AIGMVTQYQQVLATHQEALDKITEALKINNLRLVTLEHQ MLVIGLKVEAIEK**R**LYTAFAMQELGCNQNQFFCEIPKELW LRYNMTLNQTIWNHGNITLGEWYNQTKYLQQKFYEIIMD IEQNNVQGKQGLQKLQNWQDWMGWIGKIPQYLKGLLGG ILGIGLGILLLILCLPTLVDCIRNCISKVLGYTVIAMPEIDDE EETVQMELRKNGRQCGMSEKEEE | SEQ ID NO: 404 |
| MAEGFAANRQWIGPEEAEELLDFDKATQMNEEGPLNPGV NPFRVPAVTEADKQEYCKILQPRLQEIRNEIQEVKLEEGN AGKFRRARFLRYSDESILSLIHLFIGYCTYLVNRRRLGSLR HDINIEAPQEEQYSSREQGTTENIKYGRRCLIGTASLYLLLF IGVAIYLGTTNAQIVWRLPPLVVPVEESEIIFWDCWAPEEP ACQDFLGAMIHLKASTNISIQEGPTLGNWAREIWGTLFKK ATRHCRRNKIWKRWNETITGPVGCANNTCYNISVIIPDYQ CYLDRVDTWLQGKVNISLCLTGGKMLYNRDTKQLSYCT DPLQIPLINYTFGPNQTCMWNTSQIQDPEIPKCGWWNQIA YYNSCRWESTNVKFYCQRTQSQPGTWIRTISSWRQKNRW EWRPDFESEKVKISLQCNSTHNLTFAMRSSGDYGEVMGA WIEFGCHRNKSRFHTEARFRIRCRWNVGDNTSLIDTCGKN LNVSGANPVDCTMYANKMYNCSLQNGFTMKVDDLIMHF NMTKAVEMYNIAGNWSCKSDLPQNWGYMNCNCTNGTS NDNKMACPEDKGILRNWYNPVAGLRQALEKYQVVKQPE YIVVPTEVMTYKYQKRAAIHIMLALATVLSIAGAGTGAT AIGMVTQYQQVLATHQEALDKITEALKINNLRLVTLEHQ MLVIGLKVEAIEKF**R**YTAFAMQELGCNQNQFFCEIPKELW LRYNMTLNQTIWNHGNITLGEWYNQTKYLQQKFYEIIMD IEQNNVQGKQGLQKLQNWQDWMGWIGKIPQYLKGLLGG ILGIGLGILLLILCLPTLVDCIRNCISKVLGYTVIAMPEIDDE EETVQMELRKNGRQCGMSEKEEE | SEQ ID NO: 405 |

(continued)

| | SEQ ID NO: 406 |
|---|---|
| MAEGGFTQNQQWIGPEEAEELLDFDIAVQMNEEGPLNPG VNPFRVPGITSQEKEDYCKILQTKLQELKNEVKGVKIEEG NAGKFRRARYLRYSDENVLSIVYLLIGYLRYLIDHRSLGSL RHDIDIETPQEEHFNNSEKGTTLNTKYGRRCCLSTFIMNLI LFAGVGIWLGARAQVVWRLPPLVVPVDDTEIIFWDCWAP EEPACQDFLGTMVQLKASINISIQEGPTLGHWAREILETLF KKATRQCRRGRVWRRWNETITGPLGCANNACYNISVVVP DYQCYVDRVDTWLQGRINISLCLTGGKMLYNKDTQQLS YCTEPLQIPLINYTFGPNQTCMWNTSLIEDSEIPKCGWWN QAAYYNSCKWEQTDVKFQCQRTQSQPGTWLRAIASWEQ ANRWIWRPDFESDKVKISLQCNSTKNLTFAMRSSSDYYD VQGAWIEFGCHRNKSRRHSEARFRIRCKWNEGKNISLIDT CGTNPNVTGANPVDCTMKANTMYNCSLQDSFTMKIEDLI VHFNMTKAVEMYNIAGNWSCTSDLPKGWGYMNCNCTN GTDDSKTKMACPENQGILRNWYNPVAGLRQALIKYHVV KQPEYLIVPEEVMQYKVKQKRAAIHIMLALATVLSMAGA GTGATAIGMVTQYHQVLATHQQALDKITEALKINNLRLIT LEHQVLVIGLKVEAIEK**R**LYTAFAMQELGCNQNQFFCKIP PSLWSMYNMTLNQTIWNHGNISLGNWYNQTRDLQNKFY EIIMDIEQNNVQGKTGIQQLQKWENWMGWIGKIPQYLKG

LLGSVLGIGLGILLLLICLPTLVDCIRNCTNKILGYTVIAMP EIDDEEVHLSVELRRNGRQCGISEKEEE | |
| MAEGGFTQNQQWIGPEEAEELLDFDIAVQMNEEGPLNPG VNPFRVPGITSQEKEDYCKILQTKLQELKNEVKGVKIEEG NAGKFRRARYLRYSDENVLSIVYLLIGYLRYLIDHRSLGSL RHDIDIETPQEEHFNNSEKGTTLNTKYGRRCCLSTFIMNLI LFAGVGIWLGARAQVVWRLPPLVVPVDDTEIIFWDCWAP EEPACQDFLGTMVQLKASINISIQEGPTLGHWAREILETLF KKATRQCRRGRVWRRWNETITGPLGCANNACYNISVVVP DYQCYVDRVDTWLQGRINISLCLTGGKMLYNKDTQQLS YCTEPLQIPLINYTFGPNQTCMWNTSLIEDSEIPKCGWWN QAAYYNSCKWEQTDVKFQCQRTQSQPGTWLRAIASWEQ ANRWIWRPDFESDKVKISLQCNSTKNLTFAMRSSSDYYD VQGAWIEFGCHRNKSRRHSEARFRIRCKWNEGKNISLIDT CGTNPNVTGANPVDCTMKANTMYNCSLQDSFTMKIEDLI VHFNMTKAVEMYNIAGNWSCTSDLPKGWGYMNCNCTN GTDDSKTKMACPENQGILRNWYNPVAGLRQALIKYHVV KQPEYLIVPEEVMQYKVKQKRAAIHIMLALATVLSMAGA GTGATAIGMVTQYHQVLATHQQALDKITEALKINNLRLIT LEHQVLVIGLKVEAIEKF**R**YTAFAMQELGCNQNQFFCKIP PSLWSMYNMTLNQTIWNHGNISLGNWYNQTRDLQNKFY EIIMDIEQNNVQGKTGIQQLQKWENWMGWIGKIPQYLKG LLGSVLGIGLGILLLLICLPTLVDCIRNCTNKILGYTVIAMP EIDDEEVHLSVELRRNGRQCGISEKEEE | SEQ ID NO: 407 |

(continued)

| | |
|---|---|
| MAEGGFTQNQQWIGPEEAEELLDFDIAVQMNEEGPLNPG VNPFRVPGITSQEKEDYCKTLQTNLQELKNEVKEVKIEEG NAGKFRRARFLRYSDENVLSIVYLLIGYLRYLIDHRSLGSL RHDIDIEAPQEEHYNNSEKGTTLNIKYARRCCISTFIMYLIL FAGVGIWLGARAQVVWRLPPLVVPVDDTEIIFWDCWAPE EPACQDFLGTMVQLKASINIGIQEGPTLGHWAREIWSTLF KKATRQCRRGRVWRRWNETITGPLGCANNTCYNISVVVP DYQCYVDRVDTWLQGRINISLCLTGGKMLYNKDTHQLS YCTEPLQIPLINCTFGPIQTCMWNTSLIQDPEIPKCGWWTQ VAYYNNCKWEEANVTFQCHRTQSRSGSWLRTISSWRQR NRWEWRPDFESEKVKISLQCNSTKNLTFAMRSSSDYYDV QGAWIEFGCHRNKSKKHSEARFRIRCKWNEGNNISLIDTC GTNPNVTGANPVDCTMKANTMYNCSLQDSFTMKIEDLIV HFNMTKAVEMYNIAGNWSCTSDLPKGWGYMNCNCTNG TDDSKTKMACPENQGILRNWYNPVAGLRQALIKYQVVK QPEYLIVPEEVMQYKVQKRAAIHIMLALATVLSMAGAG TGATAIGMVTQYHQVLATHQQALDKITEALKINNLRLITL EHQVLVIGLKVEAIEK**R**LYTAFAMQELGCNQNQFFCKIPP SLWSMYNMTLNQTIWNHGNISLGNWYNQTRDLQNKFYE IIMDIEQNNVQGKTGIQQLQKWENWVGWIGKIPQYLKGL LGSVLGIGLGILLLLICLPTLVDCIRNCTNKILGYTVIAMPEI DDEEVHLSVELRRNGRQCGISEKEEE | SEQ ID NO: 408 |
| MAEGGFTQNQQWIGPEEAEELLDFDIAVQMNEEGPLNPG VNPFRVPGITSQEKEDYCKTLQTNLQELKNEVKEVKIEEG NAGKFRRARFLRYSDENVLSIVYLLIGYLRYLIDHRSLGSL RHDIDIEAPQEEHYNNSEKGTTLNIKYARRCCISTFIMYLIL FAGVGIWLGARAQVVWRLPPLVVPVDDTEIIFWDCWAPE EPACQDFLGTMVQLKASINIGIQEGPTLGHWAREIWSTLF KKATRQCRRGRVWRRWNETITGPLGCANNTCYNISVVVP DYQCYVDRVDTWLQGRINISLCLTGGKMLYNKDTHQLS YCTEPLQIPLINCTFGPIQTCMWNTSLIQDPEIPKCGWWTQ VAYYNNCKWEEANVTFQCHRTQSRSGSWLRTISSWRQR NRWEWRPDFESEKVKISLQCNSTKNLTFAMRSSSDYYDV QGAWIEFGCHRNKSKKHSEARFRIRCKWNEGNNISLIDTC GTNPNVTGANPVDCTMKANTMYNCSLQDSFTMKIEDLIV HFNMTKAVEMYNIAGNWSCTSDLPKGWGYMNCNCTNG TDDSKTKMACPENQGILRNWYNPVAGLRQALIKYQVVK QPEYLIVPEEVMQYKVQKRAAIHIMLALATVLSMAGAG TGATAIGMVTQYHQVLATHQQALDKITEALKINNLRLITL EHQVLVIGLKVEAIEKF**R**YTAFAMQELGCNQNQFFCKIPP SLWSMYNMTLNQTIWNHGNISLGNWYNQTRDLQNKFYE IIMDIEQNNVQGKTGIQQLQKWENWVGWIGKIPQYLKGL LGSVLGIGLGILLLLICLPTLVDCIRNCTNKILGYTVIAMPEI DDEEVHLSVELRRNGRQCGISEKEEE | SEQ ID NO: 409 |

| | |
|---|---|
| MAEGGFTHNQQWIGPEEAEELLDFDIAVQMNEEGPLNPG<br>VNPFRVPGITSQEKDDYCKILQTKLQELKNEVKEVKIEEG<br>NAGKFRRARYLRYSDENVLSIVYLLIGYLRYLIDHRSLGSL<br>RHDIDIETPQEEHYNNSEKGTTLNIKYGRRCCISTFIMYLIL<br>FAGVGIWLGARAQVVWRLPPLVVPVDDTEIIFWDCWAPE<br>EPACQDFLGTMIYLKANVNISIQEGPTLGNWAREIWSTLF<br>KKATRQCRRGRIWRRWNETITGPLGCANNTCYNISVVVP<br>DYQCYVDRVDTWLQGKVNISLCLTGGKMLYNKETRQLS<br>YCTDPLQIPLINYTFGPNQTCMWNTSLIKDSEIPKCGWWN<br>QVAYYDTCKWEEANVTFQCHRTQSQSGSWIRTISSWKQR<br>NRWEWRPDFESEKVKISLQCNSTKNLTFAMRSSSDYYDV<br>QGAWIEFGCHRNKSKRHSEARFRIRCKWNEGNNISLIDTC<br>GTNPNVTGANPVDCTMKANTMYNCSLQDSFTMKIEDLIV<br>HFNMTKAVELYNIAGNWSCTSDLPKGWGYMNCNCTNGT<br>DNSETKMACPKNQGILRNWYNPVAGLRQALIKYQVVKQ<br>PEYLIVPEEVMQYKFKQKRAAIHIMLALATVLSMAGAGT<br>GATAIGMVTQYHQVLATHQQALEKITEALKINNLRLITLE<br>HQVLVIGLRVEAIEK**R**LYTAFAMQELGCNQNQFFCKIPPS<br>LWSMYNMTLNQTIWNHGNISLGNWYNQTRDLQNKFYEII<br>MDIEQNNVQGKTGIQQLQKWENWVGWIGKIPQYLKGLL<br>GSVLGIGLGILLLLICLPTLVDCIRNCTNKILGYTVIAMPEI<br>DDEEVHLSVELRRNGRQCGISEKEEE | SEQ ID NO: 410 |
| MAEGGFTHNQQWIGPEEAEELLDFDIAVQMNEEGPLNPG<br>VNPFRVPGITSQEKDDYCKILQTKLQELKNEVKEVKIEEG<br>NAGKFRRARYLRYSDENVLSIVYLLIGYLRYLIDHRSLGSL<br>RHDIDIETPQEEHYNNSEKGTTLNIKYGRRCCISTFIMYLIL<br>FAGVGIWLGARAQVVWRLPPLVVPVDDTEIIFWDCWAPE<br>EPACQDFLGTMIYLKANVNISIQEGPTLGNWAREIWSTL<br>FKKATRQCRRGRIWRRWNETITGPLGCANNTCYNISVVV<br>PDYQCYVDRVDTWLQGKVNISLCLTGGKMLYNKETRQL<br>SYCTDPLQIPLINYTFGPNQTCMWNTSLIKDSEIPKCGWW<br>NQVAYYDTCKWEEANVTFQCHRTQSQSGSWIRTISSWKQ<br>RNRWEWRPDFESEKVKISLQCNSTKNLTFAMRSSSDYYD<br>VQGAWIEFGCHRNKSKRHSEARFRIRCKWNEGNNISLIDT<br>CGTNPNVTGANPVDCTMKANTMYNCSLQDSFTMKIEDLI<br>VHFNMTKAVELYNIAGNWSCTSDLPKGWGYMNCNCTN<br>GTDNSETKMACPKNQGILRNWYNPVAGLRQALIKYQVV<br>KQPEYLIVPEEVMQYKFKQKRAAIHIMLALATVLSMAGA<br>GTGATAIGMVTQYHQVLATHQQALEKITEALKINNLRLIT<br>LEHQVLVIGLRVEAIEKF**R**YTAFAMQELGCNQNQFFCKIP<br>PSLWSMYNMTLNQTIWNHGNISLGNWYNQTRDLQNKFY<br>EIIMDIEQNNVQGKTGIQQLQKWENWVGWIGKIPQYLKG<br>LLGSVLGIGLGILLLLICLPTLVDCIRNCTNKILGYTVIAMP<br>EIDDEEVHLSVELRRNGRQCGISEKEEE | SEQ ID NO: 411 |

(continued)

| | |
|---|---|
| MAEGGFTQNQQWIGPEEAEELLDFDIAVQMNEEGPLHPGI NPFRVPGITPQEKEDYCKILQPKLQELKGEIKEVKLEERNA GKFRRARYLRYSDENVLSIIYLLTGYFRYLINHRSLGALRH DIDIEAPQKEQYSNNEKGTTLNIKYGRVCCISTLLLYLLLF AGLGVWYGTSAQVVWRLPPLVVPVEDTEIIFWDCWAPEE PACQDFLGTMIHLKANVNISIQEGPTLGNWAREIWSTLFK KATRQCRRGRIWRRWNETITGPLGCANNTCYNISVVVPD YQCYVDRVDTWLQGKVNISLCLTGGKMLYNKETRQLSY CTDPLQIPLINYTFGPNQTCMWNTSLIKDSEIPKCGWWNQ VAYYNACKWEEANVTFQCHRTQSQPGSWIRTISSWKQRN RWEWRPDFESEKVKISLQCNSTKNLTFAMRSSSDYYDIQG AWIEFGYHRNKSKMHSEVRFRIRCKWNEGNNISLIDTCGT NPNVTGANPVDCTMKANTMYNCSLQDSFTMKIEDLIVHF NMTKAVEMYNIAGNWSCTSDLPKGWGYMNCNCTNGTD ESGTKMACPKSQGILRNWYNPVAGLRQALIKYQVVKQPE YLIVPEEVMQYKFKQKRAAIHIMLALATVLSMAGAGTGA TAIGMVTQYHQVLATHQQALDKITQALKINNLRLITLEHQ VLVIGLKVEAIEK**R**LYTAFAMQELGCNQNQFFCKIPPSLW SMYNMTLNQTIWNHGNISLGNWYNQTKDLQNKFYEIIMD IEQNNVQGKTGIQQLQKWENWVGWIGKIPQYLKGLLGSV LGIGVGILLLIICLPTLVDCVRNCINKVLGYSVIAMPELDDE EVSMELRRNGRQCGMSEKEEE | SEQ ID NO: 412 |
| MAEGGFTQNQQWIGPEEAEELLDFDIAVQMNEEGPLHPGI NPFRVPGITPQEKEDYCKILQPKLQELKGEIKEVKLEERNA GKFRRARYLRYSDENVLSIIYLLTGYFRYLINHRSLGALRH DIDIEAPQKEQYSNNEKGTTLNIKYGRVCCISTLLLYLLLF AGLGVWYGTSAQVVWRLPPLVVPVEDTEIIFWDCWAPEE PACQDFLGTMIHLKANVNISIQEGPTLGNWAREIWSTLFK KATRQCRRGRIWRRWNETITGPLGCANNTCYNISVVVPD YQCYVDRVDTWLQGKVNISLCLTGGKMLYNKETRQLSY CTDPLQIPLINYTFGPNQTCMWNTSLIKDSEIPKCGWWNQ VAYYNACKWEEANVTFQCHRTQSQPGSWIRTISSWKQRN RWEWRPDFESEKVKISLQCNSTKNLTFAMRSSSDYYDIQG AWIEFGYHRNKSKMHSEVRFRIRCKWNEGNNISLIDTCGT NPNVTGANPVDCTMKANTMYNCSLQDSFTMKIEDLIVHF NMTKAVEMYNIAGNWSCTSDLPKGWGYMNCNCTNGTD ESGTKMACPKSQGILRNWYNPVAGLRQALIKYQVVKQPE YLIVPEEVMQYKFKQKRAAIHIMLALATVLSMAGAGTGA TAIGMVTQYHQVLATHQQALDKITQALKINNLRLITLEHQ VLVIGLKVEAIEKF**R**YTAFAMQELGCNQNQFFCKIPPSLW SMYNMTLNQTIWNHGNISLGNWYNQTKDLQNKFYEIIMD IEQNNVQGKTGIQQLQKWENWVGWIGKIPQYLKGLLGSV LGIGVGILLLIICLPTLVDCVRNCINKVLGYSVIAMPELDDE EVSMELRRNGRQCGMSEKEEE | SEQ ID NO: 413 |

(continued)

| | |
|---|---|
| MAEGGFTQNQQWIGPEEAEELLDFDIAVQMNEEGPLNPG VNPFRVPGITSQEKDDYCKVLQTKLQELKNEVKEVKIEEG NAGKFRRARYLRYSDENVLSIVYLLIGYLRYLIDHRSLGSL RHDIDIETPQEEHYNNSEKGTTLNTKYGRRCCLSTFIMYL VLFAGVGLWLGARAQVVWRLPPLVVPVDDTEIIFWDCW APEEPACQDFLGTMIYLKANVNISIQEGPTLGNWAREIWS TLFKKATRQCRRGRIWKRWNETITGPLGCATNTCYNISVV VPDYQCYVDRVDTWLQGKVNISLCLTGGKMLYNKETRQ LSYCTDPLQIPLINYTFGPNQTCMWNTSLIKDSEIPKCGW WNQVAYYNACKWEEANVTFQCQRTQSQSGSWIRTISSW KQRNRWEWRPDFESEKVKISLQCNSTKNLTFAMRSSSDY YDVQGAWIEFGCHRNKSKKYSEARFRIRCKWNEGKNISLI DTCGTNPNVTGANPVDCTMKANTMYNCSLQDSFTMKIE DLIVHFNMTKAVEMYNIAGNWSCTSDLPKGWGYMNCNC TNGTDNSETKMTCPENQGILRNWYNPVAGLRQALIKYQV VKQPEYLIVPEEVMQYKFKQKRAAIHIMLALATVLSMAG AGTGATAIGMVTQYHQVLATHQQALEKITEALKINNLRLI TLEHQVLVIGLKVEAIEK**R**LYTAFAMQELGCHQNQFFCKI PPSLWSMYNMTLNQTIWNHGNISLGNWYNQTRDLQNKF YEIIMDIEQNNVQGKTGIQQLQKWENWMGWIGKIPQYLK GLLGSVLGIGLGILLLLICLPTLVDCIRNCTNKILGYTVIAM PEIDDEEVHLSVELRRNGRQCGISEKEEE | SEQ ID NO: 414 |
| MAEGGFTQNQQWIGPEEAEELLDFDIAVQMNEEGPLNPG VNPFRVPGITSQEKDDYCKVLQTKLQELKNEVKEVKIEEG NAGKFRRARYLRYSDENVLSIVYLLIGYLRYLIDHRSLGSL RHDIDIETPQEEHYNNSEKGTTLNTKYGRRCCLSTFIMYL VLFAGVGLWLGARAQVVWRLPPLVVPVDDTEIIFWDCW APEEPACQDFLGTMIYLKANVNISIQEGPTLGNWAREIWS TLFKKATRQCRRGRIWKRWNETITGPLGCATNTCYNISVV VPDYQCYVDRVDTWLQGKVNISLCLTGGKMLYNKETRQ LSYCTDPLQIPLINYTFGPNQTCMWNTSLIKDSEIPKCGW WNQVAYYNACKWEEANVTFQCQRTQSQSGSWIRTISSW KQRNRWEWRPDFESEKVKISLQCNSTKNLTFAMRSSSDY YDVQGAWIEFGCHRNKSKKYSEARFRIRCKWNEGKNISLI DTCGTNPNVTGANPVDCTMKANTMYNCSLQDSFTMKIE DLIVHFNMTKAVEMYNIAGNWSCTSDLPKGWGYMNCNC TNGTDNSETKMTCPENQGILRNWYNPVAGLRQALIKYQV VKQPEYLIVPEEVMQYKFKQKRAAIHIMLALATVLSMAG AGTGATAIGMVTQYHQVLATHQQALEKITEALKINNLRLI TLEHQVLVIGLKVEAIEKF**R**YTAFAMQELGCHQNQFFCKI PPSLWSMYNMTLNQTIWNHGNISLGNWYNQTRDLQNKF YEIIMDIEQNNVQGKTGIQQLQKWENWMGWIGKIPQYLK GLLGSVLGIGLGILLLLICLPTLVDCIRNCTNKILGYTVIAM PEIDDEEVHLSVELRRNGRQCGISEKEEE | SEQ ID NO: 415 |

(continued)

| | |
|---|---|
| MAEGGFTQNQQWIGPEEAEELLDFDIAVQMNEEGPLNPG VNPFRVPGITSQEKDDYCKILQTKLRELKNEVKEVKIEEG NAGKLRRARYLRYSDENVLSIVYLLIGYLRYLIDHRSLGS LRHDIDIETPQEEHYNNSEKGTTLNIKYEGRCCLSTFIMHLI LFAGVGIWLGARAQVVWRLPPLVVPVDDTEMIFWDCWA PEEPACQDFLGTMIHLKANVNISIQEGPTLGNWAREIWST LFKKATRQCRRGKIWRRWNETITGPLGCANNTCYNISVV VPDYQCYVDRVDTWLQGKVNISLCLTGGKMLYNKETRQ LSYCTDPLQIPLINYTFGPNQTCMWNASLIKDSEIPKCGW WNQAAYYNACKWEEANVTFQCHRTQSQSGSWIRTISSW RQRNRWEWRPDFESEKVKISLQCNSTKNLTFAMRSSSDY YDVQGAWIEFGCHRKKSNKHSEARFRIRCTWNEGNNISLI DTCGTNPNVTGANPVDCTMKANVMYNCTLQDSFTMKIE DLIVHFNMTKAVEMYNIAGNWSCTSDLPKGWGYMNCNC TNGTDNTKMTCPKNQGILRNWYNPVAGLRQALIKYQVV KQPEYLIVPEEVMQYKVKQKRAAIHIMLALATVLSMAGA GTGATAIGMVTQYHQVLATHQQALDKITEALKINNLRLIT LEHQVLVIGLKVEAIEK**R**LYTAFAMQELGCNQNQFFCKIP PSLWSMYNMTLNQTIWNHGNISLGNWYNQTKDLQNKFY EIIMDIEQNNVQGKTGIQQLQKWENWVGWIGKIPQYLKG LLGSVLGIGLGILLLLICLPTLVDCIRSCTNRILGYTVIAMPE IDDEEVHLSVELRRNGRQCGISEKEEE | SEQ ID NO: 416 |
| MAEGGFTQNQQWIGPEEAEELLDFDIAVQMNEEGPLNPG VNPFRVPGITSQEKDDYCKILQTKLRELKNEVKEVKIEEG NAGKLRRARYLRYSDENVLSIVYLLIGYLRYLIDHRSLGS LRHDIDIETPQEEHYNNSEKGTTLNIKYEGRCCLSTFIMHLI LFAGVGIWLGARAQVVWRLPPLVVPVDDTEMIFWDCWA PEEPACQDFLGTMIHLKANVNISIQEGPTLGNWAREIWST LFKKATRQCRRGKIWRRWNETITGPLGCANNTCYNISVV VPDYQCYVDRVDTWLQGKVNISLCLTGGKMLYNKETRQ LSYCTDPLQIPLINYTFGPNQTCMWNASLIKDSEIPKCGW WNQAAYYNACKWEEANVTFQCHRTQSQSGSWIRTISSW RQRNRWEWRPDFESEKVKISLQCNSTKNLTFAMRSSSDY YDVQGAWIEFGCHRKKSNKHSEARFRIRCTWNEGNNISLI DTCGTNPNVTGANPVDCTMKANVMYNCTLQDSFTMKIE DLIVHFNMTKAVEMYNIAGNWSCTSDLPKGWGYMNCNC TNGTDNTKMTCPKNQGILRNWYNPVAGLRQALIKYQVV KQPEYLIVPEEVMQYKVKQKRAAIHIMLALATVLSMAGA GTGATAIGMVTQYHQVLATHQQALDKITEALKINNLRLIT LEHQVLVIGLKVEAIEKF**R**YTAFAMQELGCNQNQFFCKIP PSLWSMYNMTLNQTIWNHGNISLGNWYNQTKDLQNKFY EIIMDIEQNNVQGKTGIQQLQKWENWVGWIGKIPQYLKG LLGSVLGIGLGILLLLICLPTLVDCIRSCTNRILGYTVIAMPE IDDEEVHLSVELRRNGRQCGISEKEEE | SEQ ID NO: 417 |
| MADGGFSQNQQWIGPEEAEKSLDFDIATQMNEEGPLNPG | SEQ ID NO: 418 |

(continued)

| | |
|---|---|
| VNPFRVPGITPQEKEDYCKILQTRLQELKNELKEVKVEQR NAGKFRRTRFLRYSDENVLSIVYLLIGYLRYLIDRRSLGSL RHDIDIKIPQEEHYNNSAKDTTLNIKYERRCCIGTFIMYLIL FAGIGIWFGAKAQVVWRLPPLVVPVEESEIIFWDCWAPEE PACQDFLGAMIHLKANTNISIQEGPTLGNWAREIWSTLFK KATKQCRKGGIWTKWKETITGPLGCANNTCYNISVVVPD YQCYVDRVDTWLQGKVNISLCLTGGKMLFNKETKQLSY CTDPLQIPLINYTFGPNQTCMWNTSQIQDSEIPKCGWWNQ IAYYNSCQWEKTDVKFHCQRTQSQPGSWRRAISSWRQRN RWEWRPDFESKKVKVSLKCNSTKNLTFAMRSSGDYGEV TGAWIEFGCHRNKSKLHTEARFRIRCKWNEGNNISLIDTC GTNPNVAEANPVDCTMKANTMYNCSLQDSFTMKIEDLIV HFNMTKAVEMYNIAGNWSCTSDLPKGWGYMNCNCTNG TTSNGIKMACPKQQGILRNWYNPVAGLRQALIKYQVVKQ PEYLIVPEEVLQYKFKQKRAAIHIMLALATVLSMAGAGTG ATAIGVVTQYHQVLATHQQALDKITEALKINNLRLITLEH QVLVIGLKVEAIEK**R**LYTAFAMQELGCNQNQFFCKIPLNL WNMYNMTLNQTIWNHGNISLGNWYNQTRDLQNKFYEII MDIEQNNVQGHTGIQQLQKWENWVGWIGKIPQYLKGLIG SVLGIGLGILLLLICLPTLVDCIRSCTNKMLGYTVIAMPEIG DEGVHLAMELRRNGRQCGISEKEEE | |
| MADGGFSQNQQWIGPEEAEKSLDFDIATQMNEEGPLNPG VNPFRVPGITPQEKEDYCKILQTRLQELKNELKEVKVEQR NAGKFRRTRFLRYSDENVLSIVYLLIGYLRYLIDRRSLGSL RHDIDIKIPQEEHYNNSAKDTTLNIKYERRCCIGTFIMYLIL FAGIGIWFGAKAQVVWRLPPLVVPVEESEIIFWDCWAPEE PACQDFLGAMIHLKANTNISIQEGPTLGNWAREIWSTLFK KATKQCRKGGIWTKWKETITGPLGCANNTCYNISVVVPD YQCYVDRVDTWLQGKVNISLCLTGGKMLFNKETKQLSY CTDPLQIPLINYTFGPNQTCMWNTSQIQDSEIPKCGWWNQ IAYYNSCQWEKTDVKFHCQRTQSQPGSWRRAISSWRQRN RWEWRPDFESKKVKVSLKCNSTKNLTFAMRSSGDYGEV TGAWIEFGCHRNKSKLHTEARFRIRCKWNEGNNISLIDTC GTNPNVAEANPVDCTMKANTMYNCSLQDSFTMKIEDLIV HFNMTKAVEMYNIAGNWSCTSDLPKGWGYMNCNCTNG TTSNGIKMACPKQQGILRNWYNPVAGLRQALIKYQVVKQ PEYLIVPEEVLQYKFKQKRAAIHIMLALATVLSMAGAGTG ATAIGVVTQYHQVLATHQQALDKITEALKINNLRLITLEH QVLVIGLKVEAIEKF**R**YTAFAMQELGCNQNQFFCKIPLNL WNMYNMTLNQTIWNHGNISLGNWYNQTRDLQNKFYEII MDIEQNNVQGHTGIQQLQKWENWVGWIGKIPQYLKGLIG SVLGIGLGILLLLICLPTLVDCIRSCTNKMLGYTVIAMPEIG DEGVHLAMELRRNGRQCGISEKEEE | SEQ ID NO: 419 |

The invention also encompasses the variants of the above sequences, harbouring the above mentioned mutations, and conferring to said variant a decrease of immunosuppressive properties, a substantial absence of immunosuppressive properties or an absence of immunosuppressive properties.

Advantageously: the mutated feline lentiviral proteins are:

1 - SEQ ID NO: 5, SEQ ID NO: 317 to 342 and SEQ ID NO: 374 to 419; these mutated ENV proteins having decreased immunosuppressive properties, substantially no immunosuppressive properties or no immunosuppressive properties. advantageously,

2- SEQ ID NO: 5, SEQ ID NO: 317 to 342 and SEQ ID NO: 374 to 419; these mutated ENV proteins having decreased immunosuppressive properties, substantially no immunosuppressive properties or no immunosuppressive properties, and being highly expressed at the plasma membrane,

more advantageously,

3- SEQ ID NO: 5, SEQ ID NO: 317 to 342, and SEQ ID NO: 374 to 419; these mutated ENV proteins having decreased immunosuppressive properties, substantially no immunosuppressive properties or no immunosuppressive properties, being highly expressed at the plasma membrane and possessing a medium or high fusogenic activity.

As mentioned above "possessing a medium or high fusogenic activity" means that when the ENV preteins are expressed at a cell plasma membrane, by using appropriate expression vectors the fusogenic activity of said proteins as measured by quantitating the ENV-mediated cell-cell fusion (see Materials and Methods) is:

- either at a level comparable or higher to the ability of the wild type ENV, i.e. high ability
- or at a lower level compared to the ability of the wild type ENV, i.e. medium or low ability.

In another embodiment, the invention relates to a pharmaceutical composition as defined above, wherein said isolated mutated feline lentiviral ENV protein or said fragment thereof, comprises one of the amino acid sequences SEQ ID NO: 28, 29, 30, 31, 32, 33, 34, 35, 36, 37, 38, 39, 76, 77, 78, 79, 80, 81, 82, 83, 116, 117, 118, 119, 156, 157, 158 and 159, in particular, wherein said isolated mutated feline lentiviral ENV protein consists of one of the amino acid sequences : SEQ ID NO: 5 and SEQ ID NO: 320, 324, 329, 334 and 374 to 419.

In another aspect, the invention relates to a pharmaceutical composition comprising as active substance a nucleic acid molecule coding for a mutated feline lentiviral ENV protein, or a fragment of said mutated feline lentiviral ENV protein, as defined above.

In a particular embodiment, the invention concerns a pharmaceutical as defined above, wherein said nucleic acid molecule is contained in a vector, said vector comprising means allowing the expression of the mutated feline lentiviral ENV protein, or a fragment of said mutated feline lentiviral ENV protein, as defined above.

In another embodiment, the invention relates to a pharmaceutical composition as defined above, wherein said nucleic acid molecule is comprised in a vector, said nucleic acid molecule being placed under the control of sequences that allow the expression of said mutated lentiviral ENV protein, or variant of said protein, or fragments thereof.

In another embodiment, the invention relates to a pharmaceutical composition, as defined above, in particular as a vaccine, comprising a DNA molecule coding for said mutated feline lentiviral ENV protein or a fragment thereof.

DNA vaccines expressing ENV proteins can be produced as described in Bellier et al. (Vaccine, 2009, 27(42):5772-80).

In another embodiment, the invention concerns a pharmaceutical as defined above, wherein said vector is chosen among a canarypox vector, a pox vector, a fowlpox, an adenoviral vector, a lentiviral vector, a measles vector, a Sendai virus vector, a Cytomegalovirus vector or a Modified Vaccinia Ankara vector.

In another embodiment, the invention concerns a pharmaceutical as defined above, comprising at least one nucleic acid molecule coding for a GAG protein and/or a PRO protein and/or a POL protein of a feline lentivirus, said lentivirus being preferably of the same origin as the mutated lentiviral ENV protein.

GAG expression will produce virus-like particles (VLPs) which are particularly advantageous for a vaccine, in particular if the ENV protein is associated with the VLP (Guerbois et al., Virology, 2009, 388:191-203).

In another embodiment, the invention concerns a pharmaceutical as defined above, wherein said nucleic acid molecule coding for a mutated feline lentiviral ENV protein, or a fragment of said mutated feline lentiviral ENV protein, is contained in the same vector as the one which also contains said at least one nucleic acid molecule coding for a GAG protein and/or a PRO protein and/or a POL protein.

In another embodiment, the invention concerns a pharmaceutical as defined above, wherein said nucleic acid molecule coding for a mutated feline lentiviral ENV protein, or a fragment of said mutated feline lentiviral ENV protein, is contained in the same vector as the one which also contains all said at least one nucleic acid molecule coding for a GAG protein and/or a PRO protein and/or a POL protein.

In another embodiment, the invention concerns a pharmaceutical as defined above, wherein said nucleic acid molecule coding for a mutated feline lentiviral ENV protein, or a fragment of said mutated feline lentiviral ENV protein, is contained in a vector which is different from the at least one vector containing said at least one nucleic acid molecule coding for a GAG protein and/or a PRO protein and/or a POL protein.

In another embodiment, the invention concerns a pharmaceutical as defined above, wherein said nucleic acid molecule coding for a mutated feline lentiviral ENV protein, or a fragment of said mutated feline lentiviral ENV protein, said nucleic acid molecule coding for a GAG protein, said nucleic acid molecule coding for a PRO protein and said nucleic acid molecule coding for a POL protein, are all contained in vectors which are different from each other.

In another embodiment, the invention concerns a pharmaceutical as defined above, comprising at least one nucleic acid molecule coding for a GAG protein of a feline lentivirus, said lentivirus being preferably of the same origin as the mutated lentiviral ENV protein.

In another embodiment, the invention concerns a pharmaceutical as defined above, wherein said nucleic acid molecule coding for a mutated feline lentiviral ENV protein, or a fragment of said mutated feline lentiviral ENV protein, is contained in the same vector as the one which also contains said at least one nucleic acid molecule coding for a GAG protein.

In another embodiment, the invention concerns a pharmaceutical as defined above, wherein said nucleic acid molecule coding for a mutated feline lentiviral ENV protein, or a fragment of said mutated feline lentiviral ENV protein, is contained in the same vector as the one which also contains a nucleic acid molecule coding for a GAG protein.

In another embodiment, the invention concerns a pharmaceutical as defined above, wherein said nucleic acid molecule coding for a mutated feline lentiviral ENV protein, or a fragment of said mutated feline lentiviral ENV protein, is contained in the same vector as the one which also contains a nucleic acid molecule coding for a GAG protein, said vector being preferably a canary pox vector (Poulet et al., Veterinary Record, 2003, 153(5):141-145 ; Vaccari et al., Expert Review of Vaccines, 2010, Vol. 9, No 9: 997-1005).

In another embodiment, the invention concerns a pharmaceutical as defined above, wherein said nucleic acid molecule coding for a mutated feline lentiviral ENV protein, or a fragment of said mutated feline lentiviral ENV protein, is contained in a vector which is different from the at least one vector containing said at least one nucleic acid molecule coding for a GAG protein.

In another embodiment, the invention concerns a pharmaceutical as defined above, wherein said nucleic acid molecule coding for a mutated feline lentiviral ENV protein, or a fragment of said mutated feline lentiviral ENV protein, and said nucleic acid molecule coding for a GAG protein are contained in vectors which are different from each other.

In another aspect, the invention concerns a pharmaceutical composition as defined above, in association with at least one antiviral compound, preferably for a simultaneous, separated or sequential use.

The composition according to the invention can also be used in combination with the antiviral compositions listed in Table 1 below:

**Table 1: Examples of antiviral compounds.**

| | |
|---|---|
| **Intelence**® (TMC 125/etravirine) Tibotec | Non-nucleoside reverse transcriptase inhibitor |
| **Agenerase**®(APV/amprenavir) GSK - | Protease inhibitor |
| **Aptivus**®(TPV/tipranavir) Boehringer - | Protease inhibitor |
| **Crixivan**®(IDV/indinavir) MSD - | Protease inhibitor |
| **Invirase**®(SQV/saquinavir) Roche - | Protease inhibitor |
| **Kaletra**®(LPV.r/lopinavir + ritonavir) Abbott | Protease inhibitor |
| **Norvir**®(ritonavir) Abbott - | Protease inhibitor |
| **Prezista**®(TMC 114/darunavir) Tibotec/Janssen-Cilag - | Protease inhibitor |
| **Reyataz**®(ATZ/atazanavir) BMS - | Protease inhibitor |
| **Telzir**®(APV/fosamprenavir) GSK - | Protease inhibitor |
| **Viracept**®(nelfinavir) Roche - | Protease inhibitor |
| **Fuzeon**®(T20/enfuvirtide) Roche - | Fusion inhibitor |
| **Celsentri**®(maraviroc) Pfizer - | Entry inhibitor |
| **Isentress**®(MK 0518/raltegravir) Merck - | Integrase inhibitor |
| **Rescriptor**®(delavirdine) Agouron - | Non-nucleoside reverse transcriptase inhibitor |
| **Sustiva**®(EFV/efavirenz) BMS - | Non-nucleoside reverse transcriptase inhibitor |
| **Viramune**®(nevirapine) Boehringer - | Non-nucleoside reverse transcriptase inhibitor |

(continued)

| Combivir®(Retrovir®+Epivir®) GSK - | Nucleoside reverse transcriptase inhibitor |
|---|---|
| Emtriva®(FTC, emtricitabine) Gilead - | Nucleoside reverse transcriptase inhibitor |
| Epivir®(3TC, lamivudine) GSK - | Nucleoside reverse transcriptase inhibitor |
| Kivexa®(Ziagen®+ Epivir®) GSK - | Nucleoside reverse transcriptase inhibitor |
| Retrovir®(AZT/zidovudine) QSK - | Nucleoside reverse transcriptase inhibitor |
| Trizivir®(Retrovir®+ Epivir®+ Ziagen®) GSK - | Nucleoside reverse transcriptase inhibitor |
| Videx®(ddI/didanosine) BMS - | Nucleoside reverse transcriptase inhibitor |
| Viread®(TDF/tenofovir) Gilead - | Nucleoside reverse transcriptase inhibitor |
| Zerit®(d4T/stavudine) BMS - | Nucleoside reverse transcriptase inhibitor |
| Ziagen®(ABC/abacavir) GSK - | Nucleoside reverse transcriptase inhibitor |
| Truvada®(Emtriva® + Viread®) Gilead - | Nucleoside reverse transcriptase inhibitor |
| Atripla®(Sustiva®+ Emtriva®+ Viread®) BMS / GILEAD | Nucleoside and non-nucleoside reverse transcriptase inhibitor |

In another aspect, the invention concerns a pharmaceutical composition as defined above, for its use for stimulating an immune response in a host organism.

In another aspect, the invention concerns a pharmaceutical composition as defined above, for its use for the prevention or the treatment of lentiviral infection, preferably FIV infection.

In another aspect, the invention concerns a pharmaceutical composition as defined above, for its use as a vaccine, in particular against FIV infection.

As mentioned above, the pharmaceutical composition according to the invention encompasses a vaccine, comprising as active substance, a protein as defined above, or a nucleic acid molecule as defined above, or a vector as defined above, or a combination thereof.

In a particular embodiment, the pharmaceutical composition according to the invention encompasses a vaccine as defined above, comprising a nucleic acid coding for a GAG protein as defined above and a nucleic acid coding for a mutated ENV protein as defined above, which are contained in a same vector, said same vector being preferably a canary pox vector.

In a particular embodiment, the pharmaceutical composition according to the invention encompasses a vaccine, comprising a GAG protein as defined above and a mutated ENV protein as defined above.

In a particular embodiment, the pharmaceutical composition according to the invention encompasses a vaccine, comprising a GAG protein as defined above and a mutated ENV protein as defined above which are associated to at least one adjuvant.

As examples of adjuvants, a non limitative list is given below:

- Aluminum-based compounds such as aluminum phosphate and aluminum hydroxide.
- Immunostimulatory adjuvants like CpG, MPL and QS21 orMF59 which is a squalene oil-in water emulsions.
- Freunds incomplete adjuvant (FIA) or Freund's complete adjuvant (FCA), can also be used.
- Calcium phosphate in particular orthophosphates, metaphosphates or pyrophosphates and occasionally hydrogen or hydroxide ions.
- Muramyl dipeptide (N-Acetyl muramyl-L-alanine-D-isoglutamine, MDP) and its synthetic analogs such as muramyl tripeptide phosphatidylethanolamine MTPPTdEtn),
- Trehalose-6,6'-dimycolate,
- Saponins (Triterpenoid glycosides) like QS-21
- Stearyl Tyrosine (octadecyl ester hydrochloride salt of tyrosine)
- Polysaccharides like:

  ◦ Chitosan:
  ◦ Inulin:
  ◦ Beta - Glucans
  ◦ Lipo-Polysaccharides or endotoxin

○ MGN-3Actinidia eriantha (AEPS):
○ Eldexomer:
○ CpG ODN

- Liposomes like

  ○ Dehydration-rehydration liposome vesicles (DRVs)
  ○ Cytotoxic T lymphocyte (CTL)
  ○ CAF01
  ○ Liposomes containing lipid A (LA)

- Lipid Polysine Core Peptides (LCP)
- Cytokine like GM-CSF, IL-2,IL-12 or IL-15
- Lipid A and Monophosphoryl Lipid A (MPL)
- Lipopeptide which are molecules consisting of lipid connected to a peptide. (They are derived from the lipoprotein of bacterial cell wall.)
- Exogenous Immunostimulatory Adjuvants which are compounds, or proteins that are not found in the human body like:

  ○ Proteosomes in particular Multiple Antigenic Peptides (MAP) or Exogenous Toxins.

Suitably, the total amount of mutated lentiviral ENV protein in a single dose of the immunogenic composition is 1-500 μg and/or the total amount of unfused polypeptides in a single dose of the immunogenic composition is 1-500 μg. In one embodiment, the total amount of all antigens in a single dose of the immunogenic composition is 1-1000 μg, 10-500 μg, 100-200 μg, or around 100 μg.

The amount of mutated feline lentiviral ENV protein in a dose of the immunogenic composition is selected as an amount which induces an immune response without significant, adverse side effects in typical recipients. Such amount will vary depending upon which specific immunogen is employed and the dosing or vaccination regimen that is selected. An optimal amount for a particular immunogenic composition can be ascertained by standard studies involving observation of relevant immune responses in infected animals.

Administration of the pharmaceutical composition can take the form of one or of more than one individual dose, for example as repeat doses of the same polypeptide containing composition, or in a heterologous "prime-boost" vaccination regime, including proteins and vectors. In one embodiment, the immunogenic composition of the invention is initially administered to a subject as two or three doses, wherein the doses are separated by a period of two weeks to three months, preferably one month. Conveniently, the composition is administered to an animal (for instance as a booster) every 6-24, or 9-18 months, for instance annually. For instance, the composition is administered to an animal (for instance as a booster) at six month or 1 year intervals. Suitably in this respect, subsequent administrations of the composition to the animal boost the immune response of earlier administrations of the composition to the same animal.

*In* an embodiment, the immunogenic composition of the invention is used as part of a prime-boost regimen for use in the treatment or prevention of disease or infection by FIV strains from one or more clades different from the one or more FIV clades in the immunogenic composition. Conveniently, the composition is the priming dose. Alternatively, the composition is the boosting dose.

Suitably, two or more priming and/ or boosting doses are administered. A heterologous prime-boost regime uses administration of different forms of immunogenic composition or vaccine in the prime and the boost, each of which can itself include two or more administrations. The priming composition and the boosting composition will have at least one antigen in common, although it is not necessarily an identical form of the antigen, it can be a different form of the same antigen.

Prime boost immunisations according to the invention can be homologous prime-boost regimes or heterologous prime-boost regimes. Homologous prime-boost regimes utilize the same composition for prime and boost, for instance the immunogenic composition of the invention. Heterologous prime-boost regimes can be performed with a combination of protein and DNA-based formulations. Such a strategy is considered to be effective in inducing broad immune responses. Adjuvanted protein vaccines induce mainly antibodies and CD4+ T cell immune responses, while delivery of DNA as a plasmid or a recombinant vector induces strong CD8+ T cell responses. Thus, the combination of protein and DNA vaccination can provide for a wide variety of immune responses. This is particularly relevant in the context of FIV, since neutralizing antibodies, CD4+ T cells and CD8+ T cells are thought to be important for the immune defense against FIV.

The invention also relates to a method for treating animals afflicted by pathologies related to lentiviral infections, comprising the administration to an animal in a need thereof of a pharmaceutically efficient amount of the pharmaceutical composition as defined above.

The invention also relates to a pharmaceutical composition as defined above, as a vaccine, for its use for the prevention or the traitement of FIV infection, or pathologies related to feline immunodeficiency.

In another aspect, the invention relates to a pharmaceutical composition comprising as active substance:

an isolated non naturally occurring mutated feline lentiviral ENV having a decreased immunosuppressive activity, substantially no immunosuppressive activity or no immunosuppressive activity, said mutated feline lentiviral ENV resulting from mutation of the transmembrane subunit (TM) of a wild type feline lentiviral ENV protein, the transmembrane subunit comprising an immunosuppressive domain (ISU) comprising the following amino acid sequence:

A-[I/M/T/L]-E-K-F-L-Y-T-A (SEQ ID NO: 343),

wherein the amino acids at the positions chosen among

- the position 3 of SEQ ID NO: 343, and/or
- the position 4 of SEQ ID NO: 343and/or
- the position 5 of SEQ ID NO: 343, and/or
- the position 6 of SEQ ID NO: 343, and/or
- the position 7 of SEQ ID NO: 343,

are:

- either deleted,
- or substituted by another amino acid such that

the amino acid residue at position 3 of SEQ ID NO: 1 is substituted by A, F, G, L, R, C, D, H, I, K, M, N, P, Q, S, T, V, W or Y, and/or
the amino acid residue at position 4 of SEQ ID NO: 1 is substituted by A, F, G, L, R, C, D, E, H, I, M, N, P, Q, S, T, V, W or Y, and/or
the amino acid residue at position 5 of SEQ ID NO: 1 is substituted by A, G, L, R, C, D, E, H, I, K, M, N, P, Q, S, T, V, W or Y, and/or
the amino acid residue at position 6 of SEQ ID NO: 1 is substituted by A, F, G, R, C, D, E, H, I, K, M, N, P, Q, S, T, V, W or Y, and/or
the amino acid residue at position 7 of SEQ ID NO: 1 is substituted by A, F, G, L, R, C, D, E, H, I, K, M, N, P, Q, S, T, V or W.

In another aspect, the description relates to a method to obtain the active substance of a pharmaceutical composition, as defined above, consisting in modifying the immunosuppressive property of:

a wild-type feline lentiviral ENV protein,
or a fragment of said wild-type feline lentiviral ENV protein, said fragment comprising at least 40 amino acids, in particular at least 60 amino acids,
said ENV protein or fragment thereof presenting a transmembrane subunit (TM) comprising an immunosuppressive domain (ISU) containing the following amino acid sequence:

A-[I/M/T/L]-$X_A$-$X_B$-$X_C$-$X_D$-$X_E$-T-A (SEQ ID NO: 344),
wherein
$X_A$ is C, D, E, H, I, K, M, N, P, Q, S, T, V, W or Y, and
$X_B$ is C, D, E, H, I, K, M, N, P, Q, S, T, V, W or Y, and
$X_C$ is F, C, D, E, H, I, K, M, N, P, Q, S, T, V, W or Y, and
$X_D$ is L, C, D, E, H, I, K, M, N, P, Q, S, T, V, W or Y, and
$X_E$ is C, D, E, H, I, K, M, N, P, Q, S, T, V, W or Y,
said method comprising a step of introduction of at least one mutation of $X_A$ and/or $X_B$ and/or $X_C$ and/or $X_D$ and/or $X_E$, to obtain:

an isolated mutated feline lentiviral ENV protein having a decreased immunosuppressive activity, substantially no immunosuppressive activity or no immunosuppressive activity,
said mutated feline lentiviral ENV protein having at least 70% identity, preferably at least 80% identity, to one sequence SEQ ID NO: 5,
or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,
said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),
wherein
$X_1$ is any amino acid different from E or deleted, and/or
$X_2$ is any amino acid different from K or deleted, and/or
$X_3$ is any amino acid different from F or deleted, and/or
$X_4$ is any amino acid different from L or deleted, and/or
$X_5$ is any amino acid different from Y or deleted.

In a particular embodiment, the description concerns a method to obtain the active substance of a pharmaceutical composition, as defined above, consisting in modifying the immunosuppressive property of a wild-type feline lentiviral ENV protein, or a fragment of said wild-type feline lentiviral ENV protein, said fragment comprising at least 40 amino acids, in particular at least 60 amino acids,
said ENV protein or fragment thereof presenting a transmembrane subunit (TM) comprising an immunosuppressive domain (ISU) containing the following amino acid sequence:
[V/I]-[E/R]-A-[I/M/T/L]-$X_A$-$X_B$-$X_C$-$X_D$-$X_E$-T-A-[F/L]-A-M (SEQ ID NO: 345),
wherein
$X_A$ is C, D, E, H, I, K, M, N, P, Q, S, T, V, W or Y, and
$X_B$ is C, D, E, H, I, K, M, N, P, Q, S, T, V, W or Y, and
$X_C$ is F, C, D, E, H, I, K, M, N, P, Q, S, T, V, W or Y, and
$X_D$ is L, C, D, E, H, I, K, M, N, P, Q, S, T, V, W or Y, and
$X_E$ is C, D, E, H, I, K, M, N, P, Q, S, T, V, W or Y,
said method comprising a step of introduction of at least one mutation of $X_A$ and/or $X_B$ and/or $X_C$ and/or $X_D$ and/or $X_E$, to obtain:

an isolated mutated feline lentiviral ENV protein having a decreased immunosuppressive activity, substantially no immunosuppressive activity or no immunosuppressive activity,
said mutated feline lentiviral ENV protein having at least 70% identity, preferably at least 80% identity, to one sequence SEQ ID NO: 5,
or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,
said mutated ENV protein and fragment thereof comprising a mutated immunosuppressive domain (ISU) containing the following amino sequence:
[V/I]-[E/R]-A-[I/M/T/L]-$X_1$-$X_2$-$X_3$-$X_4$-$X_5$-T-A-[F/L]-A-M (SEQ ID NO: 3),
wherein
$X_1$ is any amino acid different from E or deleted, and/or
$X_2$ is any amino acid different from K or deleted, and/or
$X_3$ is any amino acid different from F or deleted, and/or
$X_4$ is any amino acid different from L or deleted, and/or
$X_5$ is any amino acid different from Y or deleted.

In another embodiment, the description concerns a method to obtain the active substance of a pharmaceutical composition, as defined above, consisting in modifying the immunosuppressive property of a wild-type feline lentiviral ENV protein, or a fragment of said wild-type feline lentiviral ENV protein, said fragment comprising at least 40 amino acids, in particular at least 60 amino acids,
said ENV protein or fragment thereof presenting a transmembrane subunit (TM) comprising an immunosuppressive domain (ISU) containing the following amino acid sequence:
A-[I/M/T/L]-E-K-[F/P]-[L/V/I]-Y-T-A (SEQ ID NO: 346),
said method comprising a step of introduction of at least one mutation of E in position 3 and/or K in position 4 and/or [F/P] in position 5 and/or [L/V/I] in position 6 and/or Y in position 7,
to obtain:

an isolated mutated feline lentiviral ENV protein having a decreased immunosuppressive activity, substantially no immunosuppressive activity or no immunosuppressive activity,
or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,
said mutated ENV protein and fragment thereof comprising a mutated immunosuppressive domain (ISU) containing the following amino sequence:
A-[I/M/T/L]-X$_1$-X$_2$-X$_3$-X$_4$-X$_5$-T-A (SEQ ID NO: 1),
wherein
X$_1$ is any amino acid different from E or deleted, and/or
X$_2$ is any amino acid different from K or deleted, and/or
X$_3$ is any amino acid different from F or deleted, and/or
X$_4$ is any amino acid different from L or deleted, and/or
X$_5$ is any amino acid different from Y or deleted.

In another embodiment, the description concerns a method to obtain the active substance of a pharmaceutical composition, as defined above, consisting in modifying the immunosuppressive property of a wild-type feline lentiviral ENV protein, or a fragment of said wild-type feline lentiviral ENV protein, said fragment comprising at least 40 amino acids, in particular at least 60 amino acids,
said ENV protein or fragment thereof presenting a transmembrane subunit (TM) comprising an immunosuppressive domain (ISU) containing the following amino acid sequence:
[V/I]-[E/R]-A-[I/M/T/L]-E-K-[F/P]-[L/V/I]-Y-T-A-[F/L]-A-M (SEQ ID NO: 347),
said method comprising a step of introduction of at least one mutation of E in position 3 and/or K in position 4 and/or [F/P] in position 5 and/or [L/V/I] in position 6 and/or Y in position 7,
to obtain:

an isolated mutated feline lentiviral ENV protein having a decreased immunosuppressive activity, substantially no immunosuppressive activity or no immunosuppressive activity,
or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,
said mutated ENV protein and fragment thereof comprising a mutated immunosuppressive domain (ISU) containing the following amino sequence:
[V/I]-[E/R]-A-[I/M/T/L]-X$_1$-X$_2$-X$_3$-X$_4$-X$_5$-T-A-[F/L]-A-M (SEQ ID NO: 3),
wherein
X$_1$ is any amino acid different from E or deleted, and/or
X$_2$ is any amino acid different from K or deleted, and/or
X$_3$ is any amino acid different from F or deleted, and/or
X$_4$ is any amino acid different from L or deleted, and/or
X$_5$ is any amino acid different from Y or deleted.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,
or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,
said mutated ENV protein and fragment thereof comprising a mutated immunosuppressive domain (ISU) containing the following amino sequence:
A-[I/M/T/L]-X$_I$-X$_2$-X$_3$-X$_4$-X$_5$-T-A (SEQ ID NO: 1),
wherein
X$_1$ is any amino acid different from E or deleted, and X$_2$, X$_3$, X$_4$ and X$_5$ are any amino acid, or
X$_2$ is any amino acid different from K or deleted, and X$_1$, X$_3$, X$_4$ and X$_5$ are any amino acid, or
X$_3$ is any amino acid different from F or deleted, and X$_1$, X$_2$, X$_4$ and X$_5$ are any amino acid, or
X$_4$ is any amino acid different from L or deleted, and X$_1$, X$_2$, X$_3$ and X$_5$ are any amino acid, or
X$_5$ is any amino acid different from Y or deleted, and X$_1$, X$_2$, X$_3$ and X$_4$ are any amino acid, or
X$_1$ is any amino acid different from E or deleted, X$_2$ is any amino acid different from K or deleted, and X$_3$, X$_4$ and X$_5$ are any amino acid, or
X$_1$ is any amino acid different from E or deleted, X$_3$ is any amino acid different from F or deleted, and X$_2$, X$_4$ and X$_5$ are any amino acid, or
X$_1$ is any amino acid different from E or deleted, X$_4$ is any amino acid different from L or deleted, and X$_2$, X$_3$ and X$_5$ are any amino acid, or
X$_1$ is any amino acid different from E or deleted, X$_5$ is any amino acid different from Y or deleted, and X$_2$, X$_3$ and X$_4$ are any amino acid, or

$X_2$ is any amino acid different from K or deleted, $X_3$ is any amino acid different from F or deleted, and $X_1$, $X_4$ and $X_5$ are any amino acid, or

$X_2$ is any amino acid different from K or deleted, $X_4$ is any amino acid different from L or deleted, and $X_2$, $X_4$ and $X_5$ are any amino acid, or

$X_2$ is any amino acid different from K or deleted, $X_5$ is any amino acid different from Y or deleted, and $X_2$, $X_3$ and $X_4$ are any amino acid, or

$X_3$ is any amino acid different from F or deleted, $X_4$ is any amino acid different from L or deleted, and $X_2$, $X_4$ and $X_5$ are any amino acid, or

$X_3$ is any amino acid different from F or deleted, $X_5$ is any amino acid different from Y or deleted, and $X_2$, $X_3$ and $X_4$ are any amino acid, or

$X_4$ is any amino acid different from L or deleted, $X_5$ is any amino acid different from Y or deleted, and $X_2$, $X_4$ and $X_5$ are any amino acid, or

$X_1$ is any amino acid different from E or deleted, $X_2$ is any amino acid different from K or deleted, $X_3$ is any amino acid different from F or deleted, and $X_4$ and $X_5$ are any amino acid, or

$X_1$ is any amino acid different from E or deleted, $X_2$ is any amino acid different from K or deleted, $X_4$ is any amino acid different from L or deleted, and $X_3$ and $X_5$ are any amino acid, or

$X_1$ is any amino acid different from E or deleted, $X_2$ is any amino acid different from K or deleted, $X_5$ is any amino acid different from Y or deleted, and $X_3$ and $X_4$ are any amino acid, or

$X_1$ is any amino acid different from E or deleted, $X_3$ is any amino acid different from F or deleted, $X_4$ is any amino acid different from L or deleted, and $X_2$ and $X_5$ are any amino acid, or

$X_1$ is any amino acid different from E or deleted, $X_3$ is any amino acid different from F or deleted, $X_5$ is any amino acid different from Y or deleted, and $X_2$ and $X_4$ are any amino acid, or

$X_1$ is any amino acid different from E or deleted, $X_4$ is any amino acid different from L or deleted, $X_5$ is any amino acid different from Y or deleted, and $X_2$ and $X_3$ are any amino acid, or

$X_2$ is any amino acid different from K or deleted, $X_3$ is any amino acid different from F or deleted, $X_4$ is any amino acid different from L or deleted, and $X_1$ and $X_5$ are any amino acid, or

$X_2$ is any amino acid different from K or deleted, $X_3$ is any amino acid different from F or deleted, $X_5$ is any amino acid different from Y or deleted, and $X_1$ and $X_4$ are any amino acid, or

$X_2$ is any amino acid different from K or deleted, $X_4$ is any amino acid different from L or deleted, $X_5$ is any amino acid different from Y or deleted, and $X_1$ and $X_3$ are any amino acid, or

$X_3$ is any amino acid different from F or deleted, $X_4$ is any amino acid different from L or deleted, $X_5$ is any amino acid different from Y or deleted, and $X_1$ and $X_2$ are any amino acid, or

$X_1$ is any amino acid different from E or deleted, $X_2$ is any amino acid different from K or deleted, $X_3$ is any amino acid different from F or deleted, $X_4$ is any amino acid different from L or deleted, and $X_5$ is any amino acid, or

$X_1$ is any amino acid different from E or deleted, $X_2$ is any amino acid different from K or deleted, $X_3$ is any amino acid different from F or deleted, $X_5$ is any amino acid different from Y or deleted, and $X_4$ is any amino acid, or

$X_1$ is any amino acid different from E or deleted, $X_2$ is any amino acid different from K or deleted, $X_4$ is any amino acid different from L or deleted, $X_5$ is any amino acid different from Y or deleted, and $X_3$ is any amino acid, or

$X_1$ is any amino acid different from E or deleted, $X_3$ is any amino acid different from F or deleted, $X_4$ is any amino acid different from L or deleted, $X_5$ is any amino acid different from Y or deleted, and $X_2$ is any amino acid, or

$X_2$ is any amino acid different from K or deleted, $X_3$ is any amino acid different from F or deleted, $X_4$ is any amino acid different from L or deleted, $X_5$ is any amino acid different from Y or deleted and $X_1$ is any amino acid, or

$X_1$ is any amino acid different from E or deleted, $X_2$ is any amino acid different from K or deleted, $X_3$ is any amino acid different from F or deleted, $X_4$ is any amino acid different from L or deleted and $X_5$ is any amino acid different from Y or deleted.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_1$ is R, G, L, A, F or deleted, and/or

$X_2$ is R, G, L, A, F or deleted, and/or

$X_3$ is R, G, L, A, or deleted, and/or

X₄ is R, G, A, F or deleted, and/or
X₅ is R, G, L, A, F or deleted.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

[V/I]-[E/R]-A-[I/M/T/L]-X₁-X₂-X₃-X₄-X₅-T-A-[F/L]-A-M (SEQ ID NO: 3),

wherein

X₁ is R, G, L, A, F or deleted, and/or
X₂ is R, G, L, A, F or deleted, and/or
X₃ is R, G, L, A, or deleted, and/or
X₄ is R, G, A, F or deleted, and/or
X₅ is R, G, L, A, F or deleted.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity, or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-X₁-X₂-X₃-X₄-X₅-T-A (SEQ ID NO: 1),

wherein

X₁ is R, G, L, A, F or deleted, and/or
X₂ is G, L, F or deleted, and/or
X₃ is R, G, L, A, or deleted, and/or
X₄ is R, G, A, F or deleted, and/or
X₅ is G, L, F or deleted.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-X₁-X₂-X₃-X₄-X₅-T-A (SEQ ID NO: 1),

wherein

X₃ is R, G, L, A or deleted, X₄ is L, C, D, E, H, I, K, M, N, P, Q, S, T, V, W or Y, and X₁, X₂, X₅ are A, F, G, L, R, C, D, E, H, I, K, M, N, P, Q, S, T, V, W or Y, or

X₄ is R, G, A, F or deleted, X₃ is F, C, D, E, H, I, K, M, N, P, Q, S, T, V, W or Y, and X₁, X₂, X₅ are A, F, G, L, R, C, D, E, H, I, K, M, N, P, Q, S, T, V, W or Y, or

X₃ is R, G, L, A or deleted, X₄ is R, G, A, F or deleted, and X₁, X₂, X₅ are A, F, G, L, R, C, D, E, H, I, K, M, N, P, Q, S, T, V, W or Y.

In another embodiment, the invention concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-X₁-X₂-X₃-X₄-X₅-T-A (SEQ ID NO: 1),

wherein

$X_3$ is R, A or deleted, $X_4$ is L, C, D, E, H, I, K, M, N, P, Q, S, T, V, W or Y, and $X_{15}$ $X_2$, $X_5$ are A, F, G, L, R, C, D, E, H, I, K, M, N, P, Q, S, T, V, W or Y, or

$X_4$ is R, A or deleted, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or

$X_3$ is R, A or deleted, $X_4$ is R, A or deleted, and $X_1$, $X_2$, $X_5$ are A, F, G, L, R, C, D, E, H, I, K, M, N, P, Q, S, T, V, W or Y.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_3$ is R, $X_4$ is L, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or

$X_4$ is R, $X_3$ is F, C, D, E, H, I, K, M, N, P, Q, S, T, V, W or Y, and $X_1$, $X_2$, $X_5$ are A, F, G, L, R, C, D, E, H, I, K, M, N, P, Q, S, T, V, W or Y, or

$X_3$ is R, $X_4$ is R, and $X_1$, $X_2$, $X_5$ are A, F, G, L, R, C, D, E, H, I, K, M, N, P, Q, S, T, V, W or Y.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_3$ is R, and $X_1$ is E, $X_2$ is K, $X_4$ is L, $X_5$ is Y, or

$X_4$ is R, and $X_1$ is E, $X_2$ is K, $X_3$ is F, $X_5$ is Y, or

$X_3$ is R, $X_4$ is R, and $X_1$ is E, $X_2$ is K, $X_5$ is Y.

In another embodiment, the description concerns a method to obtain the active substance of a pharmaceutical composition, as defined above, wherein said isolated mutated feline lentiviral ENV protein having a decreased immunosuppressive activity, substantially no immunosuppressive activity or no immunosuppressive activity, or a fragment of said isolated mutated feline lentiviral ENV protein having a decreased immunosuppressive activity, substantially no immunosuppressive activity or no immunosuppressive activity said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_3$ is R, and $X_1$, $X_2$, $X_4$, $X_5$ are A, F, G, L, R, C, D, E, H, I, K, M, N, P, Q, S, T, V, W or Y.

In another embodiment, the description concerns a method to obtain the active substance of a pharmaceutical composition, as defined above

wherein said isolated mutated feline lentiviral ENV protein having a decreased immunosuppressive activity, substantially no immunosuppressive activity or no immunosuppressive activity

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_3$ is R, and $X_1$ is E, $X_2$ is K, $X_4$ is L, $X_5$ is Y.

In another embodiment, the description concerns a method to obtain the active substance of a pharmaceutical composition, as defined above, consisting in modifying the immunosuppressive property of:

a wild-type feline lentiviral ENV protein,

or a fragment of said wild-type feline lentiviral ENV protein, said fragment comprising at least 40 amino acids, in particular at least 60 amino acids,

said ENV protein or fragment thereof presenting a transmembrane subunit (TM) comprising an immunosuppressive domain (ISU) containing the following amino acid sequence:

A-[I/M/T/L]-$X_A$-$X_B$-$X_C$-$X_D$-$X_E$-T-A (SEQ ID NO: 344),

wherein

$X_A$ is E, and

$X_B$ is K, and

$X_C$ is F or P, and

$X_D$ is L, V or I, and

$X_E$ is Y,

said method comprising a step of introduction of at least one mutation of $X_A$ and/or $X_B$ and/or $X_C$ and/or $X_D$ and/or $X_E$, to obtain:

an isolated mutated feline lentiviral ENV protein having a decreased immunosuppressive activity, substantially no immunosuppressive activity or no immunosuppressive activity,

said mutated feline lentiviral ENV protein having at least 70% identity, preferably at least 80% identity, to one sequence SEQ ID NO: 5,

or a fragment of said isolated mutated feline lentiviral ENV protein having a decreased immunosuppressive activity, substantially no immunosuppressive activity or no immunosuppressive activity, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_1$ is any amino acid different from E, and $X_2$, $X_3$, $X_4$ and $X_5$ are any amino acid, or

$X_2$ is any amino acid different from K, and $X_1$, $X_3$, $X_4$ and $X_5$ are any amino acid, or

$X_3$ is any amino acid different from F, and $X_1$, $X_2$, $X_4$ and $X_5$ are any amino acid, or

$X_4$ is any amino acid different from L, and $X_1$, $X_2$, $X_3$ and $X_5$ are any amino acid, or

$X_5$ is any amino acid different from Y, and $X_1$, $X_2$, $X_3$ and $X_4$ are any amino acid,

in particular, wherein

$X_1$ is R, G, L, A or F, and $X_2$, $X_3$, $X_4$ and $X_5$ are any amino acid, or

$X_2$ is R, G, L, A or F, and $X_1$, $X_3$, $X_4$ and $X_5$ are any amino acid, or

$X_3$ is R, G, L or A, and $X_1$, $X_2$, $X_4$ and $X_5$ are any amino acid, or

$X_4$ is R, G, A or F, and $X_1$, $X_2$, $X_3$ and $X_5$ are any amino acid, or

$X_5$ is R, G, L, A or F and $X_1$, $X_2$, $X_3$ and $X_4$ are any amino acid.

In another aspect, the invention relates to a method to obtain the active substance of a pharmaceutical composition, as defined above, consisting in modifying the immunosuppressive property of:

a wild-type feline lentiviral ENV protein,

or a fragment of said wild-type feline lentiviral ENV protein, said fragment comprising at least 40 amino acids, in particular at least 60 amino acids,

said ENV protein or fragment thereof presenting a transmembrane subunit (TM) comprising an immunosuppressive domain (ISU) containing the following amino acid sequence:

A-[I/M/T/L]-$X_A$-$X_B$-$X_C$-$X_D$-$X_E$-T-A (SEQ ID NO: 344),

wherein

$X_A$ is E, and

$X_B$ is K, and

$X_C$ is F or P, and

$X_D$ is L, V or I, and

$X_E$ is Y,

said method comprising a step of introduction of at least one mutation of $X_A$ or $X_B$ or $X_C$ or $X_D$ or $X_E$, to obtain:

an isolated mutated feline lentiviral ENV protein that has lost at least 50%, at least 75% or 100% of its immunosuppressive activity in comparison to the immunosuppressive activity of the wild type Env protein,

said mutated feline lentiviral ENV protein having at least 70% identity, preferably at least 80% identity, to one sequence SEQ ID NO: 5,

or a fragment of said isolated mutated feline lentiviral ENV protein that has lost at least 50%, at least 75% or 100% of its immunosuppressive activity in comparison to the immunosuppressive activity of the wild type Env protein, said fragment comprising at least 40 amino acids, in particular at least 60 amino acids,

said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_1$ is R, $X_2$ is K, $X_3$ is F or P, $X_4$ is L or V or I and $X_5$ is Y, or

$X_2$ is R, $X_1$ is E, $X_3$ is F or P, $X_4$ is L or V or I and $X_5$ is Y, or

$X_3$ is R, $X_1$ is E, $X_2$ is K, $X_4$ is L or V or I and $X_5$ is Y, or

$X_4$ is R, $X_1$ is E, $X_2$ is K, $X_3$ is F or P and $X_5$ is Y, or

$X_5$ is R, $X_1$ is E, $X_2$ is K, $X_3$ is F or P and $X_4$ is L or V or I.

In other aspect, the invention also relates to pharmaceutical compositions comprising as active substance a mutated lentiviral protein isolated from other animal lentiviruses, infecting bovine, equine, ovine or caprine animal species.

In another aspect, the invention also relates to a pharmaceutical composition comprising as active substance an isolated mutated bovine lentiviral ENV protein having a decreased immunosuppressive activity, substantially no immunosuppressive activity or no immunosuppressive activity, or a fragment thereof,

said mutated bovine lentiviral ENV protein resulting from mutation of the transmembrane (TM) subunit of a wild type bovine lentiviral ENV protein,

said mutated ENV protein having at least 70% identity, preferably at least 80% identity, to the sequence SEQ ID NO: 420,

said mutated bovine lentiviral ENV protein or fragment thereof comprising a mutated immunosuppressive domain (ISU) containing the following amino acid sequence:

Y-L-$Z^A_1$-$Z^A_2$-$Z^A_3$-$Z^A_4$-$Z^A_5$-[I/V]-[R/H] (SEQ ID NO: 421),

wherein,

$Z^A_1$ is any amino acid different from E or deleted, and/or

$Z^A_2$ is any amino acid different from Y or F or deleted, and/or

$Z^A_3$ is any amino acid different from V or L or deleted, and/or

$Z^A_4$ is any amino acid different from E or *A or* deleted, and/or

$Z^A_5$ is any amino acid different from E or deleted,

in association with a pharmaceutically acceptable carrier.

In another aspect, the invention also relates to a pharmaceutical composition comprising as active substance an isolated mutated bovine lentiviral ENV protein having a decreased immunosuppressive activity, substantially no immunosuppressive activity or no immunosuppressive activity, or a fragment thereof,

said mutated bovine lentiviral ENV protein resulting from mutation of the transmembrane (TM) subunit of a wild type bovine lentiviral ENV protein,

said mutated ENV protein having at least 70% identity, preferably at least 80% identity, to the sequence SEQ ID NO: SEQ ID NO: 420,

said mutated bovine lentiviral ENV protein or fragment thereof comprising a mutated immunosuppressive domain (ISU) containing the following amino acid sequence:

V-[S/T]-Y-L-$Z^A_1$-$Z^A_2$-$Z^A_3$-$Z^A_4$-$Z^A_5$-[I/V]-[R/H]-[E/Q]-[K/L/V]-Q (SEQ ID NO: 422), wherein,

$Z^A_1$ is any amino acid different from E or deleted, and/or

$Z^A_2$ is any amino acid different from Y or F or deleted, and/or

$Z^A_3$ is any amino acid different from V or L or deleted, and/or

$Z^A_4$ is any amino acid different from E or A *or* deleted, and/or

$Z^A_5$ is any amino acid different from E or deleted,

in association with a pharmaceutically acceptable carrier.

In another aspect, the invention also relates to a pharmaceutical composition comprising as active substance an isolated mutated bovine lentiviral ENV protein having a decreased immunosuppressive activity, substantially no immunosuppressive activity or no immunosuppressive activity, or a fragment thereof,

said mutated bovine lentiviral ENV protein resulting from mutation of the transmembrane (TM) subunit of a wild type bovine lentiviral ENV protein,

said mutated ENV protein having at least 70% identity, preferably at least 80% identity, to the sequence SEQ ID NO: 423,

said mutated bovine lentiviral ENV protein or fragment thereof comprising a mutated immunosuppressive domain (ISU) containing the following amino acid sequence:

Y-L-$Z^A_1$-$Z^A_2$-$Z^A_3$-$Z^A_4$-$Z^A_5$-V-H (SEQ ID NO: 424),

wherein,

$Z^A_1$ is any amino acid different from E or deleted, and/or

$Z^A_2$ is any amino acid different from F or deleted, and/or

$Z^A_3$ is any amino acid different from L or deleted, and/or

$Z^A_4$ is any amino acid different from A *or* deleted, and/or

$Z^A_5$ is any amino acid different from E or deleted,

in association with a pharmaceutically acceptable carrier.

In another aspect, the invention also relates to a pharmaceutical composition comprising as active substance an isolated mutated bovine lentiviral ENV protein having a decreased immunosuppressive activity, substantially no immunosuppressive activity or no immunosuppressive activity, or a fragment thereof,

said mutated bovine lentiviral ENV protein resulting from mutation of the transmembrane (TM) subunit of a wild type bovine lentiviral ENV protein,

said mutated ENV protein having at least 70% identity, preferably at least 80% identity, to the sequence SEQ ID NO: 423,

said mutated bovine lentiviral ENV protein or fragment thereof comprising a mutated immunosuppressive domain (ISU) containing the following amino acid sequence:

V-T-Y-L-$Z^A_1$-$Z^A_2$-$Z^A_3$-$Z^A_4$-$Z^A_5$-V-H-E-[L/V]-Q (SEQ ID NO: 425),

wherein,

$Z^A_1$ is any amino acid different from E or deleted, and/or

$Z^A_2$ is any amino acid different from F or deleted, and/or

$Z^A_3$ is any amino acid different from L or deleted, and/or

$Z^A_4$ is any amino acid different from A or deleted, and/or

$Z^A_5$ is any amino acid different from E or deleted,

in association with a pharmaceutically acceptable carrier.

In a particular embodiment, the invention relates to a pharmaceutical composition as defined above, wherein said mutated bovine lentiviral Env protein, or said fragment thereof, further comprises additional mutations of at least one amino acid chosen among the 5th, 6th , 7th, 8th and 9th amino acids, in particular the 7th amino acid, located upstream the amino acid $Z^A_1$ of SEQ ID NO: 421, SEQ ID NO: 422, SEQ ID NO: 424 or SEQ ID NO: 425.

In another aspect, the invention relates to a pharmaceutical composition comprising as active substance a nucleic acid molecule coding for a mutated bovine lentiviral ENV protein, or a fragment of said mutated bovine lentiviral ENV protein, as defined above.

In another aspect, the invention also relates to a pharmaceutical composition comprising as active substance an isolated mutated equine lentiviral ENV protein having a decreased immunosuppressive activity, substantially no immunosuppressive activity or no immunosuppressive activity, or a fragment thereof,

said mutated equine lentiviral ENV protein resulting from mutation of the transmembrane (TM) subunit of a wild type equine lentiviral ENV protein,

said mutated ENV protein having at least 70% identity, preferably at least 80% identity, to the sequence SEQ ID NO: 426,

said mutated equine lentiviral ENV protein or fragment thereof comprising a mutated immunosuppressive domain (ISU) containing the following amino acid sequence:

L-L-$Z^B_1$-$Z^B_2$-$Z^B_3$-$Z^B_4$-$Z^B_5$-[V/I]-E (SEQ ID NO: 427),

wherein,

$Z^B_1$ is any amino acid different from K or deleted, and/or

$Z^B_2$ is any amino acid different from E or deleted, and/or

$Z^B_3$ is any amino acid different from K or Q or deleted, and/or

$Z^B_4$ is any amino acid different from Q or deleted, and/or

$Z^B_5$ is any amino acid different from Q or L or K or deleted, and/or

in association with a pharmaceutically acceptable carrier.

In another aspect, the invention also relates to a pharmaceutical composition comprising as active substance an isolated mutated equine lentiviral ENV protein having a decreased immunosuppressive activity, substantially no immunosuppressive activity or no immunosuppressive activity, or a fragment thereof,

said mutated equine lentiviral ENV protein resulting from mutation of the transmembrane (TM) subunit of a wild type equine lentiviral ENV protein,

said mutated ENV protein having at least 70% identity, preferably at least 80% identity, to the sequence SEQ ID NO: 426,

said mutated equine lentiviral ENV protein or fragment thereof comprising a mutated immunosuppressive domain (ISU) containing the following amino acid sequence:

V-[Q/R]-L-L-$Z^B_1$-$Z^B_2$-$Z^B_3$-$Z^B_4$-$Z^B_5$-[V/I]-E-E-T-F (SEQ ID NO: 428), wherein,

$Z^B_1$ is any amino acid different from K or deleted, and/or

$Z^B_2$ is any amino acid different from E or deleted, and/or

$Z^B_3$ is any amino acid different from K or Q or deleted, and/or

$Z^B_4$ is any amino acid different from Q or deleted, and/or

$Z^B_5$ is any amino acid different from Q or L or K or deleted, and/or

in association with a pharmaceutically acceptable carrier.

In a particular embodiment, the invention relates to a pharmaceutical composition as defined above, wherein said mutated equine lentiviral ENV protein, or said fragment thereof, further comprises additional mutations of at least one amino acid chosen among the 5th, 6th , 7th, 8th and 9th amino acids, in particular the 7th amino acid, located upstream the amino acid $Z^A_1$ of SEQ ID NO: 427 or SEQ ID NO: 428.

In another aspect, the invention relates to a pharmaceutical composition comprising as active substance a nucleic acid molecule coding for a mutated equine lentiviral ENV protein, or a fragment of said mutated equine lentiviral ENV protein, as defined above.

In another aspect, the invention also relates to a pharmaceutical composition comprising as active substance an isolated mutated caprine lentiviral ENV protein having a decreased immunosuppressive activity, substantially no immunosuppressive activity or no immunosuppressive activity, or a fragment thereof,

said mutated caprine lentiviral ENV protein resulting from mutation of the transmembrane (TM) subunit of a wild type caprine lentiviral ENV protein,

said mutated ENV protein having at least 70% identity, preferably at least 80% identity, to the sequence SEQ ID NO: 429,

said mutated caprine lentiviral ENV protein or fragment thereof comprising a mutated immunosuppressive domain (ISU) containing the following amino acid sequence:

R-[V/M]-$Z^A_1$-$Z^A_2$-$Z^A_3$-$Z^A_4$-$Z^A_5$-R-[M/I] (SEQ ID NO: 432),

wherein,

$Z^A_1$ is any amino acid different from E or deleted, and/or

$Z^A_2$ is any amino acid different from A or T or V or deleted, and/or

$Z^A_3$ is any amino acid different from I or L or V or M or deleted, and/or

$Z^A_4$ is any amino acid different from T or V or M or I or deleted, and/or

$Z^A_5$ is any amino acid different from D or deleted,

in association with a pharmaceutically acceptable carrier.

In another aspect, the invention also relates to a pharmaceutical composition comprising as active substance an isolated mutated caprine lentiviral ENV protein having a decreased immunosuppressive activity, substantially no immunosuppressive activity or no immunosuppressive activity, or a fragment thereof,

said mutated caprine lentiviral ENV protein resulting from mutation of the transmembrane (TM) subunit of a wild type caprine lentiviral ENV protein,

said mutated ENV protein having at least 70% identity, preferably at least 80% identity, to the sequence SEQ ID NO: 429,

said mutated caprine lentiviral ENV protein or fragment thereof comprising a mutated immunosuppressive domain (ISU) containing the following amino acid sequence:

V-A-R-[V/M]-$Z^A_1$-$Z^A_2$-$Z^A_3$-$Z^A_4$-$Z^A_5$-R-[M/I]-M-[L/I/V]-Y (SEQ ID NO: 433), wherein,

$Z^A_1$ is any amino acid different from E or deleted, and/or

$Z^A_2$ is any amino acid different from A or T or V or deleted, and/or

$Z^A_3$ is any amino acid different from I or L or V or M or deleted, and/or

$Z^A_4$ is any amino acid different from T or V or M or I or deleted, and/or

$Z^A_5$ is any amino acid different from D or deleted,

in association with a pharmaceutically acceptable carrier.

In a particular embodiment, the invention relates to a pharmaceutical composition as defined above, wherein said mutated caprine lentiviral ENV protein, or said fragment thereof, further comprises additional mutations of at least one amino acid chosen among the 5th, 6th , 7th, 8th and 9th amino acids, in particular the 7th amino acid, located upstream the amino acid $Z^A_1$ of SEQ ID NO: 432 or SEQ ID NO: 433.

In another aspect, the invention relates to a pharmaceutical composition comprising as active substance a nucleic acid molecule coding for a mutated caprine lentiviral ENV protein, or a fragment of said mutated caprine lentiviral ENV protein, as defined above.

In another aspect, the invention also relates to a pharmaceutical composition comprising as active substance an isolated mutated ovine lentiviral ENV protein having a decreased immunosuppressive activity, substantially no immunosuppressive activity or no immunosuppressive activity, or a fragment thereof,

said mutated ovine lentiviral ENV protein resulting from mutation of the transmembrane (TM) subunit of a wild type ovine lentiviral ENV protein,

said mutated ENV protein having at least 70% identity, preferably at least 80% identity, to the sequence SEQ ID NO: 430,

said mutated ovine lentiviral ENV protein or fragment thereof comprising a mutated immunosuppressive domain (ISU) containing the following amino acid sequence:

R-[V/M]-$Z^A_1$-$Z^A_2$-$Z^A_3$-$Z^A_4$-$Z^A_5$-R-[M/I] (SEQ ID NO: 432),

wherein,

$z^A_1$ is any amino acid different from E or deleted, and/or
$Z^A_2$ is any amino acid different from A or T or V or deleted, and/or
$Z^A_3$ is any amino acid different from I or L or V or M or deleted, and/or
$Z^A_4$ is any amino acid different from T or V or M or I or deleted, and/or
$Z^A_5$ is any amino acid different from D or deleted,
in association with a pharmaceutically acceptable carrier.

In another aspect, the invention also relates to a pharmaceutical composition comprising as active substance an isolated mutated ovine lentiviral ENV protein having a decreased immunosuppressive activity, substantially no immunosuppressive activity or no immunosuppressive activity, or a fragment thereof,
said mutated ovine lentiviral ENV protein resulting from mutation of the transmembrane (TM) subunit of a wild type ovine lentiviral ENV protein,
said mutated ENV protein having at least 70% identity, preferably at least 80% identity, to the sequence SEQ ID NO: 430
said mutated ovine lentiviral ENV protein or fragment thereof comprising a mutated immunosuppressive domain (ISU) containing the following amino acid sequence:

V-A-R-[V/M]-$Z^A_1$-$Z^A_2$-$Z^A_3$-$Z^A_4$-$Z^A_5$-R-[M/I]-M-[L/I/V]-Y (SEQ ID NO: 433),
wherein,
$Z^A_1$ is any amino acid different from E or deleted, and/or
$Z^A_2$ is any amino acid different from A or T or V or deleted, and/or
$Z^A_3$ is any amino acid different from I or L or V or M or deleted, and/or
$Z^A_4$ is any amino acid different from T or V or M or I or deleted, and/or
$Z^A_5$ is any amino acid different from D or deleted,
in association with a pharmaceutically acceptable carrier.

In another aspect, the invention also relates to a pharmaceutical composition comprising as active substance an isolated mutated ovine lentiviral ENV protein having a decreased immunosuppressive activity, substantially no immunosuppressive activity or no immunosuppressive activity, or a fragment thereof,
said mutated ovine lentiviral ENV protein resulting from mutation of the transmembrane (TM) subunit of a wild type ovine lentiviral ENV protein,
said mutated ENV protein having at least 70% identity, preferably at least 80% identity, to the sequence SEQ ID NO: 431
said mutated ovine lentiviral ENV protein or fragment thereof comprising a mutated immunosuppressive domain (ISU) containing the following amino acid sequence:

R-[V/M]-$Z^A_1$-$Z^A_2$-$Z^A_3$-$Z^A_4$-$Z^A_5$-R-[M/I] (SEQ ID NO: 432),
wherein,
$Z^A_1$ is any amino acid different from E or deleted, and/or
$Z^A_2$ is any amino acid different from A or T or V or deleted, and/or
$Z^A_3$ is any amino acid different from I or L or V or M or deleted, and/or
$Z^A_4$ is any amino acid different from T or V or M or I or deleted, and/or
$Z^A_5$ is any amino acid different from D or deleted,
in association with a pharmaceutically acceptable carrier.

In another aspect, the invention also relates to a pharmaceutical composition comprising as active substance an isolated mutated ovine lentiviral ENV protein having a decreased immunosuppressive activity, substantially no immunosuppressive activity or no immunosuppressive activity, or a fragment thereof,
said mutated ovine lentiviral ENV protein resulting from mutation of the transmembrane (TM) subunit of a wild type ovine lentiviral ENV protein,
said mutated ENV protein having at least 70% identity, preferably at least 80% identity, to the sequence SEQ ID NO: 431
said mutated ovine lentiviral ENV protein or fragment thereof comprising a mutated immunosuppressive domain (ISU) containing the following amino acid sequence:

V-A-R-[V/M]-$Z^A_1$-$Z^A_2$-$Z^A_3$-$Z^A_4$-$Z^A_5$-R-[M/I]-M-[L/I/V]-Y (SEQ ID NO: 433), wherein,
$Z^A_1$ is any amino acid different from E or deleted, and/or
$Z^A_2$ is any amino acid different from A or T or V or deleted, and/or
$Z^A_3$ is any amino acid different from I or L or V or M or deleted, and/or
$Z^A_4$ is any amino acid different from T or V or M or I or deleted, and/or
$Z^A_5$ is any amino acid different from D or deleted,
in association with a pharmaceutically acceptable carrier.

In a particular embodiment, the invention relates to a pharmaceutical composition as defined above, wherein said mutated ovine lentiviral ENV protein, or said fragment thereof, further comprises additional mutations of at least one amino acid chosen among the 5th, 6th , 7th, 8th and 9th amino acids, in particular the 7th amino acid, located upstream the amino acid $Z^A_1$ of SEQ ID NO: 432 or SEQ ID NO: 433.

In another aspect, the invention relates to a pharmaceutical composition comprising as active substance a nucleic acid molecule coding for a mutated ovine lentiviral ENV protein, or a fragment of said mutated ovine lentiviral ENV protein, as defined above.

## LEGEND TO THE FIGURES

### Figure 1:

(A) FIV Envelope phylogeny, with the A-D subtypes indicated and the reference Petaluma strain boxed (from Pu et al, Journal of Feline Medicine and Surgery, 2005, 7:65-70).
(B) Structure of the FIV Envelope protein, delineation of the characteristic functional domains and alignment of the 64-aa immunosuppressive-containing domains from selected FIV Env proteins. The SU and TM subunits of the FIV Env are indicated, together with the fusion peptide, the transmembrane anchoring domain of the TM subunit, and the immunosuppressive domain (ISD).

The aligned sequences of the 64-aa immunosuppressive-containing domains correspond to fragments of the Envelope protein from 33 distinct strains (*i.e.* 33 different accession numbers), only 20 of these 64-aa fragments are different (*i.e.* 20 SEQ ID numbers have been given).

### Figure 2:

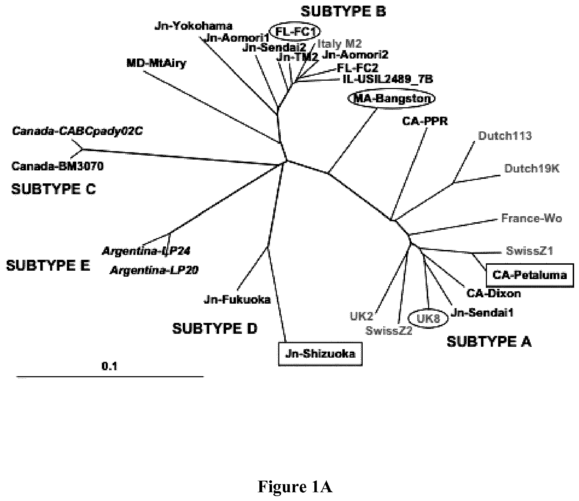

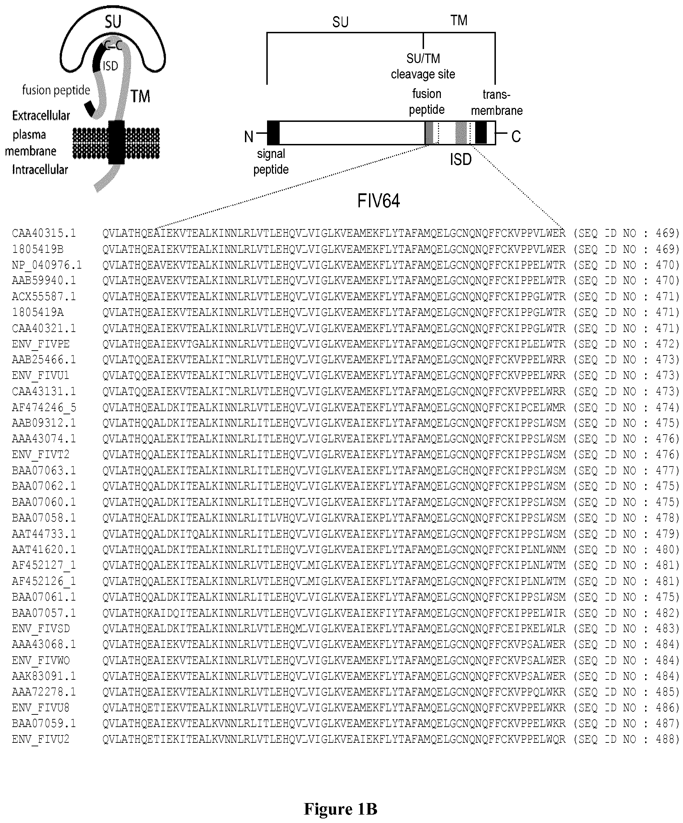

Immunosuppressive activity of the full-length FIV envelope protein (FIV Env) and of the 64 aa-long FIV envelope subdomain delineated in Figure 1 (FIV64 Env). Immunosuppression was tested using the *in vivo* MCA205 tumor rejection assay (see scheme on top and Materials and Methods): MCA205 tumor cells are transduced with an expression vector (containing a selectable hygromycin gene) for the indicated Env protein or fragment, stably transduced cells are then selected for expression of the corresponding Env, and are finally engrafted into Balb/c mice (allogenic graft, normally resulting in tumor rejection); tumor growth/rejection is then monitored and provides an immunosuppressive index (calculated as indicated in Materials and Methods). Y-axis corresponds to the value of the immunosuppression index. Immunosuppression indexes are means of at least 3 independent experiments, with standard deviation. The Murine Leukemia Virus (MLV) envelope ectodomains, wild-type and mutant (Schlecht-Louf et al., Proc Natl Acad Sci U S A. 2010, 107(8):3782-7) are used as an internal control.

### Figure 3:

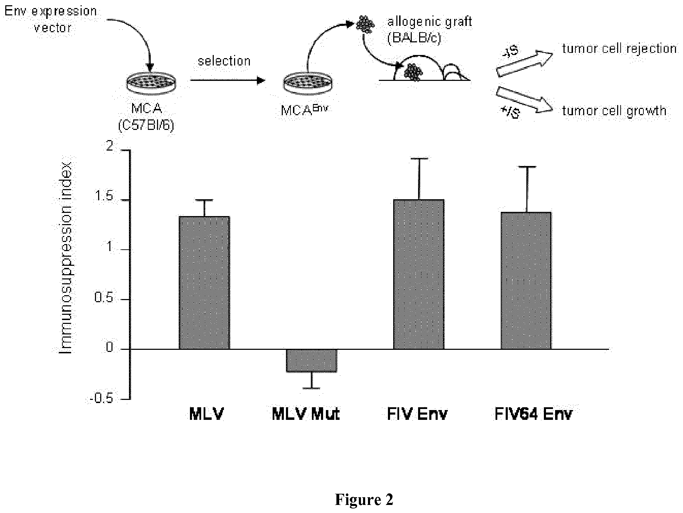

Functional identification of the aminoacids in the FIV envelope 64 aa domain directly involved in immunosuppressive activity, and search for aminoacid substitutions inhibiting this activity. Immunosuppressive activity was tested as in Figure 2, using the *in vivo* MCA205 tumor rejection assay (see Materials and Methods). The mutated aminoacids are indicated with their position (see sequence of FIV64 on top) and the nature of the substitution. Wwt and Wmut are controls (from the human endogenous retrovirus HERV-W Envelope, Mangeney et al., Proc Natl Acad Sci USA. 2007, 104(51):20534-9). Immunosuppression indexes correspond to 3 independent experiments, with standard deviation.

### Figure 4:

Antibody response of mice inoculated with cells expressing wild-type and mutant FIV64 Env domains (see scheme on top): MCA205 tumor cells are stably transduced as in Figure 2 with expression vectors for the indicated FIV64 Env wt and mutants, and cells engrafted into C57BL/6 mice (syngenic graft, resulting in constant tumor growth without rejection); specific anti-FIV64 antibodies are quantitated from the collected blood samples as indicated in Materials and Methods. Results are representative of 2 independent experiments.

### Figure 5:

Antibody response of mice injected with MBP-FIV64, wt and mutants, recombinant proteins (see scheme on top): FIV64 Env, wt and mutants, fused with MBP are i.v. injected three times with a 1-week interval into Balb/C mice (10 $\mu$g per injection); mice are then blood-sampled and anti-MBP IgG levels are determined by ELISA using plates coated with MBP-LacZ$\alpha$ (MBP-LacZ$\alpha$ is a control MBP fusion with the 83aa LacZ$\alpha$ fragment (a-subunit of E. coli $\beta$-galactosidase, see Materials and Methods). Results are representative of 2 independent experiments.

### Figure 6:

Expression of the FIV Env proteins, wt and mutants, at the surface of cells transfected with the indicated expression vectors (see scheme on top).

Expression profile of the wild-type and mutant FIV Env proteins was assayed by FACS analysis using full-length FIVenv-expressing vectors and an anti-SU FIVenv monoclonal antibody (Antibodies-online, BmbH, Germany). The data correspond to the Mean Fluorescence Intensity, from three independent experiments, with standard deviation.

**Figure 7:**
Functional characterization of the FIV Env proteins, wild-type (wt) and mutants. Fusogenic activity of the FIVenv, wild-type and mutants, are measured by a cell-cell fusion assay and quantified by a fusion index (see scheme on top and Materials and Methods). The data correspond to three independent experiments, with standard deviation.

## EXAMPLES

### The FIV envelope and a delineated subdomain of the TM subunit are immunosuppressive *in vivo*

Identification of an immunosuppressive activity of the FIV envelope and of the domain within the FIV envelope responsible for this activity was achieved using an *in vivo* tumor rejection assay that we had previously used to demonstrate the immunosuppressive activity of the Env protein of oncoretroviruses (e.g. murine MoMLV and simian MPMV, see Mangeney and Heidmann, Proc Natl Acad Sci USA, 1998, 95:14920-14925*;* Mangeney et al., Proc Natl Acad Sci USA, 2007, 104(51):20534-9).
The rationale of the assay can be summarized as follows: while injection of MCA205 tumor cells (H-2$^b$) into allogeneic Balb/c mice (H-2$^d$) leads to the formation of no tumor or transient tumors that are rapidly rejected, injection of the same cells, but stably expressing an immunosuppressive retroviral Env protein, leads to the growth of larger tumors that persist for a longer time -in spite of the expression of the new exogenous antigen.
This difference is not associated with a difference in intrinsic cell growth rate since it is not observed in syngeneic C57BL/6 mice, and is immune system-dependent.
The extent of "immunosuppression" can be quantified by an index based on tumor size: $(A_{env}-A_{none})/A_{none}$, where $A_{env}$ and $A_{none}$ are the mean areas at the peak of growth of tumors from Balb/c mice injected with *env*-expressing or control cells, respectively. A positive index indicates that *env* expression facilitates tumor growth, as a consequence of its immunosuppressive activity; a null or negative index points to no effect or even an inhibitory effect, respectively. The latter can be explained by a stimulation of the immune response of the host against the new foreign antigen, represented by a non-immunosuppressive Env protein, expressed at the surface of tumor cells. Accordingly, as illustrated in Figure 2, it can be observed that the FIV env is immunosuppressive, and that this activity is carried by a 64-mer peptide of the transmembrane (TM) subunit of the envelope protein. The immunosuppression indexes are close to those found for non-lentiviral env proteins (such as that of the murine MLV retrovirus shown in the figure as a control, together with a non-immunosuppressive mutant).

### Identification of the FIV env amino-acids critical for immunosuppression and its inhibition, via R- scanning

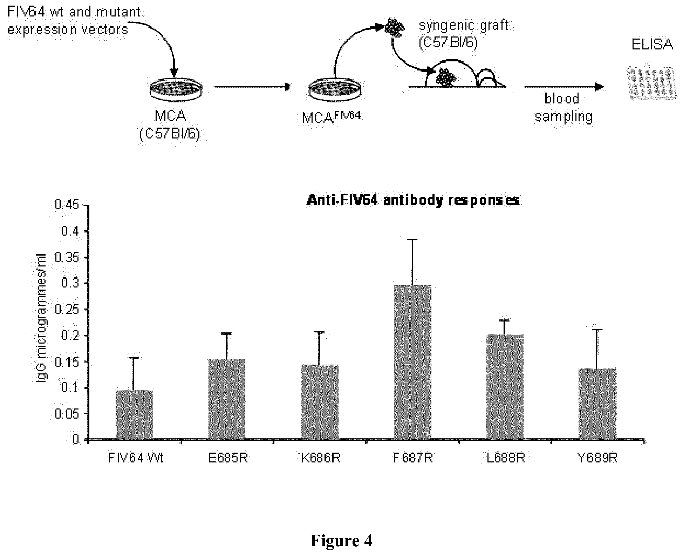

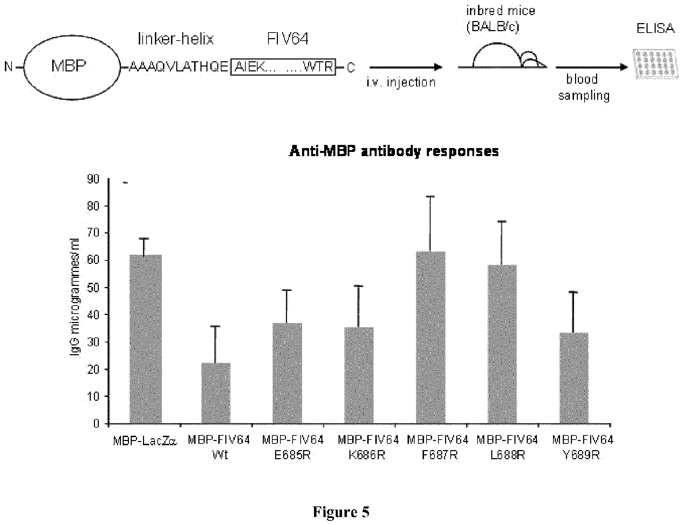

To characterize further the immunosuppressive domain (ISD) of the FIV env active *in vivo,* we analyzed the effect of a series of amino-acid substitutions within the FIV *env* 64 aa domain shown to carry the IS activity (see above). The 64-aa FIV env domain is embedded into the so-called ectodomain, which corresponds to the extracellular domain of the TM subunit, and consists in the $\alpha$-helical domain involved in FIV TM trimerization, and the N-term part of the loop containing the 2 well-conserved, 6/7 aa-distant, cysteine residues found in most retroviral envelopes. Refine delineation of the amino acis responsible for IS activity was thus performed by arginine-scanning within this domain. As illustrated in Figure 3, X-to-Arg substitutions resulted in a significant change in the IS activity of the FIVenv 64 aa domain, with maximum effect observed for the F687 position and to a lesser -but significant- extent for the adjacent E685, K686, L688 and Y689 positions. A series of other substitutions are performed at the F687 position, substitutions for the small A and G, and the hydrophobic L and F residues.
We also analyzed the effect of this series of amino-acid substitutions within the FIVenv 64 aa domain by measuring the production of anti-FIV env IgG antibodies in mice inoculated under syngenic conditions (C57Bl/6 mice) with the cells expressing the wild-type and mutant FIVenv 64 aa domains. As illustrated in Figure 4, X-to-Arg subsitutions resulted in an increase in the antibody response, consistent with an increased immunogenicity of FIVenv 64, with again a more important effect observed for the F687 position.
Additionally, a series of experiments were performed with recombinant proteins containing the FIVenv 64 domain, to assay the relative immunogenicity of the wt and mutants proteins. The domains were fused with the carrier MBP (Maltose Binding Protein), and the proteins were injected i.v. into Balb/C mice (see Materials and Methods). The immunosuppressive effects of the FIVenv 64 wt and mutants were assayed by measuring the inhibition of the anti-MBP antibody response raised in the injected mice. As illustrated in Figure 5, a significant decrease -relative to the control (MBP-LacZ$\alpha$)- is observed with FIVenv 64 wt, consistent with its immunosuppressive activity acting in cis, whereas almost no reduction is observed for the F687R and L688R mutants, indicative of the loss of its IS activity. A similar effect is observed for the other mutants, although at a lesser extent.

## Impact of the identified mutations on env protein folding and proper expression as a functional transmembrane protein

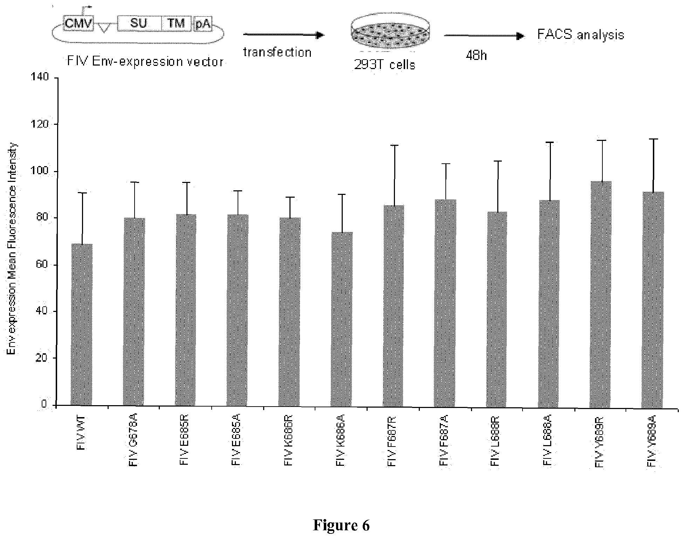

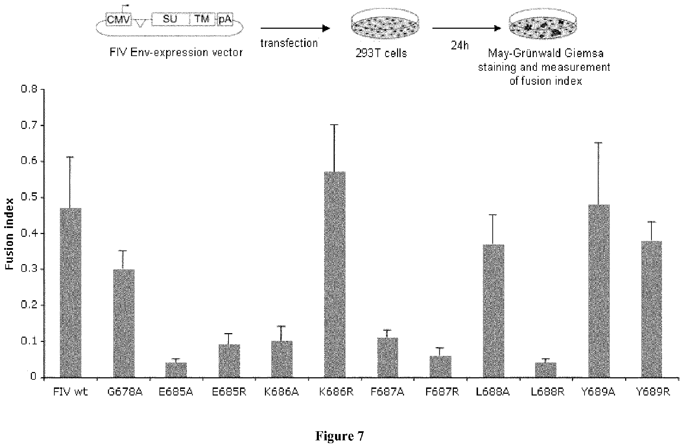

We then tested whether the above-mentioned substitutions alter the overall capacity of the FIV envelope to be expressed by an eucaryotic cell and to be exported at the cell membrane, by introducing the mutants into an expression vector for the full-length FIV envelope. A FACS analysis of cells transfected with the wild-type and the mutant FIV envelope genes inserted within a CMV-driven expression vector (see Materials and Methods) and using an anti-SU specific monoclonal antibody, demonstrated quantitative expression of the mutant envelopes at the cell surface (Figure 6). In particular, this analysis indicated that the F687R and L688R mutations did not significantly altered the FIV Env structure and/or SU-TM interaction. Finally, the fusogenic activity of the FIV full-length envelopes was tested by a cell-cell fusion assay, in which cells prone to FIVenv-mediated fusion were tranfected as above with the corresponding expression vectors, and cell-cell fusion monitored 24-48h post transfection by measuring the amount of multi-nucleated syncytia formed, with the process being quantified by a fusion index (see Materials and Methods). As illustrated in Figure 7 and despite the conservation of the amount of env protein expressed at the cell surface (cf Figure 6), variations in the fusion index exist among the various env mutants tested, although all of them remain fusion-positive,

Accordingly, the present investigation has clearly identified definite positions and definite substitutions within the FIV env resulting in the loss of its IS activity.

Being compatible with the conservation of the overall structure of the FIV Env protein, these substitutions should be introduced in all pharmaceutical preparations which include the Env protein as a vaccine antigen.

## Materials and methods

**Mice and cell lines:** C57B1/6 and Balb/c mice, 6-10 weeks old, were obtained from Harlan (France). Mice were maintained in the animal facility of the Gustave Roussy Institute in accordance with institutional regulations. 293T (ATCC CRL11268), and MCA205 cells were cultured in DMEM supplemented with 10% fetal calf serum (Invitrogen), streptomycin (100 $\mu$g/ml) and penicillin (100 units/ml).

## Plasmids constructions:

- **phCMV-FIVenv:** The pET34TF10 full feline immunodeficiency virus (FIV) genome clone (strain petaluma) (Talbott et al., Proc Natl Acad Sci USA, 1989, 86(15):5743-4747) served as template to generate full-length FIVenv PCR fragment using primers 1-2. This PCR fragment was digested with XhoI and MluI restriction enzymes to be ligated with phCMV vector opened with the same enzymes to generate a phCMV-FIVenv expression vector.

- **phCMV-FIVenv mutants:** Mutated phCMV-FIVenv was obtained by successive PCR using appropriate primers. A first series of PCRs was performed with phCMV-FIVenv as template with primer 3 and the reverse primer designed with the mutation (i.e. primers 5, 7, 9, 11, 13, 15, 17, 19, 21, 23); and primer 2 and the forward primer bearing the mutation (*i.e.* primers 4, 6, 8, 10, 12, 13, 14, 16, 18, 20, 22) to introduce the mutations E685A, E685R, K686A, K686R, F687A, F687R, L688A, L688R, Y689A and Y689R, respectively. The two PCR fragments bearing the same mutation were purified and mixed to be used as template for a subsequent PCR with primers 3 and 2. These PCR fragments were digested by SpeI and MluI restriction enzymes and purified. In the meantime the FIVenv fragment obtained after digestion of the phCMV-FIVenv with the XhoI and SpeI restriction enzymes was purified. A three-fragment ligation with phCMV opened with XhoI and MluI, the FIVenv fragment digested with XhoI and SpeI, and the SpeI-MluI-restricted PCR fragment with the desired mutation, was performed to obtain the phCMV-FIVenv mutants. Mutations of the amino acids E685, K686 and Y689 into G, L or F, mutations of the amino acid F687 into G or L, and mutations of the amino acid L688 into G or F are similarly performed using appropriate primers pairs (*i.e.* primers 31-58).

- **pDFG-FIVenv and pDFG-FIVenv mutants:** FIVenv and FIVenv mutant inserts were obtained by restriction with AgeI and MluI of the phCMV-FIVenv and mutants. These inserts were ligated with pDFG-ectoSyncytin-1 (see above Mangeney et al, PNAS, 2007) opened with the same enzymes.

- **pDFG-FIV64 and pDFG-FIV112, wild-type and mutants:** fragments of the FIVenv, wild-type and mutants, were PCR-amplified from phCMV-FIVenv using primers 24-25 or 24-26 to obtain FIV64 and FIV112 fragments, respectively. These PCR fragments were digested with SfiI and MluI and inserted into pDFG-ectoSyncytin-1 (see above Mangeney et al, PNAS, 2007) opened with the same enzymes.

- **pSIN-FIV64:** This vector was based on the lentiviral vector pHR'SIN-cPPT-SEW described in Demaison et al. (Human Gene Therapy, 2002, 13:803-813). The pSIN-FIV64 vectors were obtained by insertion of the hygromycin resistance gene under the control of the phosphoglycerate kinase promoter (PGK) at the end of WPRE sequence of pHR'SIN-cPPT-SEW. Then the FIV-64 fragments, wt and mutants, were PCR-amplified from pDFG-FIV64 wt and mutants, using primers 27-28, digested with Bam HI and Not I, and inserted downstream the pSec sequence under control of the SFFV promoter.

- **pMal-MBP-FIV:** Bacterial expression vectors for fusion proteins with E. coli maltose-binding protein (MBP) were constructed by ligation of a modified pMalc2x (described in Center et al., Protein Sci, 1998, 7(7):1612-1619) opened with PstI and HindIII, with the FIV-env ectodomain, wt and mutants, obtained by PCR from phCMV Env wt and mutants, with primer pair 29-30. These pMal-MBP-FIV64 vectors encode a 64-residue long FIV-ectodomain fused to the C terminus of MBP through a trialanine linker. The empty pMal-c2x vector encodes the 85-residue long $\alpha$-subunit of E. coli $\beta$-galactosidase fused to the C terminus of MBP and was used as a control (pMal-LacZ$\alpha$).

All the constructions were sequenced before use.

Table 2: Primer list

| N° | Name | Primer sequence (5'-3') | SEQ ID NO: |
|---|---|---|---|
| 1 | FIV env Xho Age Kozak ATG (FOR) | atacatCTCGAGACCGGTccaactagaaccATGGCAGAAGGATTTGCAGCC | 348 |
| 2 | FIV env Stop Mlu (REV) | ATACATacgcgtTCATTCCTCCTCTTTTTCAGACATGCCAC | 349 |
| 3 | FIV-env-2000-FOR | TACTGCTATAGGGATGGTAACACAATACCACCAAG | 350 |
| 4 | FIV-env-E685A-FOR | GATTAAAAGTAGAAGCTATGG**C**AAAATTTTTGTATACAGC | 351 |
| 5 | FIV-env-E685A-REV | GCTGTATACAAAAATTTT**G**CCATAGCTTCTACTTTTAATC | 352 |
| 6 | FIV-env-E685R-FOR | GATTAAAAGTAGAAGCTATG**AG**AAAATTTTTGTATACAGC | 353 |
| 7 | FIV-env-E685R-REV | GCTGTATACAAAAATTTT**CT**CATAGCTTCTACTTTTAATC | 354 |
| 8 | FIV-env-K686A-FOR | GTAGAAGCTATGGAA**G**CATTTTTGTATACAGCTTTC | 355 |
| 9 | FIV-env-K686A-REV | GAAAGCTGTATACAAAAAT**GC**TTCCATAGCTTCTAC | 356 |
| 10 | FIV-env-K686R-FOR | GTAGAAGCTATGGAA**AG**ATTTTTGTATACAGCTTTC | 357 |
| 11 | FIV-env-K686R-REV | GAAAGCTGTATACAAAAAT**CT**TTCCATAGCTTCTAC | 358 |
| 12 | FIV-env-F687A-FOR | GAAGCTATGGAAAAG**CC**TTGTATACAGCTTTC | 359 |

(continued)

| N° | Name | Primer sequence (5'-3') | SEQ ID NO: |
|---|---|---|---|
| 13 | FIV-env-F687A-REV | GAAAGCTGTATACAA**GGC**TTTTTCCATAGCTTC | 360 |
| 14 | FIV-env-F687R-FOR | GAAGCTATGGAAAAA**AGA**TTGTATACAGCTTTC | 361 |
| 15 | FIV-env-F687R-REV | GAAAGCTGTATACAA**TCT**TTTTTCCATAGCTTC | 362 |
| 16 | FIV-env-L688A-FOR | GCTATGGAAAAATTT**GCC**TATACAGCTTTCGCTATG | 363 |
| 17 | FIV-env-L688A-REV | CATAGCGAAAGCTGTATA**GGC**AAATTTTTCCATAGC | 364 |
| 18 | FIV-env-L688R-FOR | GCTATGGAAAAATTT**AG**GTATACAGCTTTCGCTATG | 365 |
| 19 | FIV-env-L688R-REV | CATAGCGAAAGCTGTATAC**CT**AAATTTTTCCATAGC | 366 |
| 20 | FIV-env-Y689A-FOR | CTATGGAAAAATTTTTG**GCC**ACAGCTTTCGCTATGC | 367 |
| 21 | FIV-env-Y689A-REV | GCATAGCGAAAGCTGT**GGC**CAAAAATTTTTCCATAG | 368 |
| 22 | FIV-env-Y689R-FOR | CTATGGAAAAATTTTTG**CGG**ACAGCTTTCGCTATGC | 369 |
| 23 | FIV-env-Y689R-REV | GCATAGCGAAAGCTGT**CCG**CAAAAATTTTTCCATAG | 370 |
| 24 | FIV64-Sfi-FOR | GGTGACGCGGCCCAGCCGGCCgctatagaaaaggtgactggagcc | 371 |
| 25 | FIV64-Mlu-REV | ATACATACGCGTTTAccttgtccacaactcaagagg | 372 |
| 26 | FIVeq112-MLU-REV | ATACATACGCGTTTAccctgttttccttgtacattattttg | 373 |
| 27 | BamHI psec-FOR | ATA GGA TCC AGA ACC ATG GAG ACA GAC ACA CTC | 435 |
| 28 | NotI FIV-REV | TAT GC GGCC GC TTA CCT TGT CCA CAA CTC AAG | 436 |
| 29 | PstI FIV72-FOR | ATAGCTGCAGCCCAAGTTCTGGCAACCCAT | 437 |
| 30 | HindIII FIV72-REV | TATAAGCTTTTACCTTGTCCACAACTCAAG | 438 |
| 31 | FIV-env-G678A-FOR | CATCAAGTACTAGTAATAGCATTAAAAGTAGAAGCTATG | 439 |
| 32 | FIV-env-G678A-REV | CATAGCTTCTACTTTTAATGCTATTACTAGTACTTGATG | 440 |
| 33 | FIV-env-E685G-FOR | GATTAAAAGTAGAAGCTATGGGAAAATTTTTGTATACAGC | 441 |

(continued)

| N° | Name | Primer sequence (5'-3') | SEQ ID NO: |
|---|---|---|---|
| 34 | FIV-env-E685G-REV | GCTGTATACAAAAATTTTCCCATAGCTTCTAC TTTTAATC | 442 |
| 35 | FIV-env-E685L-FOR | GATTAAAAGTAGAAGCTATGTTAAAATTTTT GTATACAGC | 443 |
| 36 | FIV-env-E685L-REV | GCTGTATACAAAAATTTTAACATAGCTTCTA CTTTTAATC | 444 |
| 37 | FIV-env-K686G-FOR | GTAGAAGCTATGGAAGGATTTTTGTATACAG CTTTC | 445 |
| 38 | FIV-env-K686G-REV | GAAAGCTGTATACAAAAATCCTTCCATAGCT TCTAC | 446 |
| 39 | FIV-env-K686L-FOR | GTAGAAGCTATGGAATTATTTTTGTATACAG CTTTC | 447 |
| 40 | FIV-env-K686L-REV | GAAAGCTGTATACAAAAATAATTCCATAGCT TCTAC | 448 |
| 41 | FIV-env-F687G-FOR | GAAGCTATGGAAAAGGATTGTATACAGCTT TC | 449 |
| 42 | FIV-env-F687G-REV | GAAAGCTGTATACAATCCTTTTTCCATAGCTT C | 450 |
| 43 | FIV-env-F687L-FOR | GAAGCTATGGAAAAATTATTGTATACAGCTT TC | 451 |
| 44 | FIV-env-F687L-REV | GAAAGCTGTATACAATAATTTTTCCATAGCTT C | 452 |
| 45 | FIV-env-L688G-FOR | GCTATGGAAAAATTTGGATATACAGCTTTCG CTATG | 453 |
| 46 | FIV-env-L688G-REV | CATAGCGAAAGCTGTATATCCAAATTTTTCC ATAGC | 454 |
| 47 | FIV-env-Y689G-FOR | CTATGGAAAAATTTTTGGGAACAGCTTTCGC TATGC | 455 |
| 48 | FIV-env-Y689G-REV | GCATAGCGAAAGCTGTTCCCAAAAATTTTTC CATAG | 456 |
| 49 | FIV-env-Y689L-FOR | CTATGGAAAAATTTTTGTTAACAGCTTTCGCT ATGC | 457 |
| 50 | FIV-env-Y689L-REV | GCATAGCGAAAGCTGTTAACAAAAATTTTTC CATAG | 458 |
| 51 | FIV -env-E685F-FOR | GATTAAAAGTAGAAGCTATGTTTAAATTTTT GTATACAGC | 459 |
| 52 | FIV -env-E685F-REV | GCTGTATACAAAAATTTAAACATAGCTTCTA CTTTTAATC | 460 |

(continued)

| N° | Name | Primer sequence (5'-3') | SEQ ID NO: |
|---|---|---|---|
| 53 | FIV-env-K686F-FOR | GTAGAAGCTATGGAATTTTTTTGTATACAGCTTTC | 461 |
| 54 | FIV-env-K686F-REV | GAAAGCTGTATACAAAAAAATTCCATAGCTTCTAC | 462 |
| 55 | FIV -env-L688F-FOR | GCTATGGAAAAATTTTTTTATACAGCTTTCGCTATG | 463 |
| 56 | FIV -env-L688F-REV | CATAGCGAAAGCTGTATAAAAAAATTTTTCCATAGC | 464 |
| 57 | FIV-env-Y689F-FOR | CTATGGAAAAATTTTTGTTTACAGCTTTCGCTATGC | 465 |
| 58 | FIV-env-Y689F-REV | GCATAGCGAAAGCTGTAAACAAAAATTTTTCCATAG | 466 |

**Recombinant Proteins:** Recombinant proteins were produced using BL21 (DE3) Escherichia coli cells (Stratagene) and pMal-derived expression vectors (New England Biolabs, France). Recombinant WT and mutants TM subunit ectodomains were soluble and were purified on on cross-linked Amylose Resin (New England Biolabs, France) packed in column with PBS as a binding and washing buffer and 20 mM Tris-Cl, 5 mM maltose, pH 7.5, as an elution buffer. Proteins were then dialysed against phosphate-buffered saline pH 7,4 (PBS), and endotoxins were removed using Endotrap Blue Resin (Hyglos GmbH, Germany) according to manufacturer's protocol.

**Establishment of *env*-expressing tumor cells and MCA205 tumor-rejection assay:** $7.5 \times 10^5$ 293T cells were cotransfected with the env-expressing pDFG retroviral vector to be tested (1.75 $\mu$g) and expression vectors for the MLV proteins (0.55 $\mu$g for the amphotropic MLV *env* vector and 1.75 $\mu$g for the MLV *gag* and *pol* vector). 36 hours post-transfection, supernatants were harvested for infection of MCA205 tumor cells (2.5 ml of supernatant per $5 \times 10^5$ cells with 4 $\mu$g/ml polybrene). Cells were maintained in selective medium (400 units/ml hygromycin) for 3 weeks, and then washed with PBS, trypsinized and inoculated subcutaneously in the shaved area of each mouse right flank as in Mangeney et al (see above PNAS 1998, PNAS 2007). Tumor growth was monitored by palpation twice or thrice weekly and tumor area ($mm^2$) determined by measuring perpendicular tumor diameters. The extent of "immunosuppression" was quantified by an index based on tumor size: $(A_{env}-A_{none})/A_{none}$, where $A_{env}$ and $A_{none}$ are the mean areas at the peak of growth of tumors from Balb/c mice injected with *env*-expressing or control cells, respectively.

**Analysis of the antibody response of mice inoculated with MCA205-tranduced cells expressing FIV64 env wild-type and mutants:** the MCA205-transduced cells were also inoculated as above into syngenic mice (C57Bl/6) and sera were collected 1, 2, and 3 weeks after injection. The antibody response against FIV64 env was assayed by ELISA. Briefly, several dilutions of the sera were incubated 1h at RT on a plate pre-coated with 1 $\mu$g/ml of MBP-FIV64, and antibody binding was analysed by using a labeled anti-mouse IgG secondary antibody (GE Healthcare, UK).

**Analysis of FIVenv expression:** $3 \times 10^5$ 293T cells were transfected with 2$\mu$g of the expression vector for the FIV envelope (phCMV) either wild-type or mutated at the indicated positions using Fugene HD (Roche). Cells were washed 16h later and then harvested 2 days post-transfection using PBS-EDTA 5mM. The SU1-30 monoclonal antibody (Antibodies online, BmbH, Germany) was used (1/200 dilution) to stain the FIV envelope. As a secondary antibody, we used the goat anti mouse IgG Alexa 488 (1/400) (Invitrogen). Fluorescence was acquired by flow cytometry using a FACS Calibur (BD Biosciences), and data analysed by the CellQuest software (BD Biosciences).

**Cell-cell fusion assays:** For cell-cell fusion assays, $5 \times 10^4$ to $1 \times 10^5$ cells seeded in 24-well plates were transfected by using lipofectamine LTX (Life technologies) with 250 ng of env expression plasmid. Fusion activity of each envelope protein was visualized 24 to 48 h after transfection by May-Grünwald and Giemsa staining, according to the manufacturer's instructions (Sigma). The fusion index, which represents the percentage of fusion events in a cell population, is defined as [(N - S)/T] x 100), where N is the number of nuclei in the syncytia, S is the number of syncytia, and T is the total number of nuclei counted.

**Claims**

1. Pharmaceutical composition comprising as active substance an isolated mutated feline lentiviral ENV protein, or a fragment thereof, that has lost at least 50%, at least 75% or 100% of its immunosuppressive activity in comparison to the immunosuppressive activity of the wild type Env protein,

   said fragment of said isolated mutated feline lentiviral ENV protein comprising at least 40 amino acids, in particular at least 60 amino acids,

   said mutated feline lentiviral ENV protein resulting from mutation of the transmembrane (TM) subunit of a wild type feline lentiviral ENV protein,

   said mutated ENV protein having at least 70% identity, preferably at least 80% identity, to the sequence SEQ ID NO: 5,

   said mutated feline lentiviral ENV protein or fragment thereof comprising a mutated immunosuppressive domain (ISU) containing the following amino acid sequence:

   A-[I/M/T/L]-$X_1$-$X_2$-$X_3$-$X_4$-$X_5$-T-A (SEQ ID NO: 1),

   wherein,

   $X_1$ is R, $X_2$ is K, $X_3$ is F or P, $X_4$ is L or V or I and $X_5$ is Y, or

   $X_2$ is R, $X_1$ is E, $X_3$ is F or P, $X_4$ is L or V or I and $X_5$ is Y, or

   $X_3$ is R, $X_1$ is E, $X_2$ is K, $X_4$ is L or V or I and $X_5$ is Y, or

   $X_4$ is R, $X_1$ is E, $X_2$ is K, $X_3$ is F or P and $X_5$ is Y, or

   $X_5$ is R, $X_1$ is E, $X_2$ is K, $X_3$ is F or P and $X_4$ is L or V or I,

   which respectively correspond to the following amino acid sequence:

   A-[I/M/T/L]-R-K-[F/P]-[LN/I]-Y-T-A (SEQ ID NO: 497), or
   A-[I/M/T/L]-E-R-[F/P]-[L/V/I]-Y-T-A (SEQ ID NO: 498), or
   A-[I/M/T/L]-E-K-R-[L/V/I]-Y-T-A (SEQ ID NO: 499), or
   A-[I/M/T/L]-E-K-[F/P]-R-Y-T-A (SEQ ID NO: 500), or
   A-[I/M/T/L]-E-K-[F/P]-[L/V/I]-R-T-A (SEQ ID NO: 501),

   in association with a pharmaceutically acceptable carrier,

   said loss of at least 50%, at least 75% or 100% of its immunosuppressive activity of the above mentioned mutated feline lentiviral ENV protein or of the above defined fragment being liable to be assessed by the fact that in an *in vivo* assay involving engrafted tumor cells rejection, in animals excluding human beings,

   said tumor cells being transduced either so as to express said mutated ENV protein or said fragment (mutated ENV tumor cells),

   or said tumor cells being transduced so as to express said wild type ENV protein or a fragment thereof (wild type ENV tumor cells),

   or said tumor cells being not transduced (normal tumor cells),

   the following ratio:

   $$\frac{\text{immunosuppression index of said mutated ENV protein or of said fragment } (i_{\text{mutated env}})}{\text{immunosuppression index of wild type ENV protein } (i_{\text{wild type env}})}$$

   is less than 0.5, or even less than 0.25,

   $i_{\text{mutated env}}$ being defined by:

   $$\frac{\text{maximum area reached by mutated ENV tumor cells} - \text{maximum area reached by normal tumor cells}}{\text{maximum area reached by normal tumor cells}},$$

   and

   $i_{\text{wild type env}}$ being defined by:

   $$\frac{\text{maximum area reached by wild type ENV tumor cells} - \text{maximum area reached by normal tumor cells}}{\text{maximum area reached by normal tumor cells}}.$$

2. Pharmaceutical composition according to claim 1,
   wherein said mutated feline lentiviral ENV protein or fragment thereof comprising a mutated immunosuppressive domain (ISU) containing the following amino acid sequence:

[V/I]-[E/R]-A-[I/M/T/L]-X$_1$-X$_2$-X$_3$-X$_4$-X$_5$-T-A-[F/L]-A-M (SEQ ID NO: 3), wherein,

X$_1$ is R, X$_2$ is K, X$_3$ is F or P, X$_4$ is L or V or I and X$_5$ is Y, or

X$_2$ is R, X$_1$ is E, X$_3$ is F or P, X$_4$ is L or V or I and X$_5$ is Y, or

X$_3$ is R, X$_1$ is E, X$_2$ is K, X$_4$ is L or V or I and X$_5$ is Y, or

X$_4$ is R, X$_1$ is E, X$_2$ is K, X$_3$ is F or P and X$_5$ is Y, or

X$_5$ is R, X$_1$ is E, X$_2$ is K, X$_3$ is F or P and X$_4$ is L or V or I,

in association with a pharmaceutically acceptable carrier.

3. Pharmaceutical composition according to claim 1 or 2, wherein said isolated mutated feline lentiviral ENV protein or said fragment thereof, comprises one of the amino acid sequences SEQ ID NO: 28, 29, 30, 31, 32, 33, 34, 35, 36, 37, 38, 39, 76, 77, 78, 79, 80, 81, 82, 83, 116, 117, 118, 119, 156, 157, 158 and 159, in particular, wherein said isolated mutated feline lentiviral ENV protein consists of one of the amino acid sequences : SEQ ID NO: 5 and SEQ ID NO: 320, 324, 329, 334 and 374 to 419.

4. Pharmaceutical composition comprising as active substance a nucleic acid molecule coding for a mutated feline lentiviral ENV protein, or a fragment of said mutated feline lentiviral ENV protein, as defined in any one of claims 1 to 3, wherein said nucleic acid molecule is contained in a vector, said vector comprising means allowing the expression of the mutated feline lentiviral ENV protein, or a fragment of said mutated feline lentiviral ENV protein, as defined in any one of claims 1 to 3,

in particular, wherein said vector is chosen among a canarypox vector, a pox vector, a fowlpox, an adenoviral vector, a lentiviral vector, a measles vector, a Sendaï virus vector, a Cytomegalovirus vector or a Modified Vaccinia Ankara vector.

5. Pharmaceutical composition according to claim 4, comprising at least one nucleic acid molecule coding for a GAG protein and/or a PRO protein and/or a POL protein of a feline lentivirus, said lentivirus being preferably of the same origin as the mutated lentiviral ENV protein,

in particular, wherein said nucleic acid molecule coding for a mutated feline lentiviral ENV protein, or a fragment of said mutated feline lentiviral ENV protein, is contained in the same vector as the one which also contains said at least one nucleic acid molecule coding for a GAG protein and/or a PRO protein and/or a POL protein.

6. Pharmaceutical composition as defined in any one of claims 1 to 5 for its use for stimulating an immune response in a host organism,

in particular, for use for the prevention or the treatment of lentiviral infection, preferably FIV infection,

in particular, for its use as a vaccine, in particular against FIV infection.

7. Method to obtain the active substance of a pharmaceutical composition, as defined in any one of claims 1 to 6, consisting in modifying the immunosuppressive property of:

a wild-type feline lentiviral ENV protein,

or a fragment of said wild-type feline lentiviral ENV protein, said fragment comprising at least 40 amino acids, in particular at least 60 amino acids,

said ENV protein or fragment thereof presenting a transmembrane subunit (TM) comprising an immunosuppressive domain (ISU) containing the following amino acid sequence:

A-[I/M/T/L]-X$_A$-X$_B$-X$_C$-X$_D$-X$_E$-T-A (SEQ ID NO: 344), wherein

X$_A$ is E, and

X$_B$ is K, and

X$_C$ is F or P, and

X$_D$ is L, V or I, and

X$_E$ is Y,

said method comprising a step of introduction of at least one mutation of X$_A$ or X$_B$ or X$_C$ or X$_D$ or X$_E$, to obtain:

an isolated mutated feline lentiviral ENV protein that has lost at least 50%, at least 75% or 100% of its immunosuppressive activity in comparison to the immunosuppressive activity of the wild type Env protein, said mutated feline lentiviral ENV protein having at least 70% identity, preferably at least 80% identity, to one sequence SEQ ID NO: 5,

or a fragment of said isolated mutated feline lentiviral ENV protein that has lost at least 50%, at least 75% or 100% of its immunosuppressive activity in comparison to the immunosuppressive activity of the wild type Env protein, said fragment comprising at least 40 amino acids, in particular at least 60 amino acids, said mutated ENV protein and fragment thereof comprising a mutated immunosuppressive domain (ISU) containing the following amino sequence:

A-[I/M/T/L]-$X_1$-$X_2$-$X_3$-$X_4$-$X_5$-T-A (SEQ ID NO: 1),

wherein

$X_1$ is R, $X_2$ is K, $X_3$ is F or P, $X_4$ is L or V or I and $X_5$ is Y, or

$X_2$ is R, $X_1$ is E, $X_3$ is F or P, $X_4$ is L or V or I and $X_5$ is Y, or

$X_3$ is R, $X_1$ is E, $X_2$ is K, $X_4$ is L or V or I and $X_5$ is Y, or

$X_4$ is R, $X_1$ is E, $X_2$ is K, $X_3$ is F or P and $X_5$ is Y, or

$X_5$ is R, $X_1$ is E, $X_2$ is K, $X_3$ is F or P and $X_4$ is L or V or I.

## Patentansprüche

1. Pharmazeutische Zusammensetzung, umfassend als Wirkstoff ein isoliertes, mutiertes, von Katzen stammendes lentivirales ENV-Protein oder ein Fragment davon, das, verglichen mit dem Wildtyp-ENV-Protein, mindestens 50 %, mindestens 75 % oder 100 % seiner immunsuppressiven Wirkung verloren hat,

wobei das Fragment des isolierten, mutierten, von Katzen stammenden lentiviralen ENV-Proteins mindestens 40 Aminosäuren, insbesondere mindestens 60 Aminosäuren umfasst,

wobei das mutierte, von Katzen stammende lentivirale ENV-Protein aus einer Mutation der Transmembranuntereinheit (TM) eines von Katzen stammenden lentiviralen Wildtyp-ENV-Proteins entsteht,

wobei das mutierte ENV-Protein mindestens 70 % Identität, vorzugsweise mindestens 80 % Identität zu der Sequenz SEQ ID NO: 5 aufweist,

wobei das mutierte, von Katzen stammende lentivirale ENV-Protein oder Fragment davon eine mutierte immunsuppressive Domäne (ISU) umfasst, die folgende Aminosäuresequenz enthält:

A-[I/M/T/L]-$X_1$-$X_2$-$X_3$-$X_4$-$X_5$-T-A (SEQ ID NO: 1),

wobei

$X_1$ R ist, $X_2$ K ist, $X_3$ F oder P ist, $X_4$ L oder V oder I ist und $X_5$ Y ist, oder

$X_2$ R ist, $X_1$ E ist, $X_3$ F oder P ist, $X_4$ L oder V oder I ist und $X_5$ Y ist, oder

$X_3$ R ist, $X_1$ E ist, $X_2$ K ist, $X_4$ L oder V oder I ist und $X_5$ Y ist, oder

$X_4$ R ist, $X_1$ E ist, $X_2$ K ist, $X_3$ F oder P ist und $X_5$ Y ist, oder

$X_5$ R ist, $X_1$ E ist, $X_2$ K ist, $X_3$ F oder P ist und $X_4$ L oder V oder I ist,

die jeweils der folgenden Aminosäuresequenz entsprechen:

A-[I/M/T/L]-R-K-[F/P]-[L/V/I]-Y-T-A (SEQ ID NO: 497), oder

A-[I/M/T/L]-E-R-[F/P]-[L/V/I]-Y-T-A (SEQ ID NO: 498), oder

A-[I/M/T/L]-E-K-R-[L/V/I]-Y-T-A (SEQ ID NO: 499), oder

A-[I/M/T/L]-E-K-[F/P]-R-Y-T-A (SEQ ID NO: 500), oder

A-[I/M/T/L]-E-K-[F/P]-[L/V/I]-R-T-A (SEQ ID NO: 501),

in Verbindung mit einem pharmazeutisch verträglichen Trägerstoff,

wobei der Verlust von mindestens 50 %, mindestens 75 % oder 100 % seiner immunsuppressiven Wirkung des vorstehend erwähnten mutierten, von Katzen stammenden lentiviralen ENV-Proteins oder des vorstehend definierten Fragments davon dadurch bewertet werden kann, dass in einem *in-vivo*-Assay, der die Abstoßung von transplantierten Tumorzellen bei Tieren, ausgenommen Menschen, beinhaltet,

die Tumorzellen derart transduziert werden, dass sie entweder das mutierte ENV-Protein oder das Fragment exprimieren (mutierte ENV-Tumorzellen),

oder dass die Tumorzellen derart transduziert werden, dass sie ein Wildtyp-ENV-Protein oder ein Fragment davon exprimieren (Wildtyp-ENV-Tumorzellen),

oder dass die Tumorzellen nicht transduziert werden (normale Tumorzellen),

das folgende Verhältnis:

$$\frac{\text{Immunsuppressionsindex des mutierten ENV-Proteins oder des Fragments } (i_{\text{mutiertes env}})}{\text{Immunsuppressionsindex des Wildtyp-ENV-Proteins } (i_{\text{wildtyp env}})}$$

beträgt weniger als 0,5 oder sogar weniger als 0,25, wobei $i_{\text{mutiertes env}}$ durch Folgendes definiert wird:

$$\frac{\text{maximaler von den mutierten ENV-Tumorzellen erreichter Bereich} - \text{maximaler von normalen Tumorzellen erreichter Bereich}}{\text{maximaler von normalen Tumorzellen erreichter Bereich}}$$

und

wobei $i_{\text{wiLdtyp env}}$ durch Folgendes definiert wird:

$$\frac{\text{maximaler von den Wildtyp-ENV-Tumorzellen erreichter Bereich} - \text{maximaler von normalen Tumorzellen erreichter Bereich}}{\text{maximaler von normalen Tumorzellen erreichter Bereich}}$$

**2.** Pharmazeutische Zusammensetzung nach Anspruch 1, wobei das mutierte, von Katzen stammende lentivirale ENV-Protein oder Fragment davon eine mutierte immunsuppressive Domäne (ISU) umfasst, die folgende Aminosäuresequenz enthält:

[V/I]-[E/R]-A-[I/M/T/L]-$X_1$-$X_2$-$X_3$-$X_4$-$X_5$-T-A-[F/L]-A-M (SEQ ID NO: 3),

wobei

$X_1$ R ist, $X_2$ K ist, $X_3$ F oder P ist, $X_4$ L oder V oder I ist und $X_5$ Y ist, oder

$X_2$ R ist, $X_1$ E ist, $X_3$ F oder P ist, $X_4$ L oder V oder I ist und $X_5$ Y ist, oder

$X_3$ R ist, X1 E ist, $X_2$ K ist, $X_4$ L oder V oder I ist und $X_5$ Y ist, oder

$X_4$ R ist, X1 E ist, $X_2$ K ist, $X_3$ F oder P ist und $X_5$ Y ist, oder

$X_5$ R ist, $X_1$ E ist, $X_2$ K ist, $X_3$ F oder P ist und $X_4$ L oder V oder I ist,

in Verbindung mit einem pharmazeutisch verträglichen Trägerstoff.

**3.** Pharmazeutische Zusammensetzung nach Anspruch 1 oder 2, wobei das isolierte, mutierte, von Katzen stammende lentivirale ENV-Protein oder das Fragment davon eine der folgenden Aminosäuresequenzen umfasst SEQ ID NO: 28, 29, 30, 31, 32, 33, 34, 35, 36, 37, 38, 39, 76, 77, 78, 79, 80, 81, 82, 83, 116, 117, 118, 119, 156, 157, 158 und 159, insbesondere wobei das isolierte, mutierte, von Katzen stammende lentivirale ENV-Protein aus mindestens einer der folgenden Aminosäuresequenzen besteht: SEQ ID NO: 5 und SEQ ID NO: 320, 324, 329, 334 und 374 bis 419.

**4.** Pharmazeutische Zusammensetzung, umfassend als Wirkstoff ein Nukleinsäuremolekül, das für ein mutiertes, von Katzen stammendes lentivirales ENV-Protein oder ein Fragment des mutierten, von Katzen stammenden lentiviralen ENV-Proteins, wie in einem der Ansprüche 1 bis 3 definiert, kodiert,

wobei das Nukleinsäuremolekül in einem Vektor enthalten ist, wobei der Vektor Mittel umfasst, die die Expression des mutierten, von Katzen stammenden lentiviralen ENV-Proteins oder eines Fragments des mutierten, von Katzen stammenden lentiviralen ENV-Proteins, wie in einem der Ansprüche 1 bis 3 definiert, ermöglichen,

insbesondere wobei der Vektor ausgewählt ist aus einem Kanarienpockenvektor, einem Pockenvektor, einem Vogelpockenvektor, einem Adenovirusvektor, einem Lentivirusvektor, einem Masernvektor, einem Sendaivirusvektor, einem Zytomegalievirusvektor oder einem Modified-Vaccinia-Ankara-Virusvektor.

**5.** Pharmazeutische Zusammensetzung nach Anspruch 4, umfassend mindestens ein Nukleinsäuremolekül, das für ein GAG-Protein und/oder ein PRO-Protein und/oder ein POL-Protein eines von Katzen stammenden Lentivirus kodiert, wobei das Lentivirus vorzugsweise denselben Ursprung wie das mutierte lentivirale ENV-Protein hat, insbesondere wobei das Nukleinsäuremolekül, das für ein mutiertes, von Katzen stammendes lentivirales ENV-Protein oder ein Fragment des mutierten, von Katzen stammenden lentiviralen ENV-Proteins kodiert, in demselben Vektor enthalten ist wie dem, der das mindestens eine Nukleinsäuremolekül enthält, das für ein GAG-Protein und/oder ein PRO-Protein und/oder ein POL-Protein kodiert.

**6.** Pharmazeutische Zusammensetzung, wie in einem der Ansprüche 1 bis 5 definiert, zur Verwendung für die Stimulierung einer Immunreaktion in einem Wirtsorganismus,
insbesondere zur Verwendung bei der Prävention oder der Behandlung einer lentiviralen Infektion, vorzugsweise einer FIV-Infektion,
insbesondere zur Verwendung als Impfstoff, insbesondere gegen eine FIV-Infektion.

**7.** Verfahren zum Erhalt des Wirkstoffs einer pharmazeutischen Zusammensetzung, wie in einem der Ansprüche 1 bis 6 definiert, bestehend aus dem Modifizieren der immunsuppressiven Eigenschaft von:

einem von Katzen stammenden lentiviralen Wildtyp-ENV-Protein,
oder einem Fragment des von Katzen stammenden lentiviralen Wildtyp-ENV-Proteins, wobei das Fragment mindestens 40 Aminosäuren, insbesondere mindestens 60 Aminosäuren umfasst,
wobei das ENV-Protein oder Fragment davon eine Transmembranuntereinheit (TM) enthält, umfassend eine immunsuppressive Domäne (ISU), umfassend die folgende Aminosäuresequenz:
A-[I/M/T/L]-$X_A$-$X_B$-$X_C$-$X_D$-$X_E$-T-A (SEQ ID NO: 344),
wobei
$X_A$ E ist, und
$X_B$ K ist, und
$X_C$ F oder P ist, und
$X_D$ L, V oder I ist, und
$X_E$ Y ist,
wobei das Verfahren einen Schritt des Einbringens von mindestens einer Mutation von $X_A$ oder $X_B$ oder $X_C$ oder $X_D$ oder $X_E$ umfasst,
um Folgendes zu erhalten:

ein isoliertes, mutiertes, von Katzen stammendes lentivirales ENV-Protein, das, verglichen mit der immunsuppressiven Wirkung des Wildtyp-ENV-Proteins, mindestens 50 %, mindestens 75 % oder 100 % seiner immunsuppressiven Wirkung verloren hat,
wobei das mutierte, von Katzen stammende lentivirale ENV-Protein mindestens 70 % Identität, vorzugsweise mindestens 80 % Identität zu einer Sequenz SEQ ID NO: 5 aufweist,
oder ein Fragment des isolierten, mutierten, von Katzen stammenden lentiviralen ENV-Proteins, das, verglichen mit der immunsuppressiven Wirkung des Wildtyp-ENV-Proteins, mindestens 50 %, mindestens 75 % oder 100 % seiner immunsuppressiven Wirkung verloren hat, wobei das Fragment mindestens 40 Aminosäuren, insbesondere mindestens 60 Aminosäuren umfasst,
wobei das mutierte ENV-Protein und Fragment davon eine mutierte immunsuppressive Domäne (ISU) umfassen, umfassend die folgende Aminosäuresequenz:
A-[I/M/T/L]-$X_1$-$X_2$-$X_3$-$X_4$-$X_5$-T-A (SEQ ID NO: 1),
wobei
$X_1$ R ist, $X_2$ K ist, $X_3$ F oder P ist, $X_4$ L oder V oder I ist und $X_5$ Y ist, oder
$X_2$ R ist, $X_1$ E ist, $X_3$ F oder P ist, $X_4$ L oder V oder I ist und $X_5$ Y ist, oder
$X_3$ R ist, $X_1$ E ist, $X_2$ K ist, $X_4$ L oder V oder I ist und $X_5$ Y ist, oder
$X_4$ R ist, $X_1$ E ist, $X_2$ K ist, $X_3$ F oder P ist und $X_5$ Y ist, oder
$X_5$ R ist, $X_1$ E ist, $X_2$ K ist, $X_3$ F oder P ist und $X_4$ L oder V oder I ist.

**Revendications**

**1.** Composition pharmaceutique comprenant comme substance active une protéine ENV lentivirale féline mutée isolée, ou un fragment de celle-ci, qui a perdu au moins 50%, au moins 75% ou 100% de son activité immunosuppressive en comparaison avec l'activité immunosuppressive de la protéine ENV sauvage,

ledit fragment de ladite protéine ENV lentivirale féline mutée isolée comprenant au moins 40 acides aminés, en particulier au moins 60 acides aminés,

ladite protéine ENV lentivirale féline mutée résultant de la mutation de la sous-unité transmembranaire (TM) d'une protéine ENV lentivirale féline sauvage,

ladite protéine ENV mutée ayant au moins 70% d'identité, de préférence au moins 80% d'identité avec la séquence SEQ ID NO : 5,

ladite protéine ENV lentivirale féline mutée ou un fragment de celle-ci comprenant un domaine immunosuppresseur muté (ISU) contenant la séquence d'acides aminés suivante :

A-[I/M/T/L]-$X_1$-$X_2$-$X_3$-$X_4$-$X_5$-T-A (SEQ ID NO: 1),

dans laquelle,

$X_1$ est R, $X_2$ est K, $X_3$ est F ou P, $X_4$ est L ou V ou I et $X_5$ est Y, ou

$X_2$ est R, $X_1$ est E, $X_3$ est F ou P, $X_4$ est L ou V ou I et $X_5$ est Y, ou

$X_3$ est R, $X_1$ est E, $X_2$ est K, $X_4$ est L ou V ou I et $X_5$ est Y, ou

$X_4$ est R, $X_1$ est E, $X_2$ est K, $X_3$ est F ou P et $X_5$ est Y, ou

$X_5$ est R, $X_1$ est E, $X_2$ est K, $X_3$ est F ou P et $X_4$ est L ou V ou I,

ce qui correspond respectivement à la séquence d'acides aminés suivante :

A-[I/M/T/L]-R-K-[F/P]-[L/V/I]-Y-T-A (SEQ ID NO : 497), ou

A-[I/M/T/L]-E-R-[F/P]-[LN/I]-Y-T-A (SEQ ID NO : 498), ou

A-[I/M/T/L]-E-K-R-[LN/I]-Y-T-A (SEQ ID NO : 499), ou

A-[I/M/T/L]-E-K-[F/P]-R-Y-T-A (SEQ ID NO : 500), ou

A-[I/M/T/L]-E-K-[F/P]-[L/V/I]-R-T-A (SEQ ID NO : 501),

en association avec un véhicule pharmaceutiquement acceptable,

ladite perte d'au moins 50%, d'au moins 75% ou 100% de l'activité immunosuppressive de la protéine ENV lentivirale féline mutée mentionnée ci-dessus ou du fragment défini ci-dessus étant susceptible d'être évaluée par le fait que, dans une analyse *in vivo* impliquant un rejet de cellules tumorales greffées, chez des animaux à l'exclusion des êtres humains,

lesdites cellules tumorales étant transduites pour exprimer ladite protéine ENV mutée ou ledit fragment (cellules tumorales ENV mutées),

ou lesdites cellules tumorales étant transduites de manière à exprimer ladite protéine ENV sauvage ou un fragment de celle-ci (cellules tumorales ENV sauvages),

ou lesdites cellules tumorales n'étant pas transduites (cellules tumorales normales),

le rapport suivant :

$$\frac{\text{index d'immunosuppression de ladite protéine ENV mutée ou dudit fragment (}i_{\text{env mutée}}\text{)}}{\text{index d'immunosuppression de la protéine ENV sauvage (}i_{\text{env sauvage}}\text{)}}$$

est inférieur à 0,5, ou même inférieur à 0,25,

ienv mutée étant défini par :

$$\frac{(\text{surface maximale atteinte par les cellules tumorales ENV mutées} - \text{surface maximale atteinte par les cellules tumorales normales})}{\text{surface maximale atteinte par les cellules tumorales normales}},$$

et

$i_{\text{wild type env}}$ étant défini par :

$$\frac{(\text{surface maximale atteinte par les cellules tumorales ENV sauvages} - \text{surface maximale atteinte par les cellules tumorales normales})}{\text{surface maximale atteinte par les cellules tumorales normales}}.$$

2. Composition pharmaceutique selon la revendication 1,

dans laquelle ladite protéine ENV lentivirale féline mutée ou un fragment de celle-ci comprenant un domaine immunosuppresseur muté (ISU) contenant la séquence d'acides aminés suivante :

[V/I]-[E/R]-A-[I/M/T/L]-X$_1$-X$_2$-X$_3$-X$_4$-X$_5$-T-A-[F/L]-A-M (SEQ ID NO : 3), dans laquelle,
X$_1$ est R, X$_2$ est K, X$_3$ est F ou P, X$_4$ est L ou V ou I et X$_5$ est Y, ou
X$_2$ est R, X$_1$ est E, X$_3$ est F ou P, X$_4$ est L ou V ou I et X$_5$ est Y, ou
X$_3$ est R, X$_1$ est E, X$_2$ est K, X$_4$ est L ou V ou I et X$_5$ est Y, ou
X$_4$ est R, X$_1$ est E, X$_2$ est K, X$_3$ est F ou P et X$_5$ est Y, ou
X$_5$ est R, X$_1$ est E, X$_2$ est K, X$_3$ est F ou P et X$_4$ est L ou V ou I,
en association avec un véhicule pharmaceutiquement acceptable.

3. Composition pharmaceutique selon la revendication 1 ou 2, dans laquelle ladite protéine ENV lentivirale féline mutée isolée ou un fragment de celle-ci, comprend l'une des séquences d'acides aminés SEQ ID NO : 28, 29, 30, 31, 32, 33, 34, 35, 36, 37, 38, 39, 76, 77, 78, 79, 80, 81, 82, 83, 116, 117, 118, 119, 156, 157, 158 et 159, en particulier, dans laquelle ladite protéine ENV lentivirale féline mutée isolée consiste en l'une des séquences d'acides aminés : SEQ ID NO : 5 et SEQ ID NO : 320, 324, 329, 334 et 374 à 419.

4. Composition pharmaceutique comprenant comme substance active une protéine ENV lentivirale féline mutée isolée, ou un fragment de ladite protéine ENV lentivirale féline mutée, tel que défini selon l'une quelconques des revendications 1 à 3, dans laquelle ladite molécule d'acide nucléique est contenue dans un vecteur, ledit vecteur comprenant des moyens permettant l'expression de la protéine ENV lentivirale féline mutée isolée, ou un fragment de ladite protéine ENV lentivirale féline mutée, tel que défini selon l'une quelconques des revendications 1 à 3, en particulier, dans laquelle ledit vecteur est choisi parmi un vecteur canarypox, un vecteur pox, une variole aviaire, un vecteur adénoviral, un vecteur lentiviral, un vecteur rougeole, un vecteur viral Sendai, un vecteur Cytomégalovirus ou un vecteur de la vaccine Ankara modifiée.

5. Composition pharmaceutique selon la revendication 4, comprenant au moins une molécule d'acide nucléique codant pour une protéine GAG et/ou une protéine PRO et/ou une protéine POL d'un lentivirus félin, ledit lentivirus étant de préférence de la même origine que la protéine ENV lentivirale mutée, en particulier, dans laquelle ladite molécule d'acide nucléique codant pour une protéine ENV lentivirale féline mutée, ou un fragment de ladite protéine ENV lentivirale féline mutée, est contenue dans le même vecteur que celui qui contient également ladite au moins une molécule d'acide nucléique codant pour une protéine GAG et/ou une protéine PRO et/ou une protéine POL.

6. Composition pharmaceutique telle que définie selon l'une quelconques des revendications 1 à 5 pour son utilisation pour stimuler une réponse immunitaire dans un organisme hôte, en particulier, pour son utilisation pour la prévention ou le traitement de l'infection à lentivirus, de préférence l'infection par le FIV, en particulier pour son utilisation en tant que vaccin, en particulier contre l'infection par le FIV.

7. Procédé d'obtention de la substance active d'une composition pharmaceutique, telle que définie dans l'une quelconque des revendications 1 à 6, consistant à modifier la propriété immunosuppressive de :

   une protéine ENV lentivirale féline sauvage,
   ou un fragment de ladite protéine ENV lentivirale féline sauvage, ledit fragment de ladite protéine ENV lentivirale féline mutée isolée comprenant au moins 40 acides aminés, en particulier au moins 60 acides aminés,
   ladite protéine ENV lentivirale féline mutée ou un fragment de celle-ci comprenant un domaine immunosuppresseur muté (ISU) contenant la séquence d'acides aminés suivante :
   A-[I/M/T/L]-X$_A$-X$_B$-X$_C$-X$_D$-X$_E$-T-A (SEQ ID NO : 344),
   dans laquelle
   X$_A$ est E, et
   X$_B$ est K, et
   X$_C$ est F ou P, et
   X$_D$ est L, V ou I, et
   X$_E$ est Y,
   ledit procédé comprenant une étape d'introduction d'au moins une mutation de X$_A$ ou X$_B$ ou X$_C$ ou X$_D$ ou X$_E$,
   pour obtenir :

      une protéine ENV lentivirale féline mutée isolée qui a perdu au moins 50%, au moins 75% ou 100% de son activité immunosuppressive en comparaison avec l'activité immunosuppressive de la protéine ENV sauvage,

ladite protéine ENV lentivirale féline mutée ayant au moins 70% d'identité, préférablement au moins 80% identité avec la séquence SEQ ID NO : 5,

ou un fragment de ladite protéine ENV lentivirale féline mutée isolée qui a perdu au moins 50%, au moins 75% ou 100% de son activité immunosuppressive en comparaison avec l'activité immunosuppressive de la protéine ENV sauvage, ledit fragment comprenant au moins 40 acides aminés, en particulier au moins 60 acides aminés,

ladite protéine ENV mutée et ledit fragment de celle-ci comprenant un domaine immunosuppresseur muté (ISU) contenant la séquence d'acides aminés suivante :

A-[I/M/T/L]-$X_1$-$X_2$-$X_3$-$X_4$-$X_5$-T-A (SEQ ID NO : 1),

dans laquelle

$X_1$ est R, $X_2$ est K, $X_3$ est F ou P, $X_4$ est L ou V ou I et $X_5$ est Y, ou

$X_2$ est R, $X_1$ est E, $X_3$ est F ou P, $X_4$ est L ou V ou I et $X_5$ est Y, ou

$X_3$ est R, $X_1$ est E, $X_2$ est K, $X_4$ est L ou V ou I et $X_5$ est Y, ou

$X_4$ est R, $X_1$ est E, $X_2$ est K, $X_3$ est F ou P et $X_5$ est Y, ou

$X_5$ est R, $X_1$ est E, $X_2$ est K, $X_3$ est F ou P et $X_4$ est L ou V ou I.

**SUBTYPE B**

Jn-Yokohama
Jn-Aomori1
FL-FC1
Jn-Sendai2  Italy M2
Jn-TM2  Jn-Aomori2
MD-MtAiry
FL-FC2
IL-USIL2489_7B
MA-Bangston
CA-PPR
Dutch113
Canada-CABCpady02C
Dutch19K
Canada-BM3070
**SUBTYPE C**
France-Wo
SwissZ1
Argentina-LP24
CA-Petaluma
**SUBTYPE E**  Argentina-LP20
CA-Dixon
Jn-Fukuoka
Jn-Sendai1
UK2
UK8
SwissZ2
**SUBTYPE D**  **SUBTYPE A**
Jn-Shizuoka
0.1

**Figure 1A**

FIV64

```
CAA40315.1    QVLATHQEAIEKVTEALKINNLRLVTLEHQVLVIGLKVEAMEKFLYTAFAMQELGCNQNQFFCKVPPVLWER  (SEQ ID NO : 469)
1805419B      QVLATHQEAIEKVTEALKINNLRLVTLEHQVLVIGLKVEAMEKFLYTAFAMQELGCNQNQFFCKVPPVLWER  (SEQ ID NO : 469)
NP_040976.1   QVLATHQEAVEKVTEALKINNLRLVTLEHQVLVIGLKVEAMEKFLYTAFAMQELGCNQNQFFCKIPPELWTR  (SEQ ID NO : 470)
AAB59940.1    QVLATHQEAVEKVTEALKINNLRLVTLEHQVLVIGLKVEAMEKFLYTAFAMQELGCNQNQFFCKIPPELWTR  (SEQ ID NO : 470)
ACX55587.1    QVLATHQEAIEKVTEALKINNLRLVTLEHQVLVIGLKVEAMEKFLYTAFAMQELGCNQNQFFCKIPPGLWTR  (SEQ ID NO : 471)
1805419A      QVLATHQEAIEKVTEALKINNLRLVTLEHQVLVIGLKVEAMEKFLYTAFAMQELGCNQNQFFCKIPPGLWTR  (SEQ ID NO : 471)
CAA40321.1    QVLATHQEAIEKVTEALKINNLRLVTLEHQVLVIGLKVEAMEKFLYTAFAMQELGCNQNQFFCKIPPGLWTR  (SEQ ID NO : 471)
ENV_FIVPE     QVLATHQEAIEKVTGALKINNLRLVTLEHQVLVIGLKVEAMEKFLYTAFAMQELGCNQNQFFCKIPLELWTR  (SEQ ID NO : 472)
AAB25466.1    QVLATQQEAIEKVTEALKITNLRLVTLEHQVLVIGLKVEAMEKFLYTAFAMQELGCNQNQFFCKVPPELWRR  (SEQ ID NO : 473)
ENV_FIVU1     QVLATQQEAIEKVTEALKITNLRLVTLEHQVLVIGLKVEAMEKFLYTAFAMQELGCNQNQFFCKVPPELWRR  (SEQ ID NO : 473)
CAA43131.1    QVLATQQEAIEKVTEALKITNLRLVTLEHQVLVIGLKVEAMEKFLYTAFAMQELGCNQNQFFCKVPPELWRR  (SEQ ID NO : 473)
AF474246_5    QVLATHQEALDKITEALKINNLRLVTLEHQVLVIGLKVEATEKFLYTAFAMQELGCNQNQFFCKIPCELWMR  (SEQ ID NO : 474)
AAB09312.1    QVLATHQQALDKITEALKINNLRLITLEHQVLVIGLKVEAIEKFLYTAFAMQELGCNQNQFFCKIPPSLWSM  (SEQ ID NO : 475)
AAA43074.1    QVLATHQQALEKITEALKINNLRLITLEHQVLVIGLRVEAIEKFLYTAFAMQELGCNQNQFFCKIPPSLWSM  (SEQ ID NO : 476)
ENV_FIVT2     QVLATHQQALEKITEALKINNLRLITLEHQVLVIGLRVEAIEKFLYTAFAMQELGCNQNQFFCKIPPSLWSM  (SEQ ID NO : 476)
BAA07063.1    QVLATHQQALEKITEALKINNLRLITLEHQVLVIGLKVEAIEKFLYTAFAMQELGCHQNQFFCKIPPSLWSM  (SEQ ID NO : 477)
BAA07062.1    QVLATHQQALDKITEALKINNLRLITLEHQVLVIGLKVEAIEKFLYTAFAMQELGCNQNQFFCKIPPSLWSM  (SEQ ID NO : 475)
BAA07060.1    QVLATHQQALDKITEALKINNLRLITLEHQVLVIGLKVEAIEKFLYTAFAMQELGCNQNQFFCKIPPSLWSM  (SEQ ID NO : 475)
BAA07058.1    QVLATHQHALDKITEALKINNLRLITLVHQVLVIGLKVRAIEKPLYTAFAMQELGCNQNQFFCKIPPSLWSM  (SEQ ID NO : 478)
AAT44733.1    QVLATHQQALDKITQALKINNLRLITLEHQVLVIGLKVEAIEKFLYTAFAMQELGCNQNQFFCKIPPSLWSM  (SEQ ID NO : 479)
AAT41620.1    QVLATHQQALDKITEALKINNLRLITLEHQVLVIGLKVEAIEKFLYTAFAMQELGCNQNQFFCKIPLNLWNM  (SEQ ID NO : 480)
AF452127_1    QVLATHQQALEKITEALKINNLRLVTLEHQVLMIGLKVEAIEKFLYTAFAMQELGCNQNQFFCKIPLNLWTM  (SEQ ID NO : 481)
AF452126_1    QVLATHQQALEKITEALKINNLRLVTLEHQVLMIGLKVEAIEKFLYTAFAMQELGCNQNQFFCKIPLNLWTM  (SEQ ID NO : 481)
BAA07061.1    QVLATHQQALDKITEALKINNLRLITLEHQVLVIGLKVEAIEKFLYTAFAMQELGCNQNQFFCKIPPSLWSM  (SEQ ID NO : 475)
BAA07057.1    QVLATHQKAIDQITEALKINNLRLVTLEHQVLVIGLKVEAIEKFIYTAFAMQELGCNQNQFFCKIPPELWIR  (SEQ ID NO : 482)
ENV_FIVSD     QVLATHQEALDKITEALKINNLRLVTLEHQMLVIGLKVEAIEKFLYTAFAMQELGCNQNQFFCEIPKELWLR  (SEQ ID NO : 483)
AAA43068.1    QVLATHQEAIEKVTEALKINNLRLVTLEHQVLVIGLKVEAMEKFLYTAFAMQELGCNQNQFFCKVPSALWER  (SEQ ID NO : 484)
ENV_FIVWO     QVLATHQEAIEKVTEALKINNLRLVTLEHQVLVIGLKVEAMEKFLYTAFAMQELGCNQNQFFCKVPSALWER  (SEQ ID NO : 484)
AAK83091.1    QVLATHQEAIEKVTEALKINNLRLVTLEHQVLVIGLKVEAMEKFLYTAFAMQELGCNQNQFFCKVPSALWER  (SEQ ID NO : 484)
AAA72278.1    QVLATHQEAIEKVTEALKINNLRLVTLEHQVLVIGLKVEAMEKFLYTAFAMQELGCNQNQFFCKVPPQLWKR  (SEQ ID NO : 485)
ENV_FIVU8     QVLATHQETIEKVTEALKINNLRLVTLEHQVLVIGLKVEAMEKFLYTAFAMQELGCNQNQFFCKVPPELWKR  (SEQ ID NO : 486)
BAA07059.1    QVLATHQEAIEKVTEALKVNNLRLITLEHQVLVIGLKVEAMEKFLYTAFAMQELGCNQNQFFCKVPPELWKR  (SEQ ID NO : 487)
ENV_FIVU2     QVLATHQETIEKITEALKVNNLRLVTLEHQVLVIGLKVEAIEKFLYTAFAMQELGCNQNQFFCKVPPELWQR  (SEQ ID NO : 488)
```

**Figure 1B**

Figure 2

**FIV64 :** 651 AIEKVTGALKINNLRLVTLEHQVLVIGLKVEAMEKFLYTAFAMQELGCNQNQFFCKIPLELWTR

685 686 687 688 689

**Figure 3**

Figure 4

Figure 5

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010022740 A

### Non-patent literature cited in the description

- **BENIT et al.** *Journal of Virology,* 2001, vol. 75 (23), 11709-11719
- **MANGENEY ; HEIDMANN.** *Proc Natl Acad Sci USA,* 1998, vol. 95, 14920-14925
- **MANGENEY et al.** *Proc Natl Acad Sci USA,* 2007, vol. 104 (51), 20534-9
- **CAFFREY M.** *Biochimica et Biophysica Acta,* 2001, vol. 1536, 116-122
- **CAFFREY et al.** *The EMBO Journal,* 1998, vol. 17 (16), 4572-4584
- **DENNER et al.** *Current Science, AIDS,* 1994, vol. 8, 1063-1072
- **BELLIER et al.** *Vaccine,* 2009, vol. 27 (42), 5772-80
- **GUERBOIS et al.** *Virology,* 2009, vol. 388, 191-203
- **POULET et al.** *Veterinary Record,* 2003, vol. 153 (5), 141-145

- **VACCARI et al.** *Expert Review of Vaccines,* 2010, vol. 9 (9), 997-1005
- **PU et al.** *Journal of Feline Medicine and Surgery,* 2005, vol. 7, 65-70
- **SCHLECHT-LOUF et al.** *Proc Natl Acad Sci U S A.,* 2010, vol. 107 (8), 3782-7
- **MANGENEY et al.** *Proc Natl Acad Sci USA.,* 2007, vol. 104 (51), 20534-9
- **TALBOTT et al.** *Proc Natl Acad Sci USA,* 1989, vol. 86 (15), 5743-4747
- **MANGENEY et al.** *PNAS,* 2007
- **DEMAISON et al.** *Human Gene Therapy,* 2002, vol. 13, 803-813
- **CENTER et al.** *Protein Sci,* 1998, vol. 7 (7), 1612-1619
- *PNAS,* 1998
- *PNAS,* 2007